(19) Europäisches Patentamt / European Patent Office / Office européen des brevets

(11) **EP 2 130 550 A1**

(12) **DEMANDE DE BREVET EUROPEEN**

(43) Date de publication:
**09.12.2009 Bulletin 2009/50**

(21) Numéro de dépôt: **08290522.5**

(22) Date de dépôt: **06.06.2008**

(51) Int Cl.:
*A61K 38/43* (2006.01)  *A61P 35/00* (2006.01)
*A61P 33/00* (2006.01)  *A61P 33/02* (2006.01)

(84) Etats contractants désignés:
**AT BE BG CH CY CZ DE DK EE ES FI FR GB GR HR HU IE IS IT LI LT LU LV MC MT NL NO PL PT RO SE SI SK TR**
Etats d'extension désignés:
**AL BA MK RS**

(71) Demandeurs:
• **Institut Pasteur**
  **75015 Paris (FR)**
• **INSTITUT PASTEUR DE TUNIS**
  **1002 Tunis (TN)**

(72) Inventeurs:
• **Guizani, Ikram**
  **2080 Ariana (TN)**
• **Barhoumi, Mourad**
  **2042 Tunis (TN)**
• **Garnaoui, Amel**
  **1002 Tunis (TN)**
• **Tanner, Nole Kyle**
  **1205 Geneve (CH)**

(74) Mandataire: **Rançon, Xavier Lucien Abel et al**
  **Cabinet Ores**
  **36 Rue de Saint Petersbourg**
  **75008 Paris (FR)**

(54) **Utilisation d'une ARN hélicase à boîte dead pour induire la production de cytokines**

(57) La présente invention concerne l'utilisation d'une ARN hélicase à boîte DEAD de levure, de mammifère ou de *Leishmania infantum* ou des fragments de celles-ci pour induire la production de cytokines par une cellule mononucléaire du sang périphérique (PBMC) d'un mammifère et ses applications.

EP 2 130 550 A1

**Description**

La présente invention concerne l'utilisation d'une ARN hélicase à boîte DEAD de levure, de mammifère ou de *Leishmania infantum* pour induire la production de cytokines par une cellule mononucléaire du sang périphérique (PBMC) d'un mammifère et ses applications.

Les hélicases sont des protéines capables de dérouler les duplexes d'ADN ou d'ARN en utilisant l'énergie libérée par l'hydrolyse de nucléotides triphosphates (NTP) (Caruthers and McKay, 2002 ; Tanner and Linder, 2001). Deux classes d'hélicases ont été identifiées selon la spécificité du substrat :

- Les ADN hélicases qui fixent l'ADN, déroulent les structures complémentaires d'ADN et dissocient les interactions ADN-protéine. Ce sont des protéines ubiquitaires identifiées chez différents organismes allant des virus jusqu'aux eucaryotes supérieurs. Elles interviennent dans plusieurs processus biologiques où la structure double brin de l'ADN doit être déroulée sous forme simple brin pour servir comme substrat dans différents processus biologiques tels que la réplication, la recombinaison, la transcription, et la réparation de l'ADN (Hall and Matson, 1999 ; Tuteja and Tuteja, 2004 a,b).
- Les ARN hélicases qui déroulent les duplexes d'ARN et dissocient les interactions ARN-ARN et ARN-protéine. Ces protéines se rencontrent chez tous les organismes procaryotes, eucaryotes et chez plusieurs virus (Rocak and Linder, 2004). Elles interviennent dans tous les processus du métabolisme de l'ARN : la transcription, la biogenèse des ribosomes, l'épissage des pré-ARNm, l'export nucléaire des ARNm, l'initiation de la traduction et la dégradation des ARNm (de la Cruz *et al.*, 1999 ; Schmid and Linder, 1992 ; Silverman *et al.*, 2003).

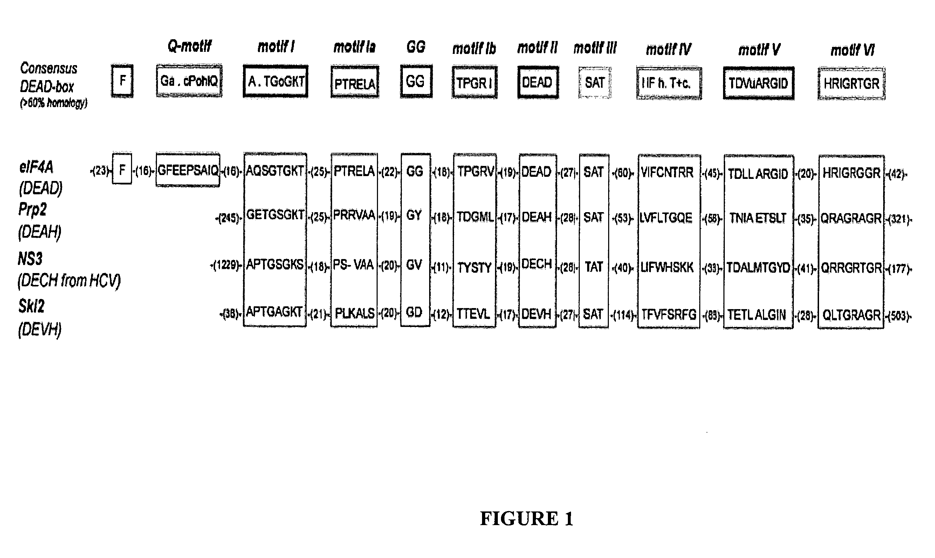

Sur la base de comparaisons de séquences protéiques, Gorbalenya et Koonin ont proposé une classification générale des hélicases en 5 familles majeures (Gorbalenya and Koonin, 1993). Chaque famille est caractérisée par des motifs conservés dont le nombre et la séquence lui sont propres. Toutes les familles possèdent les motifs de liaison et d'hydrolyse des NTP appelés walker A et B (Walker *et al.*, 1982). Les superfamilles 1 et 2 (SF1 et SF2) sont les plus représentées. Elles regroupent des ADN et ARN hélicases de bactéries, d'archaebactéries, d'eubactéries, d'eucaryotes et des virus. Elles sont caractérisées par 7 à 9 motifs conservés dont 5 à 7 motifs communs (voir Figure 1). La superfamille SF3 regroupe des protéines de petites tailles (environ 100 acides aminés) de virus à ADN et à ARN (Kadare and Haenni, 1997). Elles sont caractérisées par 3 motifs conservés. Les protéines de la superfamille SF4 sont des ADN hélicases qui interviennent dans la réplication de l'ADN des bactéries et des bactériophages (Ilyima *et al.*, 1992). Elles sont caractérisées par 5 motifs conservés H1, H1a, H2, H3 et H4. Patel et Picha ont montré que ces protéines forment des hexamères qui sont capables de dérouler l'ADN dans la direction 5'vers 3'. La superfamille SF5 est représentée par le facteur de la transcription Rho (Patel and Picha, 2000). Toutes ces protéines présentent une structure similaire, composée par un coeur (ou « core ») contenant des motifs de fixation et d'hydrolyse des NTP et des motifs de liaison à l'acide nucléique (Caruthers and McKay, 2002 ; Hall and Matson, 1999 ; Tanner and Linder, 2001 ; Ye *et al.*, 2004).

Les protéines à boîte DExD/H, appartenant à la famille SF2, forment la plus grande famille d'ARN hélicases à ce jour identifiée. Elles sont présentes chez tous les organismes, de la bactérie à l'homme, ainsi que chez certains virus (de la Cruz *et al.*, 1999 ; Rocak and Linder, 2004 ; Schmid and Linder, 1992). Ces protéines ont été identifiés pour la première fois lorsque Linder et ses collaborateurs (1989) ont aligné les séquences de huit protéines homologues du facteur d'initiation de la traduction des eucaryotes eIF4A. La taille de ces protéines varie entre 400 et 1200 résidus. Depuis, des études ont démontré l'existence de neuf motifs conservés : Q, I, Ia, Ib, II, III, IV, V et VI qui sont regroupés dans une région centrale, appelée le coeur hélicase (Cordin *et al.*, 2006 ; Tanner and Linder, 2001 ; voir Figure 1). Les protéines à boîte DEAD (« DEAD-box ») tirent leur nom de la séquence de leur motif II DEAD (pour aspartate-glutamate-alanine-aspartate). Les régions situées en position N-terminale et C-terminale du coeur hélicase sont quant à elles très variables en taille et en séquence.

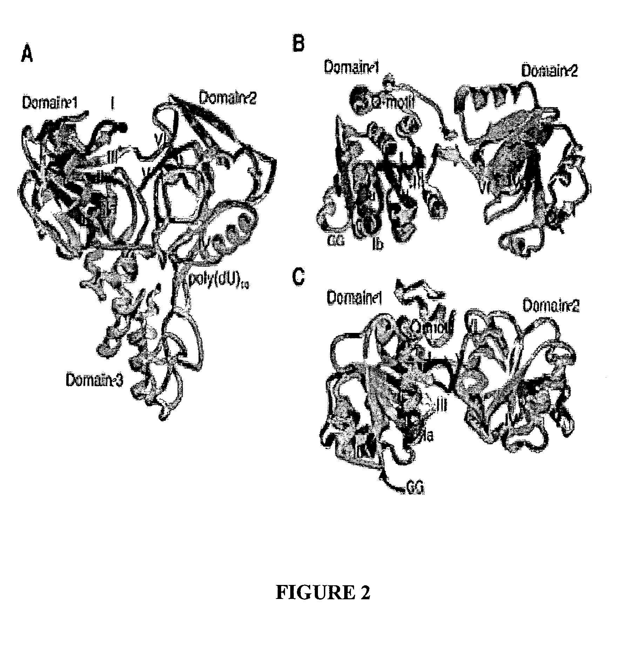

Au cours des dix dernières années, plusieurs structures d'hélicases des différentes superfamilles ont été caractérisées. Toutes les structures des protéines à boîte DEAD ainsi que celles des hélicases de la superfamille SF1 actuellement disponibles, révèlent un thème commun qui consiste en deux domaines globulaires, connectés par un lien flexible (voir Figure 2) (Story and Steitz, 1992). Le domaine 1 (D1) contient les motifs de liaison d'ATP (Q, I, II et III ; voir Figures 2B et C) alors que le domaine 2 (D2) contient les motifs de liaison d'ARN (IV, V et VI). De la même manière que pour les autres hélicases, tous les motifs conservés dans les protéines à boîte DEAD sont localisés au niveau des transitions feuillet $\beta$-boucle ou hélice $\alpha$-boucle (Tanner and Linder, 2001). Outre les cinq feuillets $\beta$ et les cinq hélices $\alpha$ des deux domaines 1 et 2, les protéines à boîte DEAD possèdent un feuillet $\beta$ et deux hélices $\alpha$ localisés en amont du motif I qui enroulent le motif Q (Tanner *et al.*, 2003).

Les protéines à boîte DEAD présentent dans leur partie centrale neuf motifs conservés, ordonnés de manière identique et espacés d'un nombre similaire d'acides aminés. Des analyses mutationnelles combinées à des études structure-fonction ont permis de leur attribuer des rôles.

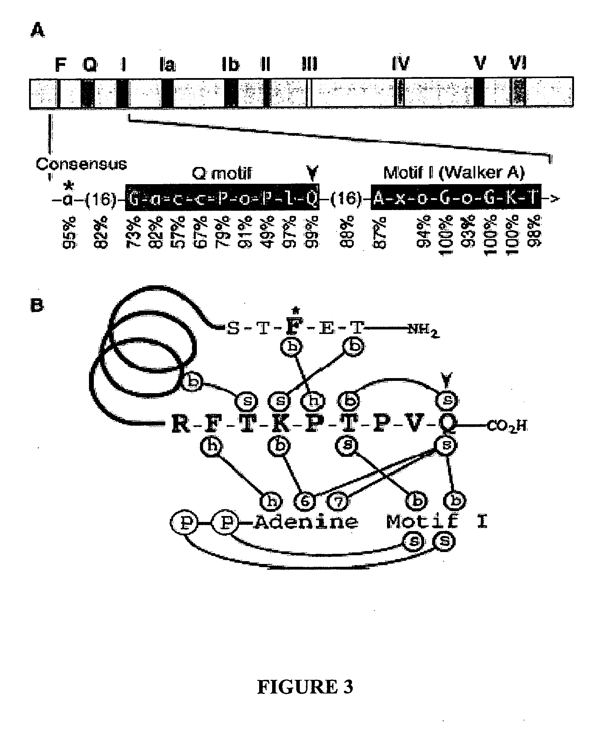

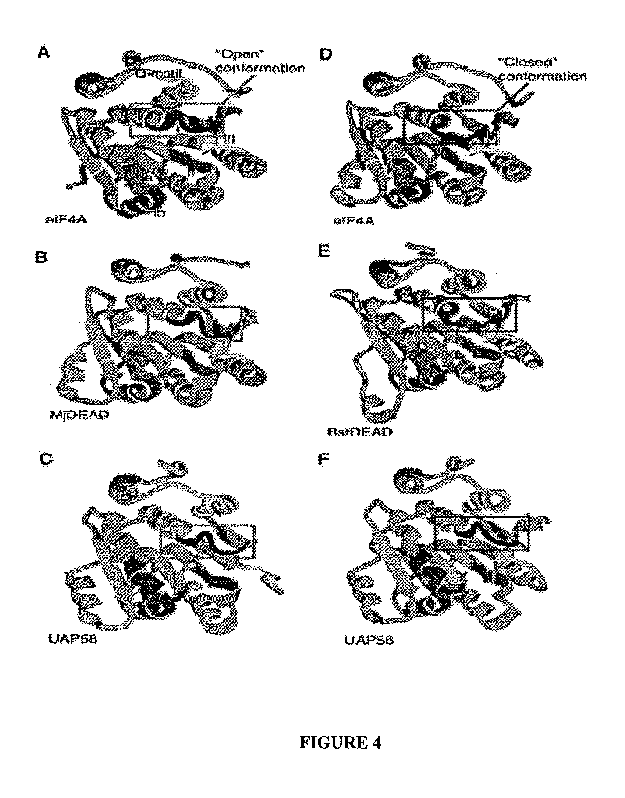

- Motif Q (SEQ ID NO: 1) : découvert très récemment, le motif Q semble être une particularité des protéines à boîte DEAD (Tanner, 2003). Ce motif consiste en neuf acides aminés parmi lesquels on trouve invariablement une glutamine (voir Figures 1 et 3A) d'où le nom du motif, auxquels s'ajoute un résidu aromatique phénylalanine (F) isolé, très conservé, situé en amont. La séquence consensus du motif Q est G-a-x-c-P-o-h-i-Q, où « a » représente F, W ou Y, « x » représente n'importe quel acide aminé, « c » représente D, E, H, K ou R, « o » représente S ou T, « h » représente A, F, G, I, L, M, P, V, W ou Y, et « i » représente I, L ou V. Les structures cristallines des protéines eIF4A et BstDEAD, obtenues en l'absence de ligand, ne montrent pas d'interactions entre le motif Q et le motif I (voir Figures 4D et E), alors que les structures de la protéine eIF4A, en présence d'une molécule ADP, ou la protéine MjDEAD, en présence d'un ion sulfate, montrent plusieurs interactions entre le motif Q et le motif I (voir Figures 4A et B ; Tanner *et al.*, 2003 ; Cordin *et al.*, 2004). En effet, la glutamine et le résidu alcool (Thr) forment une liaison hydrogène avec la première glycine conservée et le résidu alcool (Thr) du motif I. De plus, le motif Q peut établir directement des interactions avec le nucléotide lié (voir Figure 2B). La glutamine conservée forme une liaison hydrogène avec les positions N6 et N7 de la base adénine ; de même le résidu aromatique forme une liaison hydrogène avec la base adénine. Le motif Q est ainsi un motif de reconnaissance de la base adénine, ce qui a pour conséquence que les protéines à boîte DEAD ne peuvent fixer que de l'ATP (Tanner *et al.*, 2003 ; Tanner, 2003). Le motif Q est aussi nécessaire pour la liaison de l'ARN simple brin ainsi que pour les changements de conformation provoqués par la liaison à et l'hydrolyse de l'ATP ; de ce fait, il a été proposé que le motif Q fonctionne comme régulateur de l'activité ATPase en stimulant cette activité uniquement quand le substrat est correctement lié au niveau de la protéine (Cordin *et al.*, 2004).

- Motif I (SEQ ID NO: 2) : le motif I ou le motif Walker A existe dans toutes les hélicases ainsi que dans plusieurs NTPases ou ATPases (Gorbalenya et *al.*, 1989 ; Walker *et al.*, 1982). Sa séquence consensus est A-x-o-G-o-G-K-T, où « x » représente n'importe quel acide aminé, et « o » représente indépendamment S ou T (voir Figure 1). Ce motif intervient dans la liaison du NTP par la formation d'une liaison hydrogène avec les phosphates $\beta$ et $\gamma$ du nucléotide et interagit avec l'ion $Mg^{2+}$ (Caruthers and McKay, 2002). Les structures cristallines des protéines à boîte DEAD obtenues en l'absence de ligand montrent que la lysine conservée interagit avec le premier aspartate et le glutamate du motif II (Johnson and McKay, 1999 ; Carmel and Matthews, 2004), alors qu'en présence d'ATP il interagit uniquement avec le glutamate (Benz *et al.*, 1999 ; Story *et al.*, 2001). La comparaison des structures cristallines d'eIF4A et MjDEAD obtenues en présence d'une molécule ADP ou d'un ion sulfate lié au niveau du site de liaison du nucléotide respectivement, avec les structures cristallines d'eIF4A, BstDEAD ou UAP56 obtenues en l'absence de ligand montre une architecture particulière du motif I. En présence de ligand, le motif I adopte une conformation dite « ouverte » alors qu'en l'absence de ligand, il adopte une conformation dite « fermée » qui inhibe de façon stérique la liaison à l'ATP (Benz *et al.*, 1999 ; Story *et al.*, 2001 ; Zhao *et al.*, 2004) (voir Figure 4). Ce changement de conformation au niveau du motif I semble être propre aux hélicases à boîte DEAD. En effet les structures cristallines des protéines de la superfamille SF1 : PCRA, UVRB et Rep (Korolev *et al.*, 1997 ; Subramanya et *al.*, 1996 ; Theis *et al.*, 1999 ; Velankar et *al.*, 1999) ou celle de la partie hélicase de la protéine NS3 de l'hépatite C (Kim *et al.*, 1998 ; Yao *et al.*, 1997) obtenues en présence ou en absence de ligand montre que le motif I adopte toujours la conformation « ouverte ». Des études par mutagenèse ont montré que ce motif est important pour les activités ATPase et de liaison à l'ATP. Des mutations de l'alanine en première position, de la lysine et de la thréonine abolissent l'activité ATPase (Barhoumi *et al.*, 2006 ; Cordin *et al.*, 2004 ; Pause and Sonenberg, 1992 ; Rocak *et al.*, 2005 ; Rozen *et al.*, 1990 ; Tanner *et al.*, 2003).

- Les motifs Ia (SEQ ID NO: 3), Ib (SEQ ID NO:4) et le doublet GG : ces motifs font partie du domaine 1 mais ils ne participent pas directement à la liaison et à l'hydrolyse de l'ATP. Les études biochimiques réalisées sur la protéine eIF4A (Rogers *et al.*, 2002) ainsi que celles de la structure cristalline de la partie hélicase de la protéine NS3 de l'hépatite C obtenue en présence d'un poly dU (voir Figure 2A ; Kim *et al.*, 1998) montrent que ces motifs sont nécessaires pour la liaison à l'ARN. La substitution de l'alanine du motif Ia chez certaines protéines à boîte DEAD abolit les activités ATPase et hélicase (Svitkin *et al.*, 2001). Par contre, les mêmes mutations chez des protéines à boîte DEAH comme Prp22 n'affectent pas la croissance des souches de levure (Shneider *et al.*, 2004). En 1989, Linder et ses collaborateurs ont montré que les deux glycines font partie des éléments conservés qui définissent la famille des protéines à boîte DEAD (Linder *et al.*, 1989). D'un point de vue structural, ces deux glycines sont localisées au niveau de la boucle située entre les motifs Ia et Ib (voir Figures 2B et C), ainsi elles facilitent la formation d'un « sharp turn » dans cette boucle. Il a été proposé qu'elles participent aux interactions protéine-protéine telles que la liaison entre eIF4A et eIF4G (Benz *et al.*, 1999). Des mutations de ces deux glycines sont létales pour la levure (Schmid and Linder, 1992).

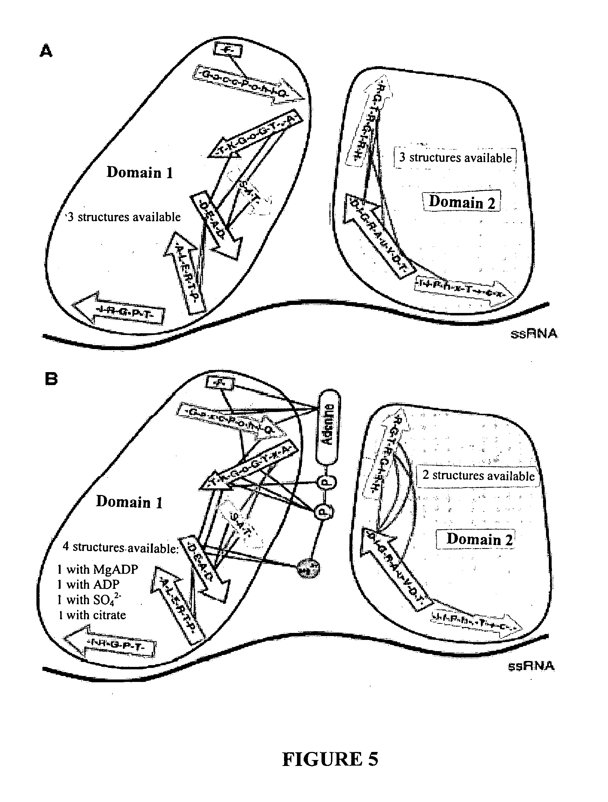

- Motif II DEAD (SEQ ID NO: 5) : ce motif représente une version du motif Walker B des protéines liant l'ATP (Walker *et al.*, 1982). La séquence consensus du motif pour les superfamilles SF1 et SF2 est DExx. Chez les protéines à boîte DEAD, la séquence du motif II (DEAD) est dominante pour la famille entière (voir Figure 1 ; Linder *et al.*, 1989). Les résidus DE sont conservés dans toutes les protéines à boîte DEAD, des mutations dans l'un de ces deux résidus réduisent ou inactivent les activités ATPase et hélicase sans altérer la liaison à l'ARN (Iost *et al.*, 1999 ; Pause and Sonenberg, 1992). Les structures cristallines des protéines à boîte DEAD montrent que le motif Walker B interagit avec les phosphates du NTP en position β et γ par l'intermédiaire d'un ion $Mg^{2+}$. Story et ses collègues ont proposé que la liaison de l'ATP ferme la fissure entre les deux domaines 1 et 2, rapprochant ainsi le dernier aspartate du motif II (de 13Å à 5Å ) à l'histidine conservée du motif VI. Le même aspartate forme une liaison hydrogène avec les acides aminés sérine et thréonine du motif III (voir Figure 5 ; Benz *et al.*, 1999 ; Caruthers *et al.*, 2000 ; Shi *et al.*, 2004 ; Story *et al.*, 2001).

- Motif III SAT (SEQ ID NO: 6) : des mutations dans le motif SAT (Ser-Ala-Thr) de la protéine eIF4A et certains protéines homologues à boîte DEAH abolissent l'activité ARN hélicase sans altérer la fixation à et l'hydrolyse de l'ATP ainsi que la liaison à l'ARN (Pause and Sonenberg, 1992 ; Schwer and Mezaros, 2000). Récemment, il a été montré que les substitutions de la sérine et de la thréonine par une alanine de la protéine à boîte DEAD Has1 de la levure dissocie partiellement les activités hélicase et ATPase (Rocak *et al.*, 2005). Dans la structure cristalline de la partie hélicase de la protéine NS3 de l'hépatite C (Kim *et al.*, 1998, voir Figure 2A), le motif III apparaît comme une partie du lien flexible entre les domaines 1 et 2, alors que dans les structures cristallines des protéines à boîte DEAD : MjDEAD (Story *et al.*, 2001), BstDEAD (Carmel and Matthews, 2004) et UAP 56 (Zhao *et al.*, 2004), il appartient au domaine 1 et il est séparé du domaine 2 par une structure hélice-boucle-feuillet β. Comme déjà signalé auparavant, le motif III interagit avec le motif DEAD en présence ou en l'absence du nucléotide. Contrairement à ce qui est observé pour la protéine PcrA de la superfamille SF1 (Subramanya *et al.*, 1996 ; Velanker *et al.*, 1999), le motif III des protéines à boîte DEAD n'interagit pas avec l'ATP lié. Korolev et ses collègues proposent que le motif III des protéines de la superfamille SF1, sert de relais qui transmet les effets de la fixation à et de l'hydrolyse de l'ATP aux motifs de liaison de l'ARN IV et V (Korolev *et al.*, 1997). Probablement, le motif III exerce la même fonction chez les protéines à boîte DEAD.

- Motif IV (SEQ ID NO: 7) : les structures cristallines établies sur ce motif montrent que ce motif est localisé dans la partie C-terminale du domaine 2 dans une région où la protéine NS3 se lie à l'oligo-dU. Toutes les données structurales obtenues pour les hélicases de la superfamille SF2 suggèrent un mode commun de liaison à l'acide nucléique via le squelette phosphodiester et la capacité des deux dernières arginines du motif IV de la protéine eIF4A à se lier à l'ARN simple brin (Caruthers and McKay, 2002). Les premiers résidus du motif V sont très proches du résidu hydrophobe du motif IV (voir Figure 5 ; Caruthers *et al.*, 2000 ; Shi *et al.*, 2004 ; Story *et al.*, 2001), ce qui suggère une communication entre les motifs IV et V. Récemment, Banroques et ses collaborteurs, par des études de mutagenèse, ont montré que le résidu conservé phénylalanine du motif IV est nécessaire pour la coopération entre la fixation à l'ARN et l'hydrolyse de l'ATP (Banroques *et al.*, 2008).

- Motif V (SEQ ID NO: 8) : le motif V, en association avec les motifs Ia, Ib et IV, est impliqué dans la liaison à l'ARN. Il est localisé au niveau de la boucle reliant les domaines 1 et 2 dans la région de liaison de l'ARN (voir Figures 1 et 4). Sa séquence consensus est T(D/N)xxARGiD, où « x » représente indépendamment n'importe quel acide aminé, et « i » représente I, L ou V (voir Figure 1). La structure cristalline d'eIF4A (voir Figure 2B ; Caruthers *et al.*, 2000) montre que l'arginine conservée du motif V interagit avec le motif II ou avec le nucléotide lié. D'autre part, le dernier aspartate interagit directement avec le ribose de l'ATP (Caruthers *et al.*, 2000 ; Caruthers and McKay, 2002). Les structures cristallines établies pour des protéines à boîte DEAD, en présence ou en l'absence du ligand, montrent des interactions différentes (voir Figure 5). En effet, dans les structures cristallines établies en présence du ligand pour les protéines MjDEAD et UAP56, le dernier aspartate interagit avec la première ou la seconde arginine, ou la thréonine du motif VI (Johnson and McKay, 1999 ; Shi *et al.*, 2004 ; Story *et al.*, 2001 ; Zhao *et al.*, 2004). Dans la structure cristalline établie pour le fragment C-terminal de la protéine eIF4A, le premier aspartate interagit avec la première arginine du motif VI (Johnson and McKay, 1999). Cependant, dans celle de la protéine UAP56 cristallisée en présence de l'ADP, le dernier aspartate du motif V interagit avec la première arginine du motif VI (voir Figure 5 ; Shi *et al.*, 2004). La comparaison de la structure du motif V chez les protéines à boîte DEAD à celle de la partie hélicase de la protéine NS3 de l'hépatite C montre que les deux motifs adoptent le même repliement (« folding »). Par conséquent, comme pour la protéine NS3, le motif V des protéines à boîte DEAD serait impliqué dans la régulation de l'hydrolyse de l'ATP par la transmission du signal de la liaison à l'ARN au domaine ATPase. Des mutations dans le motif V de la protéine à boîte DEAD Prp28 inhibent considérablement la croissance des souches de levure, suggérant que les deux arginines conservées et l'aspartate jouent un rôle important dans l'activité *in vivo* de la protéine prp28 (Chang *et al.*, 1997).

- Motif VI (SEQ ID NO: 9) : le motif VI (HRiGRzGR ; où « i » représente indépendamment I, L ou V et où « z » représente T, G ou S) des protéines à boîte DEAD est localisé à l'interface entre les deux domaines 1 et 2 (voir Figure 2 et 5) ; il est important pour les activités ATPase et la liaison à l'ARN. Des mutations au niveau des résidus conservés arginine ou histidine réduisent considérablement la liaison à l'ARN et l'activité ATPase et par conséquent abolissent l'activité hélicase (Pause *et al.*, 1993 ; Rogers *et al.*, 2002). La structure cristalline d'eIF4A montre que le résidu histidine interagit avec le deuxième aspartate du motif II. Les mêmes interactions sont observées entre la glutamine homologue du motif V et l'histidine du motif II des protéines à boîte DExH, UvrB et NS3 (Yao *et al.*, 1997 ; Kim *et al.*, 1998). Caruthers et ses collègues suggèrent que la deuxième et la troisième arginine peuvent se lier au phosphate-γ de l'ATP (Caruthers *et al.*, 2000).

Les protéines à boîte DEAD sont, comme les autres hélicases, capables de fixer et d'hydrolyser un nucléotide triphosphate. Dans le cas des protéines à boîte DEAD, seul l'ATP peut être fixé et hydrolysé à cause des interactions spécifiques entre le motif Q et la base adénine (Tanner *et al.*, 2003 ; Tanner, 2003). Les différentes études biochimiques menées sur ces protéines ont montré que leur affinité pour l'ATP est modérée, avec un Km généralement compris entre 80 et 1000 μM (Lorsh and Herschlag, 1998a). De même, leurs constantes cinétiques sont variables de 3-6 min$^{-1}$ (eIF4A : Lorsh and Herschlag, 1998a ; Has1 : Rocak *et al.*, 2005) à 600 min$^{-1}$ (Ded1 : Iost *et al.*, 1999 ; DbpA : Kossen and Uhlenbeck, 1999). La signification de ces différences n'est pas claire et il est possible que l'absence des modifications post-traductionnelles, l'absence de partenaire ou encore l'absence de substrats spécifiques en soit à l'origine. La majorité des protéines à boîte DEAD n'hydrolysent l'ATP qu'en présence de l'ARN. Cette activité est influencée parfois par la nature (séquence et/ou la longueur) du substrat ARN. En effet, la stimulation de l'activité ATPase des protéines à boîte DEAD d'*E. coli* SrmB, RhlE et CsdA dépend de la longueur de l'ARN utilisé comme substrat (Bizebard *et al.*, 2004). La majorité des protéines à boîte DEAD ne présente pas de spécificité du substrat ARN, *in vitro.* En fait, la spécificité pour un ARN donné semble plutôt être apportée par des motifs ou domaines situés à l'extérieur du coeur hélicase. Dans le cas de la protéine à boîte DEAD d'*E. coli* DbpA, son activité ATPase est stimulée par l'ARN 23S, plus précisément par un fragment de 153 nucléotides contenant l'hélice 92 du domaine V de cet ARN (Fuller-Pace *et al.*, 1993 ; Tsu and Uhlenbeck, 1998 ; Tsu *et al.*, 2001). La protéine DbpA interagirait spécifiquement avec l'hélice 92 via son domaine C-terminal et non spécifiquement avec la région adjacente via son coeur hélicase (Tsu *et al.*, 2001 ; Kossen *et al.*, 2002). Les protéines à boîte DEAD sont considérées comme des hélicases à ARN, bien que cette activité n'ait été démontrée que pour certaines d'entre elles. Comme les ADN hélicases, les protéines à boîte DEAD sont capables de dérouler des duplexes constitués d'au moins un brin d'ARN soit de 5'vers 3' soit de 3' vers 5', lorsqu'elles sont testées *in vitro.* La plupart de ces protéines à boîte DEAD nécessite la présence de régions ARN simple brin soit en 5' soit en 3' de la région appariée, probablement pour y être chargée (Rocak and Linder, 2004). La protéine eIF4A est capable de dérouler de courts duplexes ARN/ARN ou ARN/ADN ; par contre, elle est incapable de dérouler les duplexes ADN/ADN. Par ailleurs, elle est aussi capable de dérouler des duplexes d'ARN à bouts francs, suggérant qu'elle peut interagir directement avec l'ARN double brin (Rogers *et al.*, 2001a). Cette propriété est observée aussi pour la protéine à boîte DEAD d'*E. coli* RhlE (Bizebard *et al.*, 2004). Certaines protéines à boîte DEAD, comme eIF4A, p68 et RhlE sont dites bidirectionnelles, car, *in vitro*, elles sont capables de dérouler indifféremment des duplexes portant une extrémité simple brin en 3' ou en 5' (Bizebard *et al.*, 2004 ; Huang and Liu, 2002 ; Rogers *et al.*, 2001a). Les protéines à boîte DEAD comme la majorité des hélicases sont très peu processives. Seules les protéines p68 (162 pb duplex, Hirling *et al.*, 1989), p72 (36 à 46 pb, Rossler *et al.*, 2001) et RhlE (Bizebard *et al.*, 2004) présentent une activité modérément processive. L'activité hélicase dépend de la stabilité du duplex utilisé. La stabilité des duplexes fréquemment utilisés est ΔG° comprise entre -15 et -30 Kcal/mol et la longueur varie entre 10 et 25 pb. Rogers et ses collègues, en utilisant des duplexes de longueur et stabilité variables, ont montré que la protéine eIF4A est capable de dérouler quelques paires de base (Rogers *et al.*, 2001a). Récemment, Bizebard et ses collaborateurs ont montré que la longueur de la région simple brin, dont la taille minimale varie selon les protéines, est importante pour l'activité hélicase (Bizebard *et al.*, 2004).

Certaines protéines hélicases présentent, *in vitro,* une activité d'appariement de séquences homologues. Ainsi, en plus de leur activité hélicase, les deux protéines à boîte DEAD p68 et p72, sont capables, *in vitro*, de catalyser l'appariement de brins complémentaires, quand elles sont présentes en excès par rapport au substrat (Rossler *et al.*, 2001). En combinant ces deux activités, elles peuvent catalyser, *in vitro,* des réarrangements de structures secondaires d'ARN, qui sont par ailleurs trop stables pour être éliminées par leur seule activité hélicase (Rossler *et al.*, 2001). Flores-Rozas et Hurwitz ont montré qu'en présence d'analogues non hydrolysables de l'ATP, la protéine à boîte DEAD II/Gu catalyse une réaction d'appariement intramoléculaire. La concentration en ATP joue un rôle primordial dans cette réaction. A faible concentration, l'ARN hélicase II/Gu favorise la formation de duplex intramoléculaire, tandis qu'à des concentrations élevées en ATP, l'enzyme agit comme ARN-hélicase et élimine les structures secondaires (Flores-Rozas and Hurwitz, 1993). Cette activité d'appariement réside en dehors du coeur hélicase dans le domaine C-terminal, riche en Gly-Arg (Valdez *et al.*, 1997). Ceci pourrait être aussi le cas des protéines à boîte DEAD p68 et p72 qui possèdent des domaines N-terminaux et C-terminaux riches en glycine et arginine (Lamm *et al.*, 1996). Les activités d'appariement et ARN-hélicase sont séparées physiquement et fonctionnellement. Des mutations dans les motifs II et III de la protéine à boîte DEAD II/Gu abolissent l'activité ARN-hélicase sans altérer l'activité d'appariement. D'autre part, la délétion du domaine d'appariement n'inhibe pas l'activité hélicase de l'enzyme (Valdez *et al.*, 1997 ; Valdez, 2000). La protéine eIF4B qui stimule les activités hélicase et ATPase de la protéine eIF4A (Rogers *et al.*, 2001b) possède aussi l'activité d'appariement (Altmann *et al.*, 1995), ce qui prouve encore que les deux activités sont physiquement séparées.

Différents modèles ont été proposés pour l'activité hélicase (Tanner and Linder, 2001 ; Caruthers and McKay, 2002 ; Singleton and Wigley, 2002). Les deux modèles les plus discutés sont : « inchowrm » et « active rolling ».

Les protéines à boîte DEAD interviennent aussi dans tous les processus du métabolisme de l'ARN (transcription, maturation, transport, traduction, biogenèse des ribosomes, interférence à ARN, stabilité et dégradation des ARN).

Au cours de la transcription, plusieurs hélicases s'associent aux transcrits. Toutefois, elles ne semblent jouer un rôle actif que pendant les étapes tardives de l'épissage ou du transport (Linder and Stutz, 2002 ; Silverman *et al.*, 2003). Des études récentes ont montré que certaines protéines à boîte DEAD participent à la régulation de la transcription en interagissant avec d'autres facteurs de transcription (Gillian and Svaren, 2004 ; Yan *et al.*, 2003). Cependant, l'utilité des activités ATPase et hélicase dans le processus de transcription n'est pas clairement établie. Toutefois, Wilson et ses collaborateurs ont montré que les coeurs hélicase des protéines à boîte DEAD p68 et p72 répriment la transcription alors que leur région C-terminale stimule la transcription (Wilson *et al.*, 2004). Récemment, Bates et ses collaborateurs (2005) ont montré que la protéine à boîte DEAD p68 active la transcription du p53 suite à une aberration d'ADN (Bates *et al.*, 2005).

L'épissage est un processus qui se déroule en plusieurs étapes impliquant deux réactions de trans-estérification et des réarrangements structuraux dans l'étape d'assemblage du splicéosome qui nécessite l'énergie provenant de l'hydrolyse du NTP. Le rôle exact des ARN hélicases dans l'assemblage du splicéosome n'est pas connu, mais il est généralement admis qu'elles interviennent pour dérouler les petits duplexes formés entre snARN et pré-ARNm, ce qui est le cas de la protéine à boîte DExH, Prp 22. Un mutant dans le motif III de cette protéine présente un phénotype léthal à des températures inférieures à 30°C et un ralentissement de la croissance à 34°C et 37°C. *In vitro*, la protéine mutante est capable d'hydrolyser l'ATP, mais est incapable de dérouler des duplexes d'ARN et de libérer l'ARNm du splicéosome, suggérant que la perte de l'activité hélicase est la cause du phénotype léthal. Une seconde mutation dans le motif Ib, qui restaure l'activité hélicase et la dissociation de l'ARNm du splicéosome, se comporte comme un suppresseur intragénique, suggérant que l'activité hélicase est nécessaire à cette étape (Schwer and Meszaros, 2000). La majorité des protéines à boîte DEAD qui sont impliquées dans le processus de l'épissage de l'ARNm interviennent au niveau des étapes précoces de l'assemblage de splicéosome, ce qui est le cas de la protéine à boîte DEAD Prp5. Récemment, il a été rapporté que cette protéine est capable de se lier au snARN U1 et U2 (Xu *et al.*, 2004). La protéine à boîte DEAD Prp5 utilise l'énergie provenant de l'hydrolyse de l'ATP pour réarranger les interactions locales ARN-ARN ou ARN-protéine, qui permettent au complexe snARN U2 de rejoindre le complexe snARN U1. La protéine à boîte DEAD Prp28 utilise l'énergie libérée par l'hydrolyse de l'ATP pour déstabiliser le complexe snARN U1 et le remplacer par le complexe snARN U6 au niveau du site d'épissage (Staley and Gutherie, 1999). La protéine à boîte DEAD p68 est impliquée dans la dissociation du complexe snARN U1 à partir du site 5', nécessaire à l'épissage selon un mécanisme ATP dépendant.

Une large proportion des ARN hélicases à boîte DEAD participent à la biogenèse des ribosomes, dans laquelle leur activité hélicase ou RNPase pourrait permettre une régulation fine de l'organisation des multiples interactions ARN-ARN ou ARN-protéine transitoirement établies au cours de la biogenèse et de la maturation des ARN ribosomiques (Luking *et al.*, 1998 ; Kressler *et al.*, 1999 ; Rocak and Linder, 2004). Seule la protéine à boîte DEAD Has1 est impliquée à la fois dans la biogenèse des sous-unités 40S et 60S (Emry *et al.*, 2004). Des mutations dans le motif I de la protéine Has1, de même que sa déplétion affectent la biosynthèse de l'ARNr 18S des sous-unités 40S, en ralentissant, ou inhibant les clivages aux sites A0, A1 et A2.

Le transport des ARNm du noyau vers le cytoplasme à travers les pores nucléaires implique des protéines spécifiques qui se lient à l'ARNm naissant. Les ARN hélicases qui interviennent dans ce processus pourraient remodeler les complexes mRNP avant ou au cours du passage à travers les pores nucléaires (Silverman *et al.*, 2003). Elles pourraient aussi intervenir pour dissocier des facteurs nucléaires de l'ARNm pour permettre l'export de ce dernier, ou préparer le messager au premier cycle de la traduction (Rocak and Linder, 2004). La protéine à boîte DEAD Dbp5 participe à ce processus. Des expériences de double hybride et de co-immunoprécipitations ont permis d'identifier plusieurs partenaires parmi lesquels, Gle1, un facteur d'export de l'ARN associé au pore nucléaire, et GFd1/ymr255, un facteur qui interagit à la fois avec Dbp5 et GLe1 (Tseng *et al.*, 1998 ; Hodge *et al.*, 1999 Schmitt *et al.*, 1999). Les protéines Dbp5 de levure et humaine interagissent également avec Nup159, un composé du complexe du pore nucléaire (Zhao *et al.*, 2002). Chez la levure, l'activité ATPase de la protéine à boîte DEAD Sub2 est nécessaire pour son détachement de l'ARNm et son remplacement par le facteur de transport des ARNm Mex 67 (Strasser and Hurt, 2001).

La dégradation médiée des non-sens (Non Sense mediated Decay ou NMD) est un mécanisme de surveillance qui dégrade les ARNm contenant un codon de terminaison prématuré (PTC). Plusieurs protéines qui font partie du complexe de jonction d'exon sont impliquées dans ce mécanisme (Reed and Hurt, 2002). Récemment, Ferraiuolo et ses collègues (2004) ont montré que la protéine à boîte DEAD eIF4AIII appartient au complexe de jonction d'exon (EJC) et qu'elle se lie à l'ARN durant l'épissage (Chan et *al.*, 2004 ; Ferraiuolo *et al.*, 2004 ; Shibuya *et al.*, 2004). La protéine eIF4AIII interagit avec eIF4G et eIF4B mais contrairement à eIF4AI et eIF4AII, elle inhibe la traduction (Li *et al.*, 1999). Son rôle dans le noyau n'est pas encore établi mais certains auteurs proposent un rôle dans le transport de l'ARN (Palacios *et al.*, 2004) et dans la dégradation médiée des non-sens (Ferraiuolo *et al.*, 2004 ; Palacios *et al.*, 2004 ; Shibuya *et al.*, 2004).

La protéine à boîte DEAD Ded1 intervient aussi dans l'initiation de la traduction, vraisemblablement indépendamment d'eIF4A (Chuang *et al.*, 1997 ; de la Cruz *et al.*, 1997 ; Iost *et al.*, 1999 ; Linder, 2003). Le rôle exact de Ded1 dans le processus d'initiation de la traduction n'est pas encore connu, mais elle pourrait intervenir au cours du processus de déplacement de la petite sous-unité 40S jusqu'au codon d'initiation pour éliminer des structures secondaires sur l'ARNm ou des appariements fortuits entre ARNm et ARNt, ou des interactions codon/anticodon imparfaites (Linder, 2003). Une mutation dans le coeur hélicase de la protéine Ded1 inhibe sélectivement la traduction de la polymérase 2A, supprimant ainsi la réplication du génome ARN2 du virus de la mosaïque du brome alors qu'elle assure la traduction générale de la cellule. Ceci suggère que la protéine Ded1 pourrait avoir des fonctions régulatrices distinctes de son rôle général dans la traduction ou que la protéine mutante a une activité hélicase faible, néanmoins, suffisante pour assurer la traduction cellulaire mais pas la traduction de l'ARN polymérase 2A (Noueiry *et al.*, 2000).

L'utilisation des approches immunothérapeutiques visant à améliorer les réponses immunes de l'hôte aux tumeurs en développement a été démontrée. Elles incluent des cytokines immuno-modulatrices telles que le TNF alpha, les INFs de type I et de type II, l'IL-2, l'IL-12, l'IL-15 et l'IL-18 qui sont parmi les inducteurs les plus puissants de l'activité anti-tumorale selon les études précliniques. L'IL-12 en particulier fait l'objet d'une attention particulière à cause de son rôle central dans la régulation des réponses immunes innées et adaptatives. Elle peut par elle-même induire des effets anticancéreux puissants et aussi agir en synergie avec d'autres cytokines pour augmenter ses activités immunorégulatrices et antitumorales (Weiss *et al.*, 2007). Parmi les approches actuellement considérées, on peut citer l'utilisation d'IL-12 recombinante dans des modèles expérimentaux ou en essais cliniques (Shiratori *et al.*, 2007 ; Lenzi *et al.*, 2007) ; la transduction de cellules dendritiques par un adénovirus recombinant exprimant l'IL-12 et leur utilisation comme vaccin (He *et al.*, 2008) ; ou même l'utilisation de microsphères chargées par de l'ADN codant pour l'IL-12 (Son et Kim, 2007) ou par des combinaisons synergiques des cytokines IL-12 et TNF-alpha (Sabel *et al.*, 2007).

Les *Leishmania* sont des protozoaires flagellés, appartenant à l'ordre des Kinetoplastidae et à la famille des trypanosomatidae. Ils sont responsables des leishmanioses.

La protéine LeIF a été identifiée par criblage d'une banque d'ADN génomique de promastigotes de *Leishmania braziliensis* avec des sérums de patients atteints de leishmaniose cutanéomuqueuse due à *L. braziliensis* (Skeiky *et al.*, 1995). La comparaison de séquences à celles présentes dans les banques de données a permis de l'identifier comme étant homologue au facteur d'initiation de la traduction eIF4A (Skeiky *et al.*, 1995). C'est une protéine cytoplasmique de 403 acides aminés, de masse moléculaire de 45,3 KDa, dont les transcrits sont détectés aussi bien au stade promastigote que chez l'amastigote (Skeiky *et al.*, 1998 ; Salay *et al.*, 2007) et dont le gène est présent en double exemplaire dans le génome de *Leishmania* (Myler *et al.*, 1999). Des études protéomiques ont montré que la protéine LeIF est surexprimée au stade amastigote et promastigote (Bente *et al.*, 2003 ; Nugent *et al.*, 2004) ; elle est hautement conservée entre les différentes espèces du genre *Leishmania* (Barhoumi *et al.*, 2006 ; Skeiky *et al.*, 1998). Elle possède les motifs caractéristiques des protéines à boîte DEAD (Barhoumi *et al.*, 2006). Cependant, dans des expériences faites chez la levure, il a été montré que la protéine LeIF de *Leishmania* ne complémente pas pour la perte de la protéine eIF4A dans la levure mais interagit avec les facteurs d'initiation de la traduction (eIF4G) de façon non productive générant un phénotype dominant négatif (Barhoumi *et al.*, 2006).

La protéine LeIF de l'espèce *L. braziliensis* a la capacité d'induire la production d'IFN-γ et de TNF-α par des PBMC (cellule mononucléaire du sang périphérique) de patients atteints de leishmaniose cutanée, de leishmaniose muqueuse ou de leishmaniose cutanée diffuse, et d'IL-12 aussi bien par des PBMC de patients que d'individus non infectés (Skeiky *et al.*, 1995). Cette protéine est aussi capable d'induire la sécrétion des cytokines IL-12, IL-10 et TNF-α par les cellules présentatrices d'antigènes : macrophages et cellules dendritiques issus d'individus normaux (Probst *et al.*, 1997). L'activité inductrice de cytokines de la protéine LeIF semble être localisée au niveau de la région N-terminale (1-226) (Probst *et al.*, 1997). Ces résultats ont été confirmés par les mêmes auteurs dans le modèle murin. Skeiky et ses collaborateurs (1998) ont ainsi montré que la protéine recombinante LeIF de l'espèce *Leishmania major* était capable de stimuler la sécrétion d'IFN-γ par les cellules spléniques de souris BALB/c infectées par *L. major,* de favoriser le développement de clones TH1 chez des souris BALB/c immunisées avec cette protéine en l'absence d'adjuvant et enfin de stimuler la production d'IFN-γ par des splénocytes de souris SCID, par un mécanisme dépendant de l'IL-12. Ils ont aussi montré que cette activité était maintenue avec la partie N-terminale de la protéine (1-226) et perdue avec la partie C-terminale (196-403). La protéine LeIF est un des constituants d'un vaccin contre *Leishmania* de seconde génération composé de trois protéines de *Leishmania* fusionnées, qui a été décrit comme étant immunogène et conférant un degré de protection significative chez la souris immunisée par *Leishmania major* ou *L. infantum* (Coler *et al.*, 2002 ; 2007), mais qui ne semble pas être protecteur chez les souris immunisées par *L. braziliensis* (Salay *et al.*, 2007). Ce vaccin est encore en phases d'essais cliniques chez l'humain. La protéine LeIF a aussi été utilisée en immunothérapie chez un patient atteint de leishmaniose cutanéomuqueuse (Badaro *et al.*, 2001). Elle est aussi capable d'augmenter l'expression des molécules B7-1 et CD54 (ICAM-1) (molécules de co-stimulation intervenant dans l'interaction entre cellules T et cellules présentatrices d'antigènes) à la surface des macrophages et des cellules dendritiques de donneurs sains, testés *in vitro* (Probst *et al.*, 1997).

La présente invention s'est donné pour but de pourvoir à de nouvelles molécules capables de moduler la production de cytokines par les cellules hôtes de mammifères, pour traiter ou prévenir les maladies infectieuses ou les cancers notamment.

Ainsi, les Inventeurs ont utilisé des monocytes dérivés de PBMC de donneurs sains primés ou non avec l'IFN-γ pour tester la capacité de protéines recombinantes à induire la production des cytokines IL-12 (en particulier IL-12p70), IL-10 et TNF-α, de manière à démontrer leurs propriétés immunomodulatrices.

Cinq protéines recombinantes à boîte DEAD possédant des activités biochimiques ATPase et ARN hélicase, présentes chez la levure ou les mammifères, ont été testées pour leurs propriétés immunomodulatrices :

- hueIF4A : facteur d'initiation de traduction chez les mammifères, elle est supposée dérouler les structures secondaires à l'extrémité 5'UTR des ARNm pour permettre le recrutement et éventuellement le déplacement de la petite sous-unité 40S du ribosome (Rogers *et al.*, 2002 ; Sonenberg and Diver, 2003). Elle possède 406 acides aminés et une masse moléculaire de 46,1 KDa. Elle présente 56,1 % d'identité avec la protéine LeIF de *L. infantum* ;

- eIF4AIII : protéine de mammifères de 406 acides aminés et de masse moléculaire de 46,8 KDa. Elle présente 55,6 % d'identité avec la protéine LeIF de *L. infantum.* Elle est impliquée dans les processus d'épissage et la dégradation de l'ARNm (non sense mediated mRNA decay) (Chan *et al.*, 2004 ; Ferraiulo *et al.*, 2004 ; Shibuya *et al.*, 2004 ; Palacios *et al.*, 2004) ;

- yeIF4A : facteur d'initiation de la traduction chez la levure (Linder, 2003). Elle possède 394 acides aminés et une masse moléculaire de 44,5 KDa. Elle a 54,6 % d'identité avec la protéine LeIF de *L. infantum ;*

- FAL1 : protéine nucléaire de la levure. Elle possède 399 acides aminés et une masse moléculaire de 45,2 KDa. Elle présente 52,6 % d'identité avec la protéine LeIF de *L. infantum.* Elle est impliquée dans la biogenèse des ribosomes (Kressler *et al.*, 1997) ; et

- Ded1 : protéine de levure de 603 acides aminés organisés en trois parties : le coeur hélicase, une extension N-terminale de 144 acides aminés et une extension C-terminale de 110 acides aminés. Elle présente 31% d'identité avec la protéine LeIF de *L. infantum.* Elle intervient dans l'initiation de la traduction chez la levure (Chuang *et al.*, 1997 ; Kressler *et al.*, 1997 ; Iost *et al.*, 1999 ; Linder, 2003).

De manière surprenante, les Inventeurs ont montré que les protéines à boîte DEAD et notamment hueIF4A, eIF4AIII, yeIF4A, FAL1 et Ded1, bien que présentant chacune moins de 57 % d'homologie avec la protéine LeIF de *L. infantum*, possèdent aussi une activité immunomodulatrice et sont capables d'induire la sécrétion des cytokines IL-12, IL-10 et TNF-alpha.

Les Inventeurs ont aussi testé des fragments de la protéine LeIF de *L. infantum*, 26-403, 1-226, 1-237, 1-195, 25-237, 129-226, 129-261, 196-403 et 237-403, ainsi que la protéine mutante LeIFK76A présentant une substitution de la lysine en position 76 (dans le motif consensus I de la protéine LeIF de *L. infantum*) par une alanine, pour leurs propriétés immunomodulatrices.

Les Inventeurs ont montré que l'ensemble des fragments de LeIF de *L. infantum* testés induit la production d'IL-12p70 par des monocytes d'individus sains *in vitro,* et que la mutation, dans le motif I, de la lysine en position 76 en alanine qui abolit l'activité ATPase de la protéine LeIF, n'affecte pas son activité inductrice des cytokines IL-12p70, IL-10 et TNF-α.

La présente invention a donc pour objet l'utilisation d'une ARN hélicase à boîte DEAD de levure ou de mammifère de type sauvage ou présentant une mutation dans le motif consensus I (SEQ ID NO : 2), ou d'un fragment de celles-ci comprenant soit les six motifs consensus Q (SEQ ID NO : 1), I (SEQ ID NO : 2), Ia (SEQ ID NO : 3), Ib (SEQ ID NO : 4), II (SEQ ID NO : 5) et III (SEQ ID NO : 6) mais pas les trois motifs consensus IV (SEQ ID NO : 7), V (SEQ ID NO : 8) et VI (SEQ ID NO : 9), soit les trois motifs consensus IV, V et VI (définis ci-avant) mais pas les six motifs consensus Q, I, Ia, Ib, II et III définis ci-avant, pour induire la production *in vitro* ou *ex vivo* d'au moins une cytokine par une cellule mononucléaire du sang périphérique (PBMC), de préférence un monocyte, d'un mammifère.

On entend par « ARN hélicase à boîte DEAD » une protéine possédant une activité ARN hélicase et comprenant dans sa partie centrale, ordonnés de l'extrémité N-terminale vers l'extrémité C-terminale de (1) à (9), neufs motifs d'acides aminés définis par les motifs consensus suivants :

(1) motif Q, de séquence d'acides aminés G-a-x-c-P-o-h-i-Q (SEQ ID NO : 1), où « a » représente F, W ou Y, « x » représente n'importe quel acide aminé, « c » représente D, E, H, K ou R, « o » représente S ou T, « h » représente A, F, G, I, L, M, P, V, W ou Y, et « i » représente I, L ou V;

(2) motif I, de séquence d'acides aminés A-x-o-G-o-G-K-T (SEQ ID NO : 2), où « x » représente n'importe quel acide aminé, et « o » représente S ou T ;

(3) motif Ia, de séquence d'acides aminés P-T-R-E-L-A (SEQ ID NO : 3) ;

(4) motif Ib, de séquence d'acides aminés T-P-G-R-i (SEQ ID NO : 4), où « i » représente I, L ou V ;

(5) motif II, de séquence d'acides aminés D-E-A-D (SEQ ID NO : 5) ;

(6) motif III, de séquence d'acides aminés S-A-T (SEQ ID NO : 6) ;

(7) motif IV, de séquence d'acides aminés i-i-F-C/h-x-T-x-b-c (SEQ ID NO : 7), où « i » représente indépendamment I, L ou V, « h » représente A, F, G, I, L, M, P, V, W ou Y, « x » représente indépendamment n'importe quel acide aminé, « b » représente H, K ou R, et « c » représente D, E, H, K ou R ;

(8) motif V, de séquence d'acides aminés T-D/N-x-x-A-R-G-i-D (SEQ ID NO : 8), où « x » représente indépendamment n'importe quel acide aminé, et « i » représente I, L ou V ; et

(9) motif VI, de séquence d'acides aminés H-R-i-G-R-z-G-R (SEQ ID NO : 9), où « i » représente indépendamment I, L ou V et où « z » représente T, G ou S.

L'activité ARN hélicase d'une protéine peut être déterminée par toute méthode connue de l'Homme du métier. A titre d'exemple non limitatif, on peut utiliser les méthodes décrites par Rogers *et al.*, 1999 et Cordin *et al.*, 2004.

Selon un mode de mise en oeuvre avantageux, ladite ARN hélicase possède une activité ATPase. L'activité ATPase d'une protéine peut être déterminée par toute méthode connue de l'Homme du métier. A titre d'exemple non limitatif, on peut utiliser la méthode décrite par Cordin *et al.*, 2004 ; Rocak *et al.*, 2005 ou Tanner *et al.*, 2003.

L'ARN hélicase à boîte DEAD peut être :

- une protéine isolée à partir d'une levure ou d'un mammifère,
- une protéine recombinante, ou
- une protéine synthétique.

Selon un autre mode de mise en oeuvre avantageux, ladite ARN hélicase présente une mutation au niveau de la lysine en position 7 du motif I ci-dessus. De préférence, la mutation est une substitution de ladite lysine par tout autre acide aminé, de préférence, par l'alanine.

Selon un mode de mise en oeuvre préféré de la présente invention, ladite levure appartient au genre *Saccharomyces,* et de préférence à l'espèce *Saccharomyces cerevisiae.*

Selon un autre mode de mise en oeuvre préféré de la présente invention, ladite ARN hélicase à boîte DEAD de mammifère, est une ARN hélicase à boîte DEAD d'humain.

De manière avantageuse, l'ARN hélicase à boîte DEAD de levure est choisie parmi les protéines yeIF4A (SEQ ID NO : 12), FAL1 (SEQ ID NO: 13) et Ded1 (SEQ ID NO : 14), et l'ARN hélicase à boîte DEAD de mammifère est choisie parmi les protéines hueIF4A (SEQ ID NO: 15) et eIF4AIII (SEQ ID NO : 16).

D'autres protéines à boîte DEAD de levure ou humaine peuvent également être mises en oeuvre ; des exemples de telles protéines sont mentionnés ci-dessus. On peut notamment citer les protéines P68 (protéine à boîte DEAD de mammifère ; Iggo RD and Lane DP, *EMBO J.*, 1989) et Has1 (protéine à boîte DEAD de levure ; Rocack *et al.*, *N.A.R.*, 2005).

Selon un autre mode de mise en oeuvre avantageux de la présente invention, ladite cytokine est sélectionnée dans le groupe constitué par IL-12 (en particulier IL-12p70), TNF-alpha et IL-10.

Selon un mode de mise en oeuvre particulier de la présente invention, ladite cellule mononucléaire du sang périphérique est préalablement activée par l'interféron gamma (IFN-γ) pour induire la production d'IL-12.

A titre d'exemple non limitatif, l'IL-12p70 peut être produite par des monocytes pré-activés avec l'IFN-γ, de préférence pendant 12 heures, puis stimulés avec l'ARN hélicase à boîte DEAD de levure ou de mammifère telle que définie ci-dessus, de préférence pendant 24 heures.

La présente invention a aussi pour objet l'utilisation d'une ARN hélicase à boîte DEAD de levure ou de mammifère de type sauvage ou présentant une mutation dans le motif consensus I ou d'un fragment de celles-ci, tels que définis ci-dessus, pour la préparation d'un médicament destiné à traiter ou prévenir les maladies infectieuses ou les cancers.

Ledit médicament est notamment utile comme adjuvant de vaccination pour induire une réponse immunitaire de type Th1.

Ladite réponse immunitaire de type Th1 consiste de préférence en la production d'au moins l'une des cytokines IL-12 et TNF-alpha.

Plus particulièrement, les maladies infectieuses sont des infections parasitaires, de préférence des infections induites par un parasite intracellulaire et de préférence encore les leishmanioses.

La présente invention a aussi pour objet une composition pharmaceutique comprenant une ARN hélicase à boîte DEAD de levure ou de mammifère de type sauvage ou présentant une mutation dans le motif consensus I, ou d'un fragment de celles-ci, tels que définis ci-dessus, et au moins un véhicule pharmaceutiquement acceptable.

Les véhicules pharmaceutiquement acceptables sont ceux classiquement utilisés et sont choisis en fonction du mode d'administration utilisé pour le traitement envisagé.

La présente invention a aussi pour objet des produits contenant (i) une ARN hélicase à boîte DEAD de levure ou de mammifère de type sauvage ou présentant une mutation dans le motif consensus I, ou d'un fragment de celles-ci, tels que définis ci-dessus et (ii) l'IFN-γ, pour une utilisation simultanée, séparée ou séquentielle dans le traitement ou la prévention du cancer et des maladies infectieuses, de préférence les infections parasitaires, et plus préférentiellement les leishmanioses.

La présente invention a également pour objet un fragment de la protéine LeIF de *Leishmania infantum* de séquence SEQ ID NO : 11, choisi parmi :

- les fragments de celle-ci comprenant soit les six motifs consensus Q (SEQ ID NO : 1), I (SEQ ID NO : 2), Ia (SEQ ID NO : 3), Ib (SEQ ID NO : 4), II (SEQ ID NO : 5) et III (SEQ ID NO : 6) mais pas les trois motifs consensus IV (SEQ ID NO : 7), V (SEQ ID NO : 8) et VI (SEQ ID NO : 9), soit les trois motifs consensus IV, V et VI (définis ci-avant) mais pas les six motifs consensus Q, I, Ia, Ib, II et III définis ci-avant, et
- les fragments de celle-ci correspondant aux acides amines 1-195 (SEQ ID NO : 17), 1-226 (SEQ ID NO : 18), 1-237 (SEQ ID NO : 19), 25-237 (SEQ ID NO : 20), 26-403 (SEQ ID NO : 21), 129-226 (SEQ ID NO : 22), 129-261 (SEQ ID NO : 23), 196-403 (SEQ ID NO: 24), 237-403 (SEQ ID NO : 25) ou 261-403 (SEQ ID NO: 26) de ladite protéine LeIF de *L. infantum.*

La présente invention a aussi pour objet un fragment de la protéine LeIF de *Leishmania infantum* tel que défini ci-dessus, de préférence les fragments de séquence SEQ ID NO: 17, 18, 19, 20, 21, 24 et 26, ou de la protéine LeIF de *Leishmania infantum* de type sauvage (SEQ ID NO : 11) ou mutée dans le motif consensus I pour une utilisation comme médicament.

Selon un autre mode de mise en oeuvre avantageux, la protéine LeIF de *L. infantum* présente une mutation au niveau de la lysine en position 7 du motif I ci-dessus, correspondant au résidu lysine en position 76 de la protéine. De préférence, la mutation est une substitution de ladite lysine par tout autre acide aminé, de préférence, par l'alanine.

La protéine LeIF de *Leishmania infantum* mutante présentant une substitution de la lysine en position 7 du motif I défini ci-dessus par l'alanine est dénommée LeIFK76A. Sa séquence en acides aminés est représentée à la séquence SEQ ID NO : 27.

Selon un autre mode de mise en oeuvre avantageux de cette utilisation, le médicament est destiné à traiter ou prévenir les maladies infectieuses ou les cancers.

Le médicament est en outre utile comme adjuvant de vaccination pour induire une réponse immunitaire de type Th1, qui consiste de préférence en la production d'au moins l'une des cytokines IL-12 (en particulier IL-12p70) et TNF-alpha.

La présente invention a aussi pour objet des produits contenant (i) un fragment de la protéine LeIF de *Leishmania infantum* tel que défini ci-dessus, de préférence les fragments de séquence SEQ ID NO : 17, 18, 19, 20, 21, 24 et 26, ou la protéine LeIF de *Leishmania infantum* telle que définie ci-dessus et (ii) l'IFN-γ, pour une utilisation simultanée, séparée ou séquentielle dans le traitement ou la prévention du cancer et des maladies infectieuses, de préférence les infections parasitaires, de préférence encore les infections induites par un parasite intracellulaire et plus préférentiellement les leishmanioses.

La présente invention a aussi pour objet un fragment de la protéine LeIF de *Leishmania infantum* tel que défini ci-dessus, de préférence les fragments de séquence SEQ ID NO : 17, 18, 19, 20, 21, 24 et 26, ou la protéine LeIF de *Leishmania infantum* telle que définie ci-dessus pour induire la production *in vitro* ou *ex vivo* d'IL-12 par une cellule mononucléaire du sang périphérique (PBMC), de préférence un monocyte, d'un mammifère, de préférence un humain.

La présente invention a également pour objet une méthode de modulation de la production de cytokines, **caractérisée en ce qu'**elle comprend la stimulation de la production des cytokines IL-12, TNF-α et IL-10, par des cellules d'un hôte et notamment des monocytes, ladite méthode comprenant l'administration simultanée, séparée ou séquentielle des protéines à boîte DEAD ou leurs fragments, tels que définis ci-dessus, et d'IFN-γ.

Outre les dispositions qui précèdent, l'invention comprend encore d'autres dispositions, qui ressortiront de la description qui va suivre, qui se réfère à des exemples illustrant les propriétés immunomodulatrices d'ARN hélicases à boîte DEAD de levure ou de mammifère, ou de fragments de la protéine LeIF de *L. infantum*, ainsi qu'aux dessins annexés, dans lesquels :

- la **Figure 1** illustre les motifs conservés des protéines ARN hélicases à boîte DEXD/H. Cette figure illustre les séquences des motifs conservés des protéines : eIF4A de la levure (protéine à boîte DEAD), Prp22 (protéine à boîte DEAH), NS3 (hélicase du virus de l'hépatite C de la famille DECH) et SKi2 (DExH) ainsi que les espacements qui séparent les motifs. Divers symboles sont utilisés pour représenter la séquence consensus des motifs des protéines DEAD-box : - o: S, T; - l: I, L, V; - a: F, w, Y; - c: D, E, H, K, R; -h: A, F, G, I, L, M, P, V, W, Y; +: H, K, R; - u: A, G ;- .: n'importe quel résidu.
- la **Figure 2** illustre les structures cristallines des hélicases de la superfamille 2 (SF2) représentées par les protéines NS3, eIF4A et MjDEAD. (**A**) La structure cristalline de la protéine NS3 est obtenue en présence d'un oligonucléotide lié (Kim *et al.*, 1998, numéro d'accession PDB 1A1V). L'ion sulfate co-cristallisé n'est pas représenté. (**B**) la structure cristalline de la protéine entière eIF4A (Caruthers *et al.*, 2000, numéro d'accession PDB 1FUU). (**C**) la structure de la protéine MjDEAD (Story *et al.*, 2001, numéro d'accession PDB 1HV8).
- la **Figure 3** illustre le motif Q des protéines à boîte DEAD. (**A**) Conservation du motif Q des protéines à boîte DEAD (d'après Tanner *et al.*, 2003). (**B**) Représentation schématique des interactions à l'intérieur du motif Q et entre le motif Q, le motif I et l'ADP lié. La séquence est celle de la protéine Ded1 (Cordin *et al.*, 2004).

- la **Figure 4** illustre les deux conformations ouverte et fermée du motif 1 des protéines à boîte DEAD. (**A**) structure de la partie N-terminale de la protéine eIF4A obtenue en présence de l'ADP lié (Benz *et al.*, 1999; numéro d'accession PDB: 1QDE) ; l'ADP lié n'est pas montré. (**B**) structure du domaine 1 de la protéine MjDEAD (Story *et al.*, 2001 ; numéro d'accession PDB: 1HV8). (C) la structure du domaine 1 de la protéine UAP56 cristallisée en présence de l'ADP (Shi *et al.*, 2004; numéro d'accession PDB: 1XTJ). (**D**) la structure du domaine 1 de la protéine eIF4A cristallisée en l'absence du nucléotide (Caruthers *et al.*, 2000; numéro d'accession PDB: 1FUU). (**E**) la structure du domaine 1 de la protéine BstDEAD cristallisée en l'absence du nucléotide (Carmel & Matthews, 2004; numéro d'accession PDB: 1QOU). (**F**) la structure du domaine 1 de la protéine UAP56 cristallisée en absence de l'ADP (Shi *et al.*, 2004; numéro d'accession PDB: 1XTI). (**A**) à (**C**) le rectangle montre la conformation ouverte du motif I. (**D**) à (**F**) le rectangle montre la conformation fermée du motif I.
- la **Figure 5** illustre les interactions intramoléculaires des protéines à boîte DEAD en présence et en l'absence du nucléotide et représente schématiquement les interactions entre les motifs conservés dans les domaines 1 et 2 basées sur les données structurales des protéines eIF4A, MjDEAD, BstDEAD et UAP56. Les flèches indiquent l'orientation des motifs. (**A**) les interactions en l'absence du nucléotide (Caruthers *et al.*, 2000; Carmel & Matthews, 2004; Shi *et al.*, 2004; Zhao *et al.*, 2004). (**B**) les interactions entre les motifs en présence du ligand (ADP, ion sulfate ou citrate; Benz *et al.*, 1999; Story *et al.*, 2001; Shi *et al.*, 2004).

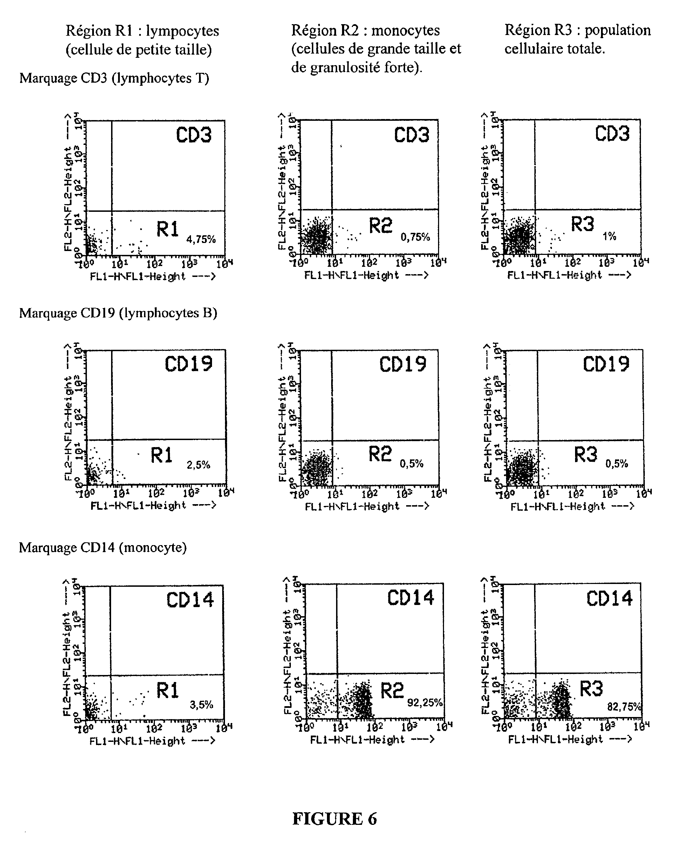

- la **Figure 6** illustre l'analyse de la pureté de la population monocytaire par cytométrie en flux. La quantité de fluorescence émise par les cellules est représentée sur les diagrammes, et déterminée par rapport à un seuil de positivité fixé grâce au contrôle isotypique. Les lymphocytes T et B (respectivement CD3$^+$ et CD19$^+$) ne dépassent pas les 2 % de la population cellulaire en R3, les monocytes sont présents à plus de 80 %, dans cette région, et représentent 92,25 % des cellules de la région R2.

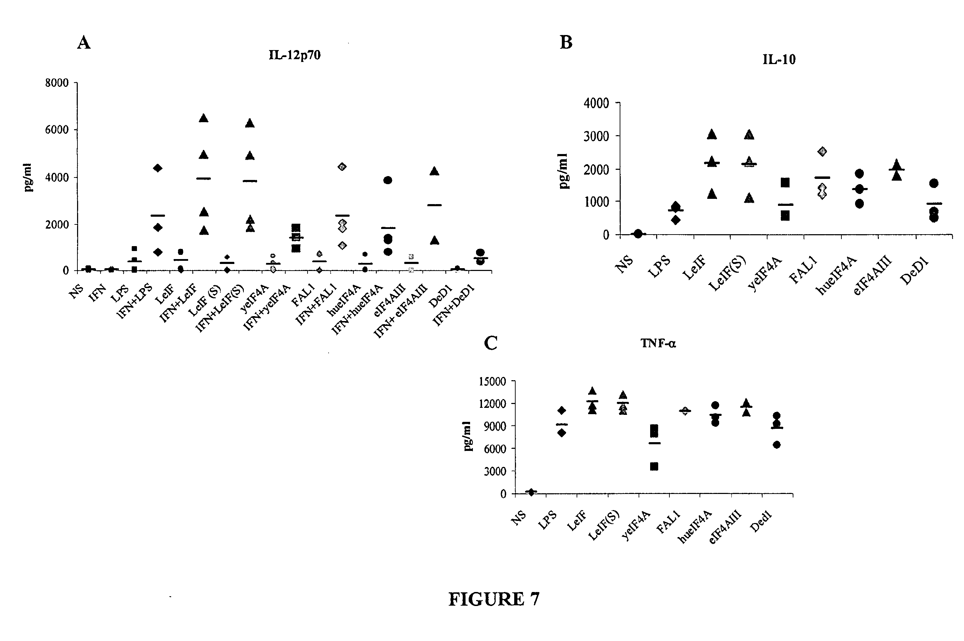

- la **Figure 7** illustre l'induction par les protéines yeIF4A, FAL1 et Ded1 de la levure et hueIF4A et eIF4AIII humaines, homologues de la protéine LeIF, de la sécrétion de cytokines IL-12p70 (**A**), IL-10 (**B**) et TNF-$\alpha$ (**C**) par les monocytes humains *in vitro*. NS: monocytes non stimulés. IFN: monocytes primés par l'IFN-$\gamma$ à une concentration finale de 3000 U/ml pendant 12 h, LPS : monocytes stimulés pendant 18h avec du LPS à 1 $\mu$g/ml.

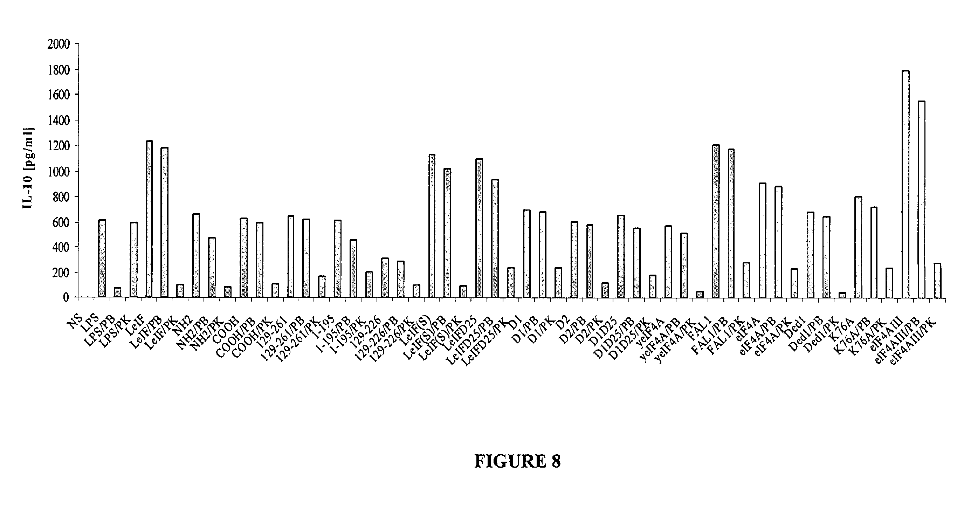

- la **Figure 8** illustre l'activité d'induction de la production des cytokines par différentes protéines recombinantes en présence de protéinase K et de polymixine B. Les monocytes purifiés à partir des PBMC d'un même individu sain ont été stimulés par les différentes protéines recombinantes seules, ou co-incubées avec la polymixine B (10 $\mu$g/ml), ou prétraitées pendant 30 min à 42°C avec la protéinase K (100 $\mu$g/ml). Comme contrôles, les monocytes ont été stimulés par le LPS (1 $\mu$g/ml) seul ou en présence de polymixine B (10 $\mu$g/ml), ou après prétraitement pendant 30 min à 42°C avec la protéinase K (100 $\mu$g/ml). Les surnageants de culture ont été collectés après 18h d'incubation et les taux d'IL-10 ont été déterminés par ELISA sandwich. NS : monocytes non stimulés.

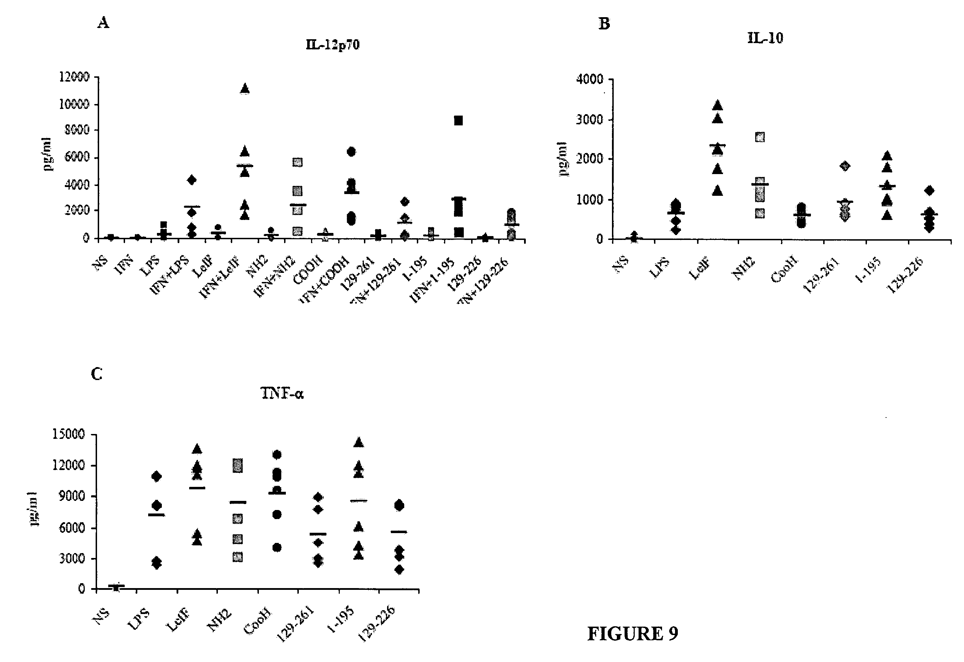

- la **Figure 9** illustre l'induction par différents fragments de la protéine LeIF de *L. infantum* de la production des cytokines IL-12p70 (**A**), IL-10 (**B**) et TNF-$\alpha$ (**C**) par des monocytes humains *in vitro*. NS : monocytes non stimulé, IFN-$\gamma$ : monocytes stimulés par l'IFN-$\gamma$ à une concentration finale de 3000 U/ml. LPS : monocytes stimulés par du LPS à 1 $\mu$g/ml, pendant 18h.

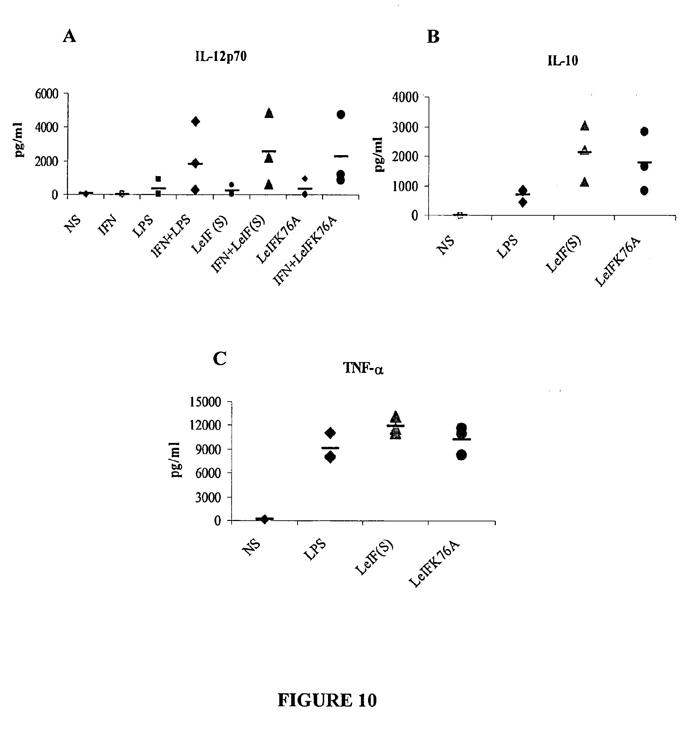

- la **Figure 10** illustre l'induction par la protéine LeIFK76A (SEQ ID NO : 27) de la production des cytokines IL-12p70 (**A**), IL-10 (**B**) et TNF-$\alpha$ (**C**) par des monocytes humains *in vitro*. NS : monocytes non stimulés, IFN: monocytes primés par l'IFN-$\gamma$ (3000 U/ml) pendant 12 h, LPS : monocytes stimulés pendant 18 h avec du LPS à 1 $\mu$g/ml

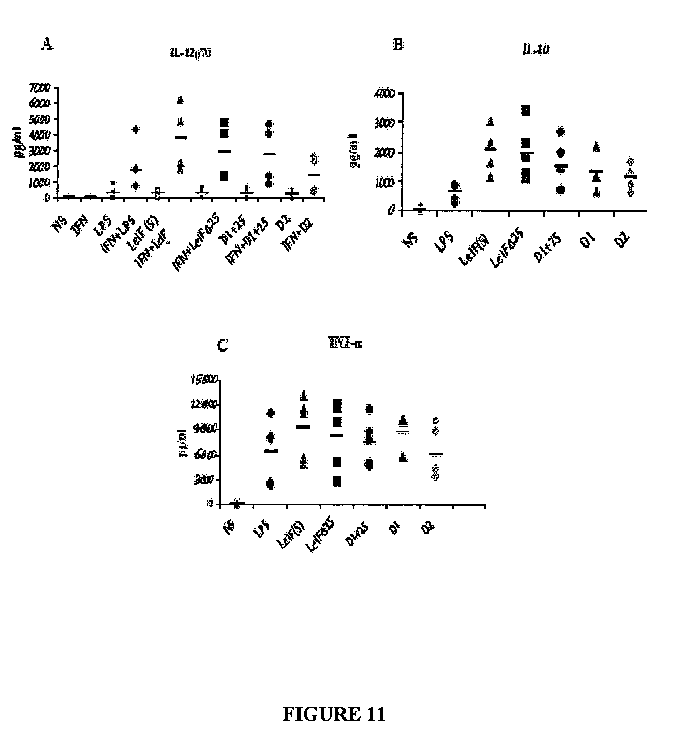

- la **Figure 11** illustre l'induction par différents domaines de la protéine LeIF de *L. infantum* de la production des cytokines IL-12p70 (**A**), IL-10 (**B**) et TNF-$\alpha$ (**C**) par les monocytes humains *in vitro*. NS : monocytes non stimulé, IFN-$\gamma$ : monocytes stimulés par l'IFN-$\gamma$ à une concentration finale de 3000 U/ml pendant 12h, LPS : monocytes stimulés pendant 18h avec du LPS à 1 $\mu$g/ml ;

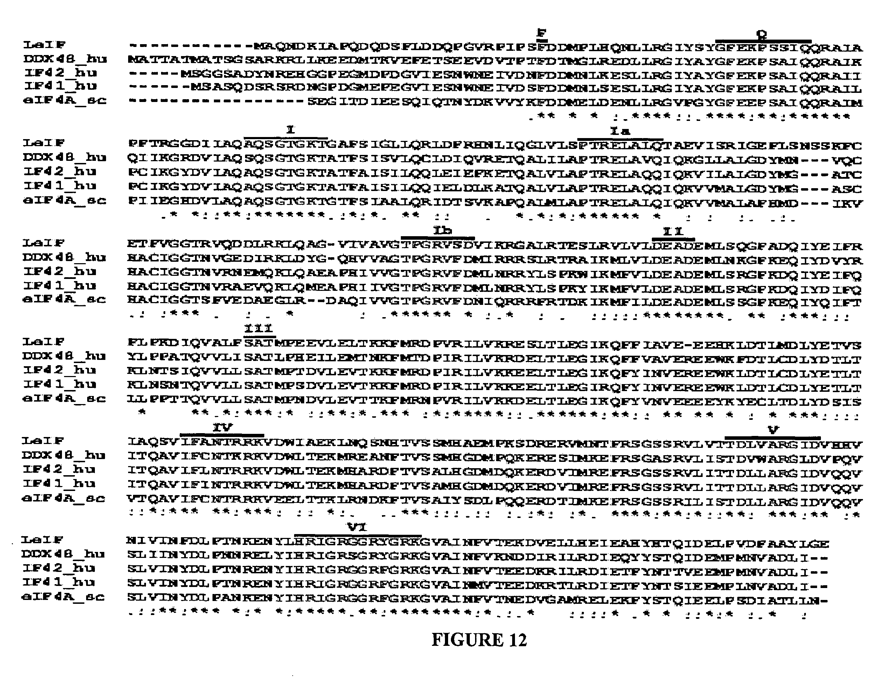

- la **Figure 12** illustre la comparaison de la séquence de la protéine LeIF de *L. infantum* avec les séquences des protéines humaines (Hu) ou de levure (Sc) suivantes : DDX48_hu, IF42_hu, IF41_hu et eIF4A_sc (telles que décrites dans Barhoumi *et al.*, 2006).

## EXEMPLE 1: EVALUATION DE L'EFFET INDUCTEUR DE LA PRODUCTION DE CYTOKINES MONOCYTAIRES PAR DIFFERENTES ARN HELICASE A BOITE DEAD DE LEVURE OU DE MAMMIFERE

### I. Matériel

#### I.1. Donneurs

Les monocytes humains ont été isolés à partir de dons de sang (Centre de transfusion sanguine de Tunis), prélevés sur anticoagulant (Citrate-phosphate-dextrose CPD) chez des volontaires adultes sains.

I.2. Protéines recombinantes

- ARN hélicases à boîte DEAD de levure : yeIF4A (SEQ ID NO : 12), FAL1 (SEQ ID NO : 13) et Ded1 (SEQ ID NO : 14) (séquences d'acides aminés disponibles respectivement sous les numéros d'accession P10081, Q12099 et P06634 dans la base de données UniProtKB/Swiss-Prot). Les séquences nucléotidiques codant ces protéines sont disponibles respectivement sous les numéros d'accession gi|83722562, gi|93117368 et gi|84626310 dans la base de données GENBANK.
- ARN hélicases à boîte DEAD de mammifère : hueIF4A (SEQ ID NO : 15) et eIF4AIII (SEQ ID NO : 16) séquences d'acides amines disponibles respectivement sous les numéros d'accession P60842 et P38919 dans la base de données UniProtKB/Swiss-Prot). Les séquences nucléotidiques codant ces protéines sont disponibles respectivement sous les numéros d'accession gi|4503528 et gi|41327777dans la base de données GENBANK.
- LeiF de *L. infantum*, isolat Aymen (SEQ ID NO : 11) (Barhoumi *et al.*, 2006). La séquence nucléotidique codant cette protéine est représentée à la séquence SEQ ID NO : 10.

## II. Méthodes

II.1.1 Isolement des cellules mononucléées (peripheral blood mononuclear cells ou PBMC)

Le sang prélevé chez des donneurs sains, sur CPD, est centrifugé à 1800 tr/min pendant 20 min. Après avoir éliminé le plasma, le sang est dilué dans un milieu RPMI 1640 additionné de 100 U/ml de pénicilline, 100 $\mu$g/ml de streptomycine et 2 mM de L-glutamine (RPMI/PS/GLU). Le plasma est décomplémenté à 56°C pendant 30 min, puis clarifié par centrifugation à 3000 tr/min pendant 10 min à température ambiante. Les cellules mononucléées (PBMC) sont isolées sur un gradient de densité constitué de ficoll hypaque (Amersham). Le sang est déposé sur le ficoll, puis centrifugé à 1600 tr/min pendant 20 min à température ambiante. Les globules rouges et les polynucléaires passent à travers le ficoll et se retrouvent dans le culot, tandis que les PBMC restent à l'interface du ficoll. L'anneau de PBMC est prélevé, dilué dans du RPMI/PS/Glu et lavé trois fois par centrifugation à 1600 tr/min pendant 10 min à température ambiante. Les cellules sont comptées sur lame de Malassez.

II.1.2. Purification des monocytes à partir des PBMC

Des flasques de culture stériles sont traitées par une solution de gélatine à 2 % par incubation pendant 2h à 37°C, puis l'excès de gélatine est aspiré et ces flasques sont placées dans un four à 56°C pendant la nuit. Ces flasques peuvent être gardées à température ambiante pendant quelques jours jusqu'à utilisation.

Le plasma autologue (PA) décomplémenté est déposé dans les flasques de culture gélatinées. Ce plasma est riche en fibronectine, protéine qui se fixe sur la gélatine et permet l'adhérence des monocytes via le récepteur pour la fibronectine CD49 exprimé à la surface de ces cellules. Après élimination du plasma de cette suspension, les PBMC à 2.10$^6$/ml sont resuspendus dans du RPMI/PS/Glu supplémenté de 5 % plasma autologue, puis sont rajoutées dans les flasques. Ces cultures sont incubées pendant 1 heure à 37°C en présence de 5 % de $CO_2$. Les cellules non adhérentes sont éliminées par trois lavages délicats, les cellules adhérentes sont décollées après une incubation de 15 min à 37°C avec un tampon contenant 50 % d'une solution de PBS 10 mM EDTA et 50 % de RPMI/10 % SVF ou PA. Les cellules décollées sont lavées pour éliminer toute trace d'EDTA. Une fraction des cellules ainsi purifiées est prélevée pour estimer la pureté de la préparation monocytaire, par cytométrie en flux.

II.1.3. Estimation de la pureté des monocytes par FACS

10$^6$ cellules sont récupérées à partir de la préparation monocytaire par adhérence, lavées et resuspendues dans du PBS 1 % BSA, 0.01 % NaN$_3$ puis réparties à raison de 2.10$^5$ cellules/tube dans des tubes à hémolyse. Des anticorps (Becton Dickinson) marqués à la fluorescéine (FITC) ou à la phycoérythrine (PE) et spécifiques des marqueurs de surface CD14 (présent sur la majorité des monocytes), CD 19 (marqueur membranaire des lymphocytes B), et CD3 (présent sur les lymphocytes T) sont rajoutés à raison de 5 $\mu$l/tube et incubés pendant 30 min à 4°C, à l'abri de la lumière. La fluorescence non spécifique de chaque anticorps qui peut se faire à travers le Fc des IgG, est contrôlée par l'incubation des cellules avec un anticorps de même isotype que l'anticorps spécifique, utilisé pour identifier les différentes populations cellulaires, et marqué au même fluorochrome que cet anticorps. Les suspensions cellulaires sont alors lavées deux fois, les culots sont resuspendus dans une solution de fixation (PBS-paraformaldehyde (PFA) 0,3 %) puis analysés par FACS.

## II.2. Induction de la sécrétion des cytokines et dosage par ELISA sandwich

### II.2.1. Stimulation in vitro

Les monocytes humain sont resuspendus à raison de $10^6$ cellules/ml dans du RPMI/PS/Glu 3% PA et répartis dans des plaques de culture à 24 puits (Nunc), à raison de $5 \times 10^5$ monocytes/puits ($10^6$ cellules/ml). Les cellules en culture sont stimulées soit avec du LPS (1 μg/ml) soit avec les différentes protéines recombinantes (10 μg/ml), pour le dosage de l'IL-10 et de TNF-α. Après 18 heures d'incubation à 37°C en présence de 5 % de $CO_2$, les cellules sont centrifugées à 3000 rpm pendant 10 min et les surnageants de culture sont récupérés et gardés à - 80°C jusqu'à utilisation.

Pour doser l'IL-12, les monocytes sont primés avec l'IFN-γ (3000 U/ml) pendant 12 heures avant l'ajout du LPS (1 μg/ml) ou des différentes protéines recombinantes (10 μg/ml). Après 24 h d'incubation, les surnageants sont collectés et gardés à -80°C jusqu'à utilisation.

Pour vérifier l'absence des contaminants d'origine bactérienne (LPS) dans les préparations de protéines recombinantes, des cultures contrôles ont été rajoutées dans les mêmes conditions expérimentales. Pour cela, le LPS (1 μg/ml) et les différentes protéines recombinantes (10 μg/ml) sont incubés en présence de la protéinase K, à une concentration finale de 100 μg/ml, à 42°C pendant 30 min. Par la suite, l'enzyme est inactivée à 75°C pendant 10 min, puis ces préparations sont utilisées pour stimuler les monocytes. La polymixine B, qui a pour effet de bloquer l'action du LPS, est rajoutée directement aux cellules, à une concentration finale de 10 μg/ml.

### II.2.2. Dosage des cytokines par le test ELISA (Enzyme Linked Immuno Sorbent Assay)

Les taux de cytokines sécrétées (IL-10, TNF-α et IL-12) dans les surnageants de culture sont quantifiés par un test ELISA de type sandwich. Un premier anticorps ou anticorps de capture, adsorbé sur le plastique d'une plaque de microtitration (Maxisorp Nunc), se lie de façon spécifique à la cytokine à doser. Un deuxième anticorps ou anticorps de détection couplé à une enzyme se fixe aux complexes anticorps de capture/cytokine. Cette fixation est détectée par une réaction enzymatique qui transforme un substrat chromogène en un produit coloré. Les taux des cytokines sécrétées dans les surnageants de culture sont déterminés à partir d'une gamme étalon tracée avec des concentrations connues d'une cytokine standard.

Le test ELISA est pratiqué selon les étapes suivantes :

- Les puits d'une plaque de microtitration sont sensibilisés avec 100 μl/puits d'anticorps monoclonaux anti-cytokine humaine purifiés (Becton dickinson) dilués à 1/250 dans du tampon bicarbonate pH 9,6 (0,1 M $NaHCO_3$ et 0,1 M $Na2CO_3$), pendant une nuit à + 4°C.
- Les anticorps non fixés sont éliminés par trois lavages avec du PBS, 0,05 % Tween 20 pH 7,0 (PBS-T).
- Les sites restés libres sont saturés par 200 μl/puits de PBS-lait écrémé 5 % pendant 1 heure à température ambiante. La plaque est lavée 2 fois avec du PBS-T.
- Les surnageants de culture et la cytokine recombinante, utilisée aux dilutions recommandés par le fournisseur (Becton Dickinson) et constituant la gamme standard (7,8, 15,6, 31,3, 62,5, 125, 250, 500 et 1000 pg/ml), sont déposés à raison de 100 μl/puits et incubés pendant deux heures à température ambiante. La plaque est lavée 5 fois avec du PBS-T.
- 100 μl d'une solution de détection sont déposés par puits. Cette solution contient l'anticorps monoclonal anti-cytokine humaine, biotinylé et l'enzyme (Avidin-Horseradish peroxidase conjugate (Avidin-HRP)), dilués dans le tampon PBS-lait écrémé (dilution au 1/500, pour l'anticorps anti-IL-10, et au 1/250 pour les anticorps anti-IL-12p70 et anti-TNF-α et au 1/250 pour l'enzyme Avidin-HRP). Après une incubation d'une heure à température ambiante, l'excès d'anticorps biotinylés est éliminé par 7 lavages avec du PBS-T.
- La révélation de l'activité peroxydase se fait par l'addition de 100 μl/puits de la solution contenant le substrat de l'enzyme : TMB préparée extemporanément. Elle contient deux réactifs à volume égal : le réactif A : contient du peroxyde d'hydrogène et le réactif B : contient le 3.3', 5.5'tétraméthylbenzidine (TMB). La solution substrat prend une coloration bleue pâle après mélange des deux réactifs et vire au bleu en présence de peroxydase. La plaque est incubée pendant 30 min à température ambiante à l'obscurité.
- Les réactions sont arrêtées en rajoutant 50 μl/puits de $H_2SO_4$ 2N. Après une incubation pendant 30 min à température ambiante, la couleur bleue vire vers le jaune et les densités optiques sont mesurées au spectrophotomètre à une longueur d'onde de 450 nm.

### II.2.3. Analyses statistiques

Le traitement des résultats a été effectué grâce à un logiciel d'analyses statistiques S plus (version 6.2) en appliquant le test Paired t-test. Ce test compare la différence des moyennes avec zéro. Il est calculé selon l'équation

suivante

$$t = \dfrac{\overline{d}}{\dfrac{s}{\sqrt{n}}} \ ,$$

où

   d : la moyenne de différence
   $S^2$ : la variance des échantillons
   n : la taille de l'échantillonnage
   t : student t.

## III. Résultats

III.1. Evaluation de la pureté des monocytes

Les monocytes humains ont été isolés à partir des PBMCs d'individus sains par adhérence sur un support gélatiné traité avec du plasma autologue. Une fraction ($10^6$) des cellules récupérées a été analysée par cytométrie en flux, pour estimer leur pureté et leur viabilité. Pour cela, les cellules ont été incubées avec des anticorps couplés à un fluochrome et reconnaissent les antigènes de surface : CD 14 (marqueur de monocytes), CD3 (marqueur des cellules T) ou CD 19 (marqueur des cellules B). La quantité de fluorescence spécifique émise par les cellules marquées est corrélée avec l'expression de ces marqueurs.

Le marquage immunofluorescent a permis d'estimer le pourcentage des monocytes et celui des lymphocytes, T et B, contaminants. La Figure 6 montre que la population cellulaire totale représentée dans la région R3, est constituée à 82,7 % de monocytes (cellules CD14[+]) et de seulement 2 % de lymphocytes T (cellules CD3[+]) et de lymphocytes B (cellules CD19[+]).

III.2. Les protéines yeIF4A, FAL1 et Ded1 chez la levure et hueIF4A et eIF4AIII de mammifères induisent la sécrétion de cytokines IL-12p70, IL-10 et TNF-α par les monocytes humains *in vitro.*

Les monocytes purifiés à partir des PBMC de 5 donneurs sains ont été activés ou non par l'IFN-γ puis stimulés par les protéines recombinantes LeIF (témoin), yeIF4A, FAL1, hueIF4A, eIF4AIII et Ded1 à des concentrations finales de 10 μg/ml.

Les résultats illustrés à la Figure 7A montrent que les protéines recombinantes yeIF4A, FAL1, hueIF4A, eIF4AIII et Ded1 induisent la production de taux d'IL-12p70 significativement plus élevés que ceux induits dans les surnageants de culture des monocytes non stimulés (34,17±17,77 pg/ml) ou stimulés par l'IFN-γ seul (35,53±17,84 pg/ml) ou encore par les protéines recombinantes seules (p<0.05, cf. Tableau 1 ci-dessous).

Les protéines yeIF4A, FAL1, hueIF4A et eIF4AIII induisent des taux d'IL-12p70 (1391,3±266,98 ; 2338,37±738,34 ; 1820,28±681,35 et 2790±1467,5 pg/ml, respectivement) comparables à ceux induits par la protéine LeIF soluble ou insoluble (3805,9±1069,1 et 3921,6±1094 pg/ml ; p>0.05, cf. Tableau 1). Cependant, la protéine Ded1 induit la sécrétion de taux d'IL-12p70 (497,6±79,19 pg/ml) significativement plus faibles que ceux induits par la protéine LeIF soluble ou insoluble (p<0,05, Tableau 1).

Le Tableau 1 ci-dessous montre l'analyse statistique des différences observées pour les taux de production d'IL-12p70 dans les surnageants de culture des monocytes stimulés par les protéines yeIF4A, FAL1 et Ded1 de la levure et eIF4AI et eIF4AIII des mammifères. Les valeurs du p déterminés par le paired t test sont indiquées.

Tableau 1 :

| | NS | IFN-γ | IFN-γ+LPS | IFN-γ+LeIF | IFN-γ+LeIF (S) | IFN-γ+yeIF4A | IFN-γ+FAL1 | IFN-γ+hueIF4A | IFN-γ+eIF4AIII |
|---|---|---|---|---|---|---|---|---|---|
| | | | | IL-12p70 | | | | | |
| IFN-γ | 0.3132 | | | | | | | | |
| IFN-γ + LPS | 0.0108 | 0.0108 | | | | | | | |
| IFN-γ+LeIF | 0.0189 | 0.0186 | 0.3049 | | | | | | |
| IFN-γ +LeIF (S) | 0.0193 | 0.0190 | 0.3373 | 0.2936 | | | | | |
| IFN-γ+yeIF4A | 0.0436 | 0.0435 | 0.1611 | 0.1343 | 0.1222 | | | | |
| IFN-γ+FAL1 | 0.0266 | 0.0266 | 0.0953 | 0.1959 | 0.2141 | 0.1145 | | | |
| IFN-γ+hueIF4A | 0.0401 | 0.0402 | 0.4967 | 0.1268 | 0.1348 | 0.2619 | 0.0104 | | |
| IFN-γ+eIF4AIII | 0.0369 | 0.0369 | 0.2633 | 0.1613 | 0.0859 | 0.1844 | 0.0719 | 0.0581 | |
| IFN-γ+Ded1 | 0.0169 | 0.007 | 0.1086 | 0.0488 | 0.0498 | 0.0497 | 0.0701 | 0.1177 | 0.1017 |

Les résultats illustrés aux Figures 7B et C montrent que les protéines yeIF4A, FAL1, hueIF4A, eIF4AIII et Ded1 induisent la sécrétion de taux d'IL-10 et de TNF-$\alpha$ significativement plus élevés que ceux induits dans les surnageants de cultures des monocytes non stimulés (p<0,05, cf. Tableau 2 ci-dessous).

Les protéines FAL1, hueIF4A, eIF4AIII et Ded1 induisent la production de taux d'IL-10 et de TNF-$\alpha$ comparables à ceux induits par la protéine LeIF soluble ou insoluble (p<0.05). Cependant, la protéine yeIF4A de la levure induit la production de taux d'IL-10 et de TNF-$\alpha$ significativement plus faibles que ceux induits par la protéine LeIF soluble ou insoluble (p<0,05, Tableau 2).

Le Tableau 2 ci-dessous montre l'analyse statistique des différences observées dans les taux d'IL-10 et de TNF-$\alpha$ dans les surnageants de culture des monocytes stimulés par les protéines recombinantes yeIF4A, FAL1, hueIF4A, eIF4AIII et Ded1. Les valeurs du p déterminés par le paired t test sont indiquées.

Tableau 2 :

| | | NS | LPS | LeIF | LeIF (S) | yeIF4A | FAL1 | hueIF4A | eIF4AIII |
|---|---|---|---|---|---|---|---|---|---|
| IL-10 | LPS | 0.0014 | | | | | | | |
| | LeIF | 0.0013 | 0.004 | | | | | | |
| | LeIF (S) | 0.0037 | 0.0121 | 0.1634 | | | | | |
| | yeIF4A | 0.0022 | 0.151 | 0.0049 | 0.0089 | | | | |
| | FAL1 | 0.0029 | 0.0227 | 0.1584 | 0.2619 | 0.0007 | | | |
| | hueIF4A | 0.0362 | 0.0682 | 0.09 | 0.1181 | 0.1152 | 0.2091 | | |
| | eIF4AIII | 0.0394 | 0.0168 | 0.6321 | 0.6096 | 0.0776 | 0.0559 | 0.0552 | |
| | Ded1 | 0.0229 | 0.5827 | 0.724 | 0.0888 | 0.8532 | 0.029 | 0.1484 | 0.1141 |
| TNF-$\alpha$ | LPS | 0.0062 | | | | | | | |
| | LeIF | 0.0063 | 0.0006 | | | | | | |
| | LeIF(S) | 0.005 | 0.0006 | | | | | | |
| | yeIF4A | 0.0049 | 0.3789 | 0.0442 | 0.0365 | | | | |
| | FAL1 | 0.0066 | 0.0927 | 0.4029 | 0.3803 | 0.0541 | | | |
| | hueIF4A | 0.0046 | 0.4905 | 0.3365 | 0.3523 | 0.1347 | 0.4965 | | |
| | eIF4AIII | 0.0288 | 0.2621 | 0.1469 | 0.063 | 0.1673 | 0.5381 | 0.335 | |
| | Ded1 | 0.016 | 0.7602 | 0.1256 | 0.132 | 0.0514 | 0.1768 | 0.2411 | 0.135 |

L'ensemble de ces résultats montre que les protéines homologues de la protéine LeIF : yeIF4A, FAL1 et Ded1 chez la levure et hueIF4A et eIF4AIII de mammifères sont aussi capables d'induire la production des cytokines IL-12p70, IL-10 et TNF-$\alpha$ par les monocytes humains *in vitro.*

III.3. L'activité inductrice de la production de cytokines par les différentes protéines recombinantes est protéinase K sensible et polymixine B résistante.

Pour vérifier l'absence de contaminants d'origine bactérienne (LPS) dans les préparations de protéines recombinantes utilisées, les taux d'IL-10 produits dans les cultures de monocytes stimulés par la protéine recombinante ou par le LPS en présence ou non de protéinase K (endopeptidase qui dégrade les protéines) ou de polymixine B (qui a pour effet de bloquer l'action du LPS) ont été comparés. Les résultats illustrés à la Figure 8, montrent que la protéinase K inhibe à plus de 50 % la production d'IL-10 par les protéines recombinantes, alors que la polymixine B n'a pas d'effet sur cette activité. Contrairement aux protéines recombinantes, l'activité inductrice d'IL-10 du LPS est fortement inhibée par la polymixine B, mais résiste au traitement par la protéinase K (Figure 8).

Ces résultats montrent que les différentes préparations antigéniques utilisées ne sont pas contaminées par des endotoxines bactériennes.

**EXEMPLE 2 : IDENTIFICATION DES FRAGMENTS DE LA PROTEINE LEIF INDUISANT LA PRODUCTION *IN VITRO* DES CYTOKINES IL-12p70, IL-10 ET TNF-alpha**

Dans le but d'identifier la région minimale responsable de l'induction des cytokines au sein de la protéine LeIF, plusieurs fragments de cette protéine ont été produits. Se basant sur le fait que les régions NH$_2$ de LeIF de *L. braziliensis* et *L. major* ont été décrites comme étant responsables de l'activité immunomodulatrice de ces protéines, alors qu'une perte de l'activité était observée pour les régions centrale et COOH (Probst *et al.*, 1997 ; Skeiky *et al.*, 1998), il a été

construit les parties NH$_2$ (1-226 ; SEQ ID NO : 18), centrale (129-261 ; SEQ ID NO : 23) et COOH (196-403 ; SEQ ID NO: 24) de la protéine LeIF de *L. infantum.* Il a été également construit deux autres fragments au sein de la région NH$_2$ : 1-195 (SEQ ID NO : 17) et 129-226 (SEQ ID NO : 22).

L'isolement des cellules mononucléées (PBMC), l'induction de la sécrétion des cytokines et le dosage par ELISA sandwich ont été effectués dans les mêmes conditions que celles décrites à l'Exemple 1. Brièvement, les monocytes purifiés à partir des PBMC de 6 individus sains, ont été primés pendant 12h à 37°C par l'IFN-$\gamma$, puis stimulés, soit par les différentes protéines ou fragments à des concentrations finales de 10 $\mu$g/ml soit par le LPS à 1 $\mu$g/ml, pendant 24h pour la production de l'IL-12p70. Pour la production de l'IL-10 et de TNF-$\alpha$, les monocytes ont été stimulés pendant 18h avec les différentes protéines recombinantes ou par le LPS. Les taux d'IL-12, d'IL-10 et de TNF-$\alpha$ sont déterminés par ELISA sandwich.

1. Analyse de la capacité inductrice d'IL-12p70

La Figure 9(A) montre les résultats des expériences effectuées chez cinq individus. Le Tableau 3 ci-dessous montre l'analyse statistique des différences observées dans les taux d'IL-12p70 induits dans les surnageants de culture des monocytes stimulés par la protéine LeIF et ses différents fragments insolubles. Les valeurs du p déterminés par le paired t test sont indiquées.

Tableau 3 :

| | | | IL-12p70 | | | | | |
|---|---|---|---|---|---|---|---|---|
| | NS | IFN-$\gamma$ | IFN-$\gamma$+LPS | IFN-$\gamma$+LeIF | IFN-$\gamma$ -NH2 | IFN-$\gamma$ + COOH | IFN-$\gamma$+129-261 | IFN-$\gamma$+1-195 |
| IFN-$\gamma$ | 0.3132 | | | | | | | |
| ITN-$\gamma$-LPS | 0.0108 | 0.0108 | | | | | | |
| IFN-$\gamma$+LeIF | 0.0169 | 0.0169 | 0.1276 | | | | | |
| IFN-$\gamma$ +NH2 | 0.0273 | 0.0272 | 0.1614 | 0.0144 | | | | |
| IFN-$\gamma$ + COOH | 0.0251 | 0.0250 | 0.0787 | 0.0363 | 0.0606 | | | |
| IFN-$\gamma$+129-261 | 0.0686 | 0.0685 | 0.3537 | 0.0193 | 0.0191 | 0.0085 | | |
| IFN-$\gamma$-1-195 | 0.0284 | 0.0283 | 0.1387 | 0.0052 | 0.589 | 0.2398 | 0.0332 | |
| IFN-$\gamma$+129-226 | 0.0575 | 0.0570 | 0.2529 | 0.0212 | 0.0398 | 0.0143 | 0.1690 | 0.1238 |

Contrairement aux deux fragments 129-261 et 129-226, les taux d'IL-12p70 induits par la stimulation par les fragments NH$_2$ (1-226) (3313,43$\pm$1375,7 pg/ml) ; (1-195) (3532$\pm$1485,8 pg/ml) et COOH (196-403) (4764,2$\pm$1935,5 pg/ml) sont significativement plus élevés que ceux produits par les monocytes non stimulés (24,78$\pm$ 10,76 pg/ml, p<0,05, Tableau 3) ou stimulés par les protéines recombinantes NH$_2$ (1-226), 1-195 ou COOH (196-403) seules (202,91$\pm$94,55, 205,93$\pm$89,43 et 267,19$\pm$101,87 pg/ml, respectivement ; p<0.05) ou l'IFN-$\gamma$ seul (21,56$\pm$11,64 pg/ml) (p <0,05, cf. Tableau 3).

Les taux d'IL-12p70 induits dans les surnageants de culture des monocytes activés par l'IFN-$\gamma$ et stimulés par la protéine LeIF entière (5375,63$\pm$1683 pg/ml) sont significativement supérieurs à ceux induits par tous les fragments (p< 0,05, cf. Tableau 3) et comparables à ceux induits par le LPS (2678,3$\pm$783,51 pg/ml, p> 0,05, cf. Tableau 3).

Contrairement à ce qui a été rapporté dans la littérature (Probst *et al*., 1997), la partie COOH (195-403) de la protéine LeIF de l'espèce *L. infantum* induit la production de l'IL-12p70 (4764,2$\pm$1935,5 pg/ml) par les monocytes activés par l'IFN-$\gamma$. Ces taux d'IL-12p70 sont comparables à ceux induits par la partie NH$_2$ (1-226) (3313,43$\pm$1375,7 pg/ml, p>0,05, Tableau 3) ou le fragment 1-195 (3532$\pm$1485,8 pg/ml, p>0,05, Tableau 3). Etant donné que le fragment 129-261, induit des taux d'IL-12p70 faibles, l'activité inductrice au sein de la partie COOH (196-403) pourrait être localisée au niveau de la région minimale correspondant aux positions 261 à 403.

Au sein de la région NH$_2$, il a été montré que contrairement aux résultats obtenus avec le fragment 129-226, le fragment 1-195 induit des taux significatifs d'IL-12p70, ce qui suggère qu'au sein de cette région NH$_2$ l'activité inductrice est localisée au niveau du fragment 1-129.

2. Analyse de la capacité inductrice d'IL-10

Les résultats illustrés dans la Figure 9(B) montrent que tous les fragments induisent la sécrétion de taux d'IL-10 significativement plus élevés que ceux induits dans les surnageants de culture des monocytes non stimulés ($27,53 \pm 24,75$ ; p< 0,05, cf. Tableau 4). La différence entre les taux d'IL-10 induits par la partie NH$_2$ ou le fragment 1-195 et la partie COOH (196-403), n'est pas significative (p >0,05, cf. Tableau 4 ci-dessous).

Les fragments NH2 (1-226) et 1-195 induisent la sécrétion de taux d'IL-10 significativement plus élevés que ceux induits par la stimulation par du LPS ($1361,43 \pm 261,9$ pg/ml pour NH2 et $1331,17 \pm 228,35$ pg/ml pour 1-195 ; p< 0.05, cf. Tableau 4) et par les fragments 129-261 ($960,26 \pm 229,99$ pg/ml, p< 0.05, tableau 10) et 129-226 ($631,09 \pm 134,59$ pg/ml, p< 0.05, cf. Tableau 4).

Cependant, la protéine LeIF entière reste plus efficace que toutes les fragments analysés ainsi que le LPS dans l'induction de la production d'IL-10 ($2329,94 \pm 322,02$ pg/ml pour LeIF et $664,8 \pm 106,71$ pg/ml pour LPS ; p= 0.0032, cf. Tableau 4).

Le Tableau 4 ci-dessous montre l'analyse statistique des différences observées pour les taux de production d'IL-10 dans les surnageants de culture des monocytes stimulés par les différentes protéines recombinantes. Les valeurs du p déterminés par le paired t test sont indiquées.

Tableau 4 :

|  | NS | LPS | LeIF | NH2 (1-226) | COOH (195-403) | 129-261 | 1-195 |
|---|---|---|---|---|---|---|---|
| LPS | 0.0014 | | | | | | |
| LeIF | 0.0009 | 0.0032 | | | | | |
| NH2 (1-226) | 0.0042 | 0.0447 | 0.0098 | | | | |
| COOH (195-403) | 0.072 | 0.4294 | 0.0139 | 0.0895 | | | |
| 129-261 | 0.0174 | 0.28 | 0.0029 | 0.0263 | 0.2569 | | |
| 1-195 | 0.0026 | 0.0356 | 0.0008 | 0.826 | 0.5313 | 0.0322 | |
| 129-226 | 0.0064 | 0.8171 | 0.0017 | 0.0041 | 0.4238 | 0.0254 | 0.0057 |

3. Analyse de la capacité inductrice de TNF-$\alpha$

Le dosage de TNF-$\alpha$ a été effectué dans les surnageants de culture de monocytes purifiés à partir des PBMC de 6 donneurs sains. La Figure 9(C) montre que la protéine LeIF entière ainsi que tous ses fragments induisent la production de taux de TNF-$\alpha$ significativement plus élevés que ceux observés dans les surnageants de culture des monocytes non stimulés (LeIF ($9781,51 \pm 1535,1$ pg/ml); NH$_2$ (1-226) ($8430,47 \pm 1653$ pg/ml) ; COOH(196-403) ($9339,07 \pm 1334,3$ pg/ml) ; 129-261 ($5341,45 \pm 1281,1$ pg/ml) ; 1-195 ($8556,9 \pm 1856,3$ pg/ml) ; 129-226 ($5543,69 \pm 1185,7$ pg/ml) et NS ($136,64 \pm 54,5$ pg/ml), p<0,05, cf Tableau 5 ci-dessous).

Les fragments COOH (195-403), NH$_2$ (1-226) et 1-195 induisent la sécrétion de taux de TNF-$\alpha$ comparables à ceux induits par la protéine LeIF (p>0,05, Tableau 5). Une fois encore, les fragments 129-261 et 129-226 induisent les taux de TNF-$\alpha$ les plus faibles ($5341,45 \pm 1281,1$ et $5543,69 \pm 1185,7$ pg/ml, respectivement).

Le Tableau 5 montre l'analyse statistique des différences observées dans les taux de TNF-$\alpha$ dans les surnageants de culture des monocytes stimulés par la protéine LeIF soluble et ses différents domaines. Les valeurs du p déterminés par le paired t test sont indiquées.

Tableau 5 :

|  | NS | LPS | LeIF | NH2 (1-226) | COOH (195-403) | 129-261 | 1-195 |
|---|---|---|---|---|---|---|---|
| LPS | 0.0062 | | | | | | |
| LeIF | 0.0015 | 0.0008 | | | | | |
| NH2 (1-226) | 0.0038 | 0.1423 | 0.1597 | | | | |
| COOH (196-403) | 0.0009 | 0.0521 | 0.4819 | 0.3622 | | | |
| 129-261 | 0.0138 | 0.3044 | 0.0213 | 0.0188 | 0.0126 | | |
| 1-195 | 0.006 | 0.1414 | 0.2958 | 0.8145 | 0.5755 | 0.0254 | |
| 129-226 | 0.0056 | 0.0838 | 0.0058 | 0.0057 | 0.013 | 0.3635 | 0.0122 |

**EXEMPLE 3 : INDUCTION PAR LA PROTEINE MUTANTE LeIFK76A DE LA SECRETION DES CYTOKINES IL-12p70, IL-10 ET TNF-alpha PAR LES MONOCYTES HUMAINS *IN VITRO***

L'effet de la substitution dans le motif I, de la lysine en position 76 en alanine, sur la capacité inductrice des cytokines de la protéine LeIF a été évalué. Cette mutation abolit l'activité ATPase de la protéine LeIF *(Barhoumi et al.*, 2006).

L'isolement des cellules mononucléées (PBMC), l'induction de la sécrétion des cytokines et le dosage par ELISA sandwich ont été effectués dans les mêmes conditions que celles décrites à l'Exemple 1. Brièvement, les monocytes purifiés à partir des PBMC de 3 individus primés ou non par l'IFN-$\gamma$ pendant 12 h, sont stimulés par les protéines LeIF (SEQ ID NO : 11) ou LeIFK76A (SEQ ID NO : 27) à des concentrations finales de 10 $\mu$g/ml, ou par le LPS à 1 $\mu$g/ml. Les surnageants de culture sont collectés après 24 h d'incubation pour le dosage de l'IL-12p70 ou 18 h pour le dosage de l'IL-10 et de TNF-$\alpha$. Les taux des cytokines sont déterminés par ELISA sandwich.

- IL-12p70 :

Les résultats illustrés à la Figure 10 (A) montrent que la protéine LeIFK76A induit la production de taux élevés d'IL-12p70 (2272,78$\pm$1257,5 pg/ml) par des monocytes activés par l'IFN-$\gamma$. Ces taux sont significativement plus élevés que ceux induits dans les surnageants de culture des monocytes non stimulés (29,69$\pm$24, 31 pg/ml) ou stimulés par la protéine recombinante LeIFK76A seule (342,55$\pm$323,24 pg/ml) ou l'IFN-$\gamma$ seul (22,43$\pm$17,14 pg/ml ; p<0,05, cf. Tableau 6 ci-dessous).

Ils sont comparables à ceux induits par la protéine recombinante LeIF soluble (2564,46$\pm$1237,1 pg/ml).

Le Tableau 6 montre l'analyse statistique des différences observées pour les taux de production d'IL-12p70 dans les surnageants de culture des monocytes stimulés par les protéines LeIF et LeIFK76A. Les valeurs du p déterminés par le paired t test sont indiquées.

Tableau 6 :

|  | NS | IFN-$\gamma$ | IFN-$\gamma$ + LPS | IFN-$\gamma$+LeIF |
|---|---|---|---|---|
| IFN-$\gamma$ | 0.3132 |  |  |  |
| IFN-$\gamma$-LPS | 0.0108 | 0.0108 |  |  |
| IFN-$\gamma$-LeIF(S) | 0.0193 | 0.0190 | 0.0468 | 0.1468 |
| IFN-$\gamma$+LeIFK76A | 0.0440 | 0.0434 | 0.1828 | 0.1387 |

- IL-10 et TNF-$\alpha$ :

Les résultats illustrés à la Figure 10(B) et (C) montrent que la protéine LeIFK76A induit la production de taux d'IL-10 (1766,24$\pm$582,5 pg/ml) et de TNF-$\alpha$ (10303,36$\pm$1032,5 pg/ml) significativement plus élevés que ceux induits dans les surnageants de culture des monocytes non stimulés (27,53$\pm$24,75 pg/ml ; p<0,05, cf. Tableau 7 ci-dessous), mais comparables à ceux induits par la protéine LeIF soluble (2138,54$\pm$555,59 pg/ml pour l'IL-10 et 11946,49$\pm$ 623,04 pg/ml pour le TNF-$\alpha$, p>0,05, Tableau 7).

Le Tableau 7 montre l'analyse statistique des différences observées dans les taux d'IL-10 et de TNF-$\alpha$ dans les surnageants de culture des monocytes stimulés par les protéines LeIF et LeIFK76A. Les valeurs du p déterminés par le paired t test sont indiquées.

Tableau 7 :

|  |  | NS | LPS | LeIF (S) |
|---|---|---|---|---|
|  | LPS | 0.0014 |  |  |
| IL-10 | LeIF(S) | 0.0037 | 0.0121 |  |
|  | LeIFK76A | 0.0094 | 0.1665 | 0.0672 |
|  | LPS | 0.0062 |  |  |
| TNF-$\alpha$ | LeIF(S) | 0.005 | 0.0006 |  |
|  | LeIFK76A | 0.0085 | 0.2627 | 0.2307 |

L'ensemble de ces résultats montre que la substitution, dans le motif I, de la lysine en position 76 en alanine qui abolit l'activité ATPase de la protéine LeIF, n'affecte pas son activité inductrice des cytokines IL-12p70, IL-10 et TNF-$\alpha$.

**EXEMPLE 4 : INDUCTION PAR LES PROTEINES LEIF∆25, D1+25, D1 ET D2 DE LA SECRETION DES CYTOKINES IL-12p70, IL-10 ET TNF-alpha PAR LES MONOCYTES HUMAINS *IN VITRO***

Afin de caractériser l'activité inductrice de cytokines des protéines recombinantes LeIF∆25 (protéine LeIF de *L. infantum* délétée des 25 premiers résidus amino-terminaux les plus divergents entre LeIF et ses homologues de la levure et les mammifères ; SEQ ID NO : 21), D1 (25-237 ; SEQ ID NO : 20), D1+25 (1-237 ; SEQ ID NO : 19) qui correspond à la partie $NH_2$ et D2 (237-403 ; SEQ ID NO : 25) qui correspond à la partie COOH de la protéine LeIF de *L. infantum*, des monocytes purifiés à partir des PBMC de cinq individus sains par ces différentes protéines recombinantes à des concentrations finales de 10 μg/ml ont été stimulés.

Les conditions de stimulation sont identiques à celles décrites à l'Exemple 1. Brièvement, les monocytes purifiés à partir des PBMC de cinq individus sains, primés pendant 12h par l'IFN-γ (3000 U/ml) ou non, sont stimulés par la protéine LeIF soluble et ses différents domaines à des concentrations finales de 10 μg/ml ou par le LPS à 1 μg/ml. Les surnageants de culture sont collectés après 24h d'incubation pour le dosage de l'IL-12 ou 18 h pour le dosage de l'IL-10 et de TNF-α. Les taux d'IL-12, d'IL-10 et du TNFα sont déterminés par ELISA sandwich.

- IL-12 :

Les résultats illustrés à la Figure 11(A) montrent que les différentes protéines recombinantes LeIF∆25, D1+25, D1 et D2 induisent des taux d'IL-12-p70 (2921,95±905,78 ; 2766,71±942,32 ; 2226,48±1188,2 et 1482,99±596,89 pg/ml, respectivement) significativement plus élevés que ceux induits dans les surnageants de culture des monocytes non stimulés (34,17±17,77 pg/ml ; p<0,05, cf. Tableau 8 ci-dessous) ou stimulés par l'IFN-γ seul (35,53±17,84 pg/ml, p<0,05, cf. Tableau 8) ou par la protéine seule (LeIF∆25(312,22±165,25), D1+25(339,74±181,26), D1(214,26±191,55) et D2(243,28±132,6)).

Il est à noter que la délétion des résidus 25 amino-terminaux n'affecte pas l'activité inductrice de la cytokine IL-12p70 de la protéine LeIF. En effet, la différence entre les taux d'IL-12p70 induits par la protéine LeIF soluble (3805,9±1069,1 pg/ml) et la protéine LeIF∆25 (2921,95±905,78 pg/ml) n'est pas significative (p> 0,05, cf. Tableau 8). Les domaines D1+25 (1-237) et D1 induisent la production de taux d'IL-12p70 comparables à ceux induits par la protéine LeIF∆25 et significativement plus élevés que ceux induits par le domaine D2 (1482,99±596,89 pg/ml ; p<0,05, Tableau 8).

La différence entre les taux d'IL-12p70 induits par le domaine D1 et ceux induits par le domaine D2 de la protéine LeIF peut être due à la différence dans le nombre des motifs à boîte DEAD entre les deux domaines. En effet, le domaine D1 renferme 6 motifs (Q, Ia, Ib, I, II et III) et le domaine D2 contient les 3 motifs : IV, V et VI. Ceci laisse penser que les motifs à boîte DEAD pourraient jouer un rôle dans l'activité inductrice de la cytokine IL-12p70 par la protéine LeIF.

Le Tableau 8 montre l'analyse statistique des différences observées dans les taux d'IL-12p70 dans les surnageants de culture des monocytes stimulés par la protéine LeIF soluble et ses différents domaines. Les valeurs du p déterminés par le paired t test sont indiquées.

Tableau 8 :

| | NS | IFN-γ | IFN-γ +LPS | IFN-γ +LeIF (S) | IFN-γ+LeIF∆25 | IFN-γ+D1+25 | IFN-γ+D1 |
|---|---|---|---|---|---|---|---|
| IFN-γ | 0.3132 | | | | | | |
| IFN-γ +LPS | 0.0108 | 0.0108 | | | | | |
| IFN-γ+LeIF (s) | 0.0193 | 0.0190 | 0.0468 | | | | |
| IFN-γ+LeIF∆25 | 0.0252 | 0.0248 | 0.1342 | 0.0827 | | | |
| IFN-γ+D1+25 | 0.0314 | 0.0310 | 0.1641 | 0.0471 | 0.1777 | | |
| IFN-γ+D1 | 0.0345 | 0.0341 | 0.1831 | 0.0425 | 0.1364 | 0.1077 | |
| IFN-γ+ D2 | 0.0475 | 0.0465 | 0.3485 | 0.013 | 0.0098 | 0.0196 | 0.0290 |

- IL-10 :

Les résultats illustrés à la Figure 11(B) montrent que les différentes protéines recombinantes LeIF∆25, D1+25 (1-237), D1(25-237) et D2(237-403) induisent la sécrétion de taux d'IL-10 (1951,15±408,4 ; 1477,88±381,92 ; 1408,91±269,75 et 1130,55±184,9 pg/ml) significativement plus élevés que ceux induits dans les surnageants de culture des monocytes non stimulés (32,14 pg/ml ; p<0,05, cf. Tableau 9 ci-dessous).

La protéine LeIFΔ25 induit la production de taux d'IL-10 (1951,15±408,4 pg/ml) comparables à ceux induits par la protéine LeIF (2067,68± 324,69 pg/ml, p = 0.4515, cf. Tableau 9), par le domaine D1+25(1-237) (1477,88±381,92 pg/ml ; p = 0.0786, Tableau 9) ou par le domaine D1 (25-237) (1408,91±269,75 pg/ml ; p = 0.0786, cf. Tableau 9). La délétion des 25 résidus aminoterminaux n'affecte pas l'activité inductrice de l'IL-10, de la protéine LeIF.

Il est enfin à noter que le domaine D2 (237-403) de la protéine LeIF induit la production d'un taux d'IL-10 (1130,55±184,9 pg/ml) significativement plus faible que celui induit par la protéine LeIF (S) ou LeIFΔ25 (p<0,05, cf. Tableau 9) et comparable à celui induit par le domaine D1+25 (1-237) ou D1 (25-237) (p>0,05, cf. Tableau 9).

Le Tableau 9 montre l'analyse statistique des différences observées dans les taux d'IL-10 dans les surnageants de culture des monocytes stimulés par la protéine LeIF soluble et ses différents domaines. Les valeurs du p déterminés par le paired t test sont indiquées.

Tableau 9 :

|  | NS | LPS | LeIF (S) | LeIFΔ25 | D1+25(1-237) | D1 (25-237) |
|---|---|---|---|---|---|---|
| LPS | 0.0014 |  |  |  |  |  |
| LeIF (S) | 0.0037 | 0.0121 |  |  |  |  |
| LeIFΔ25 | 0.0104 | 0.0371 | 0.4515 |  |  |  |
| D1+25(1-237) | 0.0197 | 0.114 | 0.0736 | 0.0781 |  |  |
| D1 (25-231) | 0.0075 | 0.2426 | 0.0436 | 0.0786 | 0.9861 |  |
| D2 (237-403) | 0.0051 | 0.0785 | 0.0026 | 0.0272 | 0.2757 | 0.4896 |

- TNF-α :

Les résultats illustrés à la Figure 11(C) montrent que toutes les protéines recombinantes LeIFΔ25, D1+25 (1-237), D1(25-237) et D2(237-403) induisent la production de taux de TNF-α significativement plus élevés ceux induits dans les surnageant de culture des monocytes non stimulés (8353,4±1827 ; 7612,07±1250,7 ; 8743,63±1419 ; 6104,9±1419 et 136,64±54,5 pg/ml pour les protéines LeIFΔ25, D1+25(1-237), D1(25-237), D2(237-403) et NS, respectivement ; p<0,05 ; cf. Tableau 10 ci-dessous).

A l'exception du domaine D2, les trois autres protéines LeIFΔ25, D1+25, D1 induisent des taux de TNF-α comparables à ceux induits par la protéine LeIF soluble (9305,21±1656,3 pg/ml ; p>0,05, cf. Tableau 10).

Le Tableau 10 montre l'analyse statistique des différences observées dans les taux de TNF-α dans les surnageants de culture des monocytes stimulés par la protéine LeIF soluble et ses différents domaines. Les valeurs du p déterminés par le paired t test sont indiquées.

Tableau 10 :

|  | NS | LPS | LeIF(S) | LeIFΔ25 | D1-25 | D1 |
|---|---|---|---|---|---|---|
| LPS | 0.0062 |  |  |  |  |  |
| LeIF (S) | 0.005 | 0.0006 |  |  |  |  |
| LeIFΔ25 | 0.0104 | 0.0493 | 0.158 |  |  |  |
| D1-25(1-237) | 0.0036 | 0.1012 | 0.0584 | 0.4538 |  |  |
| D1(25-237) | 0.0231 | 0.8136 | 0.1531 | 0.0961 | 0.5901 |  |
| D2(237-403) | 0.0123 | 0.7107 | 0.0357 | 0.0927 | 0.0503 | 0.1943 |

L'ensemble de ces résultats montre que tous les domaines de la protéine LeIF sont dotés d'activité inductrice de l'IL-12p70, d'IL-10 et de TNF-α. Une baisse significative de cette activité est cependant observée pour le domaine D2. En outre, la délétion des 25 premiers résidus amino-terminaux, lesquels sont uniques à la protéine LeIF de *Leishmania*, ne semble pas affecter l'activité de la protéine entière.

## RÉFÉRENCES

Altmann et al., EMBO, 1995, 14, 3820-3827.
Badaro et al., Braz J Infect Dis, 2001, 5, 223-232.
Banroques et al., Mol Cell Biol, 2008, 28, 3359-71.

Barhoumi et al., FEBS J, 2006, 273, 5086-5100.
Bates et al., EMBO J, 2005, 24, 543-553.
Bente et al., Proteomics, 2003, 3, 9, 1811-29.
Benz et al., Structure Fold Des, 1999, 7, 671-679.
Bizebard et al., Biochemistry, 2004, 43, 7857-7866.
Carmel and Matthews, RNA, 2004, 10, 66-74.
Caruthers et al., Proc Natl Acad Sci USA, 2000, 97, 13080-13085.
Caruthers and McKay, Curr Opin Struct Biol, 2002, 12, 123-133.
Chan et al., RNA, 2004, 10, 200-209.
Chang et al., Nucleic Acids Res, 1997, 25, 5033-5040.
Chuang et al., Science, 1997, 275, 1468-1471.
Coler et al., Infect Immun, 2002, 70, 4215-4225.
Coler et al., Infect Immun, 2007, 75, 4648-4654.
Cordin et al., EMBO J, 2004, 23, 2478-2487.
Cordin et al., Gene, 2006, 367, 17-37.
de la Cruz et al., Proc Natl Acad Sci USA, 1997, 94, 10, 5201-6.
de la Cruz et al., Trends Biochem Sci, 1999, 24, 192-198.
Emery et al., Mol Microbiol, 2004, 52, 141-158.
Ferraiuolo et al., Proc Natl Acad Sci USA, 2004, 101, 4118-4123.
Flores-Rozas and Hurwitz, J Biol Chem, 1993, 268, 21372-21383.
Fuller-Pace et al., EMBO J, 1993, 12, 3619-3626.
Gillian and Svaren, J Biol Chem, 2004, 279, 9056-9063.
Gorbalenya et al., Nucleic Acids Res., 1989, 17, 12, 4713-30.
Gorbalenya and Koonin, Curr Opin Struct Biol, 1993, 3, 419-429.
Hall and Matson, Mol Microbiol, 1999, 34, 867-877.
He et al., World J Gastroenterol, 2008, 14, 4, 532-40
Hirling et al., Nature, 1989, 339, 562-564.
Hodge et al., EMBO J, 1999, 18, 5778-5788.
Huang and Liu, J Biol Chem, 2002, 277, 12810-12815.
Ilyina et al., J Mol Evol., 1992, 34, 4, 351-7.
Iost et al., JBiol Chem, 1999, 274, 17677-17683.
Johnson and McKay, RNA, 1999, 5, 1526-1534.
Kadare and Haenni, J Virol, 1997, 71, 2583-2590.
Kim et al., Structure, 1998, 6, 89-100.
Korolev et al., Cell, 1997, 90, 635-647.
Kossen and Uhlenbeck, Nucleic Acids Res, 1999, 27, 3811-3820.
Kossen et al., J Mol Biol, 2002, 324, 625-636.
Kressler et al., Mol Cell Biol, 1997, 17, 7283-7294.
Kressler et al., Mol Cell Biol, 1999, 19, 12, 7897-912.
Lamm et al., Nucleic Acids Res, 1996, 24, 3739-3747.
Lenzi et al., J. Transl Med., 2007, 5, 66.
Li et al., Mol Cell Biol, 1999, 19, 7336-7346.
Linder, Biol Cell, 2003, 95, 3-4, 157-167.
Linder et al., Nature, 1989, 337, 121-122.
Linder and Stutz, Curr Biol, 2002, 11, 961-963.
Lorsh and Herschlag, Biochemistry, 1998a, 37, 2180-2193.
Luking et al., Crit Rev Biochem Mol Biol, 1998, 33, 259-296.
Myler et al., Proc Natl Acad Sci USA, 1999, 96, 2902-2906.
Noueiry et al., Proc Natl Acad Sci USA, 2000, 97, 12985-12990.
Nugent et al., Mol Biochem Parasitol, 2004, 136, 51-62.
Palacios et al., Nature, 2004, 427, 753-757.
Patel and Picha, Annu Rev Biochemi, 2000, 69, 651-697.
Pause and Sonenberg, EMBO J, 1992, 11, 2643-2653.
Pause et al., Mol Cell Biol, 1993, 13, 6789-6798.
Probst et al., Eur J Immunol, 1997, 27, 2634-2642.
Reed and Hurt, Cell, 2002, 108, 523-531.
Rocak and Linder, Nat Rev Mol Cell Biol, 2004, 5, 232-241.
Rocak et al., Nucleic Acids Res, 2005, 33, 999-10009.

Rogers et al., J Biol Chem, 1999, 274, 12236-12244.
Rogers et al., J Biol Chem, 2001 a, 276, 12598-12608.
Rogers et al., J Biol Chem, 2001b, 276, 30914-30922.
Rogers et al., Prog Nucleic Acid Res Mol Biol, 2002, 72, 307-331.
Rossler et al., Nucleic Acids Res, 2001, 29, 2088-2096.
Rozen et al., Mol Cell Biol, 1990, 10, 1134-1144.
Sabel et al., Surgery, 2007, 142, 749-760.
Salay et al., Clin Vaccine Immunol, 2007, 14, 1173-1181.
Schmitt et al., EMBO J, 1999, 18, 4332-4347.
Schmid and Linder, Mol Microbiol, 1992, 6, 283-291.
Schwer and Mezaros, EMBO J, 2000, 19, 6582-6591.
Shi et al., Proc Natl Acad Sci USA, 2004, 101, 17628-17633.
Shibuya et al., Nat Struct Mol Biol, 2004, 11, 346-351.
Shiratori et al., Cancer Sci., 2007, 98, 1936-1942.
Shneider et al., J Biol Chem, 2004, 279, 8617-8626.
Silverman et al., Gene, 2003, 312, 1-16.
Singleton and Wigley, J Bacteriol, 2002, 184, 1819-1826.
Skeiky et al., J Exp Med, 1995, 181, 1527-1537.
Skeiky et al., J Immunol, 1998, 161, 6171-6179.
Son & Kim, Arch Pharm Res., 2007, 30, 8, 1047-50.
Sonenberg and Diver, Curr Opin Stuct Biol, 2003, 13, 56-63.
Staley and Gutherie, Mol Cell, 1999, 3, 55-64.
Story and Steitz, Nature, 1992, 55, 374-376.
Story et al., Proc Natl Acad Sci USA, 2001, 98, 1465-1470.
Strasser and Hurt, Nature, 2001, 413, 648-652.
Subramanya et al., Nature, 1996, 28, 379-383.
Svitkin et al., RNA, 2001, 7, 382-394.
Tanner, Cell Cycle, 2003, 2, 9-18.
Tanner et al., Mol Cell, 2003, 11, 127-138.
Tanner and Linder, Mol Cell, 2001, 8, 251-262.
Theis et al., EMBO J, 1999, 18, 6899-6907.
Tseng et al., EMBO J, 1998, 17, 2651-2662.
Tsu and Uhlenbeck, Biochemistry, 1998, 37, 16989-16996.
Tsu et al., RNA, 2001, 7, 702-709.
Tuteja and Tuteja, Eur J Biochem, 2004a, 271, 1835-1840.
Tuteja and Tuteja, Eur J Biochem, 2004b, 271, 1849-1863.
Valdez et al., Eur J Biochem, 1997, 250, 800-807.
Valdez, Eur J Biochem, 2000, 267, 21, 6395-402.
Velankar et al., Cell, 1999, 97, 75-84.
Walker et al., EMBO J, 1982, 1, 945-951.
Weiss et al., Expert Opin Biol Ther, 2007, 7, 1705-1721.
Wilson et al., BMC Mol Biol, 2004, 5, 11.
Xu et al., EMBO J, 2004, 23, 376-385.
Yan et al., Mol Cell Biol, 2003, 23, 414-423.
Yao et al., Nat Struct Biol, 1997, 4, 463-467.
Ye et al., Biochim Biophys Acta, 2004, 1659, 1-18.
Zhao et al., EMBO J, 2002, 21, 1177-1187.
Zhao et al., Structure, 2004, 12, 1373-1381.

**Revendications**

1. Utilisation d'une ARN hélicase à boîte DEAD de levure ou de mammifère de type sauvage ou présentant une mutation dans le motif consensus I (SEQ ID NO: 2), ou d'un fragment de celles-ci comprenant soit les six motifs consensus Q (SEQ ID NO : 1), I (SEQ ID NO : 2), Ia (SEQ ID NO : 3), Ib (SEQ ID NO : 4), II (SEQ ID NO : 5) et III (SEQ ID NO : 6) mais pas les trois motifs consensus IV (SEQ ID NO : 7), V (SEQ ID NO : 8) et VI (SEQ ID NO : 9), soit lesdits trois motifs consensus IV, V et VI mais pas lesdits six motifs Q, I, Ia, Ib, II et III, pour induire la production *in vitro* ou *ex vivo* d'au moins une cytokine par une cellule mononucléaire du sang périphérique (PBMC) d'un mammifère.

2. Utilisation selon la revendication 1, **caractérisée en ce que** ladite levure appartient au genre *Saccharomyces*.

3. Utilisation selon la revendication 1 ou la revendication 2, **caractérisée en ce que** ladite cytokine est sélectionnée dans le groupe constitué par IL-12, IL-10 et TNF-alpha.

4. Utilisation selon l'une quelconque des revendications 1 à 3, **caractérisée en ce que** ladite ARN hélicase à boîte DEAD de type sauvage est sélectionnée dans le groupe constitué par yeIF4A (SEQ ID NO : 12), FAL1 (SEQ ID NO : 13), Ded1 (SEQ ID NO : 14), hueIF4A (SEQ ID NO : 15) et eIF4AIII (SEQ ID NO : 16).

5. Utilisation d'une ARN hélicase à boîte DEAD de levure ou de mammifère ou d'un fragment de celles-ci tels que définis aux revendications 1, 2 ou 4 pour la préparation d'un médicament destiné à traiter ou prévenir les maladies infectieuses ou les cancers.

6. Utilisation selon la revendication 5, **caractérisée en ce que** ledit médicament est utile comme adjuvant de vaccination, pour induire une réponse immunitaire de type Th1.

7. Utilisation selon la revendication 5 ou la revendication 6, **caractérisée en ce que** lesdites maladies infectieuses sont des infections parasitaires, de préférence des infections induites par un parasite intracellulaire, notamment des leishmanioses.

**8.** Utilisation selon l'une quelconque des revendications 5 à 7, **caractérisée en ce que** ladite levure appartient au genre *Saccharomyces.*

**9.** Utilisation selon l'une quelconque des revendications 6 à 8, **caractérisée en ce que** ladite réponse immunitaire consiste en la production d'au moins l'une des cytokines IL-12 et TNF-alpha.

**10.** Composition pharmaceutique comprenant une ARN hélicase à boîte DEAD de levure ou de mammifère ou un fragment de celles-ci, tels que définis dans l'une quelconque des revendications précédentes et au moins un véhicule pharmaceutiquement acceptable.

**11.** Produits contenant (i) une ARN hélicase à boîte DEAD de levure ou de mammifère ou un fragment de celles-ci, tels que définis dans l'une quelconque des revendications précédentes et (ii) l'IFN-γ, pour une utilisation simultanée, séparée ou séquentielle dans le traitement ou la prévention du cancer et des maladies infectieuses, de préférence les infections parasitaires.

**12.** Fragment de la protéine LeIF de *Leishmania infantum*, **caractérisé en ce qu'**il est choisi parmi les fragments de celle-ci comprenant soit les six motifs consensus Q (SEQ ID NO : 1), I (SEQ ID NO : 2), Ia (SEQ ID NO : 3), Ib (SEQ ID NO : 4), II (SEQ ID NO : 5) et III (SEQ ID NO : 6) mais pas les trois motifs consensus IV (SEQ ID NO : 7), V (SEQ ID NO : 8) et VI (SEQ ID NO : 9), soit lesdits trois motifs consensus IV, V et VI mais pas lesdits six motifs Q, I, Ia, Ib, II et III, et les fragments correspondant aux acides aminés 1-195 (SEQ ID NO : 17), 1-226 (SEQ ID NO : 18), 1-237 (SEQ ID NO : 19), 25-237 (SEQ ID NO : 20), 26-403 (SEQ ID NO : 21), 129-226 (SEQ ID NO : 22), 129-261 (SEQ ID NO : 23), 196-403 (SEQ ID NO: 24), 237-403 (SEQ ID NO : 25) ou 261-403 (SEQ ID NO: 26) de ladite protéine LeIF.

**13.** Fragment selon la revendication 12 ou protéine LeIF de *Leishmania infantum* de type sauvage ou mutée dans le motif consensus I pour une utilisation comme médicament.

**14.** Fragment ou protéine selon la revendication 13, **caractérisés en ce que** le médicament est destiné à traiter ou prévenir les maladies infectieuses ou les cancers.

**15.** Fragment ou protéine selon la revendication 14, **caractérisés en ce que** le médicament est utile comme adjuvant de vaccination pour induire une réponse immunitaire de type Th1.

**16.** Produits contenant (i) un fragment selon la revendication 12 ou une protéine telle que définie à la revendication 13 et (ii) l'IFN-γ, pour une utilisation simultanée, séparée ou séquentielle dans le traitement ou la prévention du cancer et des maladies infectieuses, de préférence les infections parasitaires.

**17.** Utilisation d'un fragment selon la revendication 12 ou d'une protéine telle que définie à la revendication 13 pour induire la production *in vitro* ou *ex vivo* d'IL-12 par une cellule mononucléaire du sang périphérique (PBMC) d'un mammifère.

FIGURE 1

**FIGURE 2**

**FIGURE 3**

**FIGURE 4**

**FIGURE 5**

Région R1 : lympocytes
(cellule de petite taille)

Région R2 : monocytes
(cellules de grande taille et
de granulosité forte).

Région R3 : population
cellulaire totale.

Marquage CD3 (lymphocytes T)

Marquage CD19 (lymphocytes B)

Marquage CD14 (monocyte)

**FIGURE 6**

FIGURE 7

FIGURE 8

**FIGURE 9**

**FIGURE 10**

FIGURE 11

```
                     F                              R
LeIF      ----------MAQMDKIAPQDQDSFLDDQPGVRPIPSFDDMPLHQNLLRGIYSYGFEKPSSIQQRAIA
DDX48_hu  MATTATMATSGSARRRLLRKEEDMTKVEFETSEEVDVTPTFDTMGLREDLLRGIYAYGFEKPSAIQQRAIK
IF42_hu   -----MSGGSADYNREHGGPEGMDPDGVIESNWMNEIVDNFDDMNLKESLLRGIYAYGFEKPSAIQQRAII
IF41_hu   ------MSASQDSRSRDMGPDGMEPEGVIESNWMNEIVDSFDDMNLSESLLRGIYAYGFEKPSAIQQRAIL
eIF4A_sc  ---------------SEGITDIEESQIQTNYDRVVYKFDDMELDEMLLRGVFGYGFEEPSAIQQRAIM
                 .**  *  *  :.****::. ****:**:*****

                       I     Ia
LeIF      PFTRGGDIIAQAQSGTGKTGAFSIGLIQRLDFRBNLIQGLVLSPTRELAIQTAEVISRIGEFLSNSSKFC
DDX48_hu  QIIKGRDVIAQSQSGTGKTATFSISVIQCLDIQVRETQALILAPTRELAVQIQRGLLAIGDYMN---VQC
IF42_hu   PCIKGYDVIAQAQSGTGKTATFAISIIQQLEIEFKETQALVLAPTRELAQQIQKVILAIGDYMG---ATC
IF41_hu   PCIKGYDVIAQAQSGTGKTATFAISIIQQIELDLRATQALVLAPTRELAQQIQRVVMAIGDYMG---ASC
eIF4A_sc  PIIEGHDVIAQAQSGTGKTGTFSIAAIQRIDTSVKAPQAIMLAPTRELAIQIQRVVMALAFHMD---IKV
           .*  *:.**:******_.:*:*. ** ::    .*.*:*:****** *  :  :.  .:.

                            Ib                          II
LeIF      ETFVGGTRVQDDLRRIQAG-VIVAVGTPGRVSDVIRRGALRTESLRVLVLDEADEMLSQGFADQIYEIFR
DDX48_hu  HACIGGTNVGEDIRKLDYG-QHVVAGTPGRVFDMIRRRSLRTRAIKMLVLDEADEMLNKGFKEQIYDVYR
IF42_hu   HACIGGTNVRNEMQKLQAEAPHIVVGTPGRVFDMLNRRYLSPRWIKMFVLDEADEMLSRGFKDQIYEIFQ
IF41_hu   HACIGGTNVRAEVQKLQMEAPHIIVGTPGRVFDMLNRRYLSPRYIKMFVLDEADEMLSRGFKDQIYDIFQ
eIF4A_sc  HACIGGTSFVEDAEGLR--DAQIVVGTPGRVFDNIQRRRFRTDKIKMFILDEADEMLSSGFKEQIYQIFT
           .: :***  _  :  . *    : .****** * :.*  :    :::::***** **  :***:::

               III
LeIF      FLPKDIQVALFSATMPEEVLELTKKFMRDPVRILVRRESLTLEGIKQFFIAVE-EEHKLDTIMDLYETVS
DDX48_hu  YLPPATQVVLISATLPHEILEMTKKFMTDPIRILVKRDELTLEGIKQFFVAVEREEWKFDTLCDLYDTLT
IF42_hu   KLNTSIQVVLLSATMPTDVLEVTKKFMRDPIRILVKREELTLEGIKQFYINVEREEWKLDTLCDLYETLT
IF41_hu   KLNSNIQVVLLSATMPSDVLEVTKKFMRDPIRILVKREELTLEGIKQFYINVEREEWKLDTLCDLYETLT
eIF4A_sc  LLPPTTQVVLLSATMPNDVLEVTTKFMRNPVRILVKRDELTLEGIKQFYVNVEEEYKYECLTDLYDSIS
            *    **._*:.***:* ::**:*_*** :*:****::_****:*:** :: ** ** *  :  * ***:::

               IV                                              V
LeIF      IAQSVIFANTRRKVDWIAEKLNQSNHTVSSMHAEMPKSDRERVMNTFRSGSSRVLVTTDLVARGIDVHHV
DDX48_hu  ITQAVIFCNTKRRVDWLTEKMREANFTVSSMHGDMPQKERESIMREFRSGASRVLISTDVWARGLDVPQV
IF42_hu   ITQAVIFLNTRRKVDWLTEKMHARDFTVSALHGDMDQKERDVIMREFRSGSSRVLITTDLLARGIDVQQV
IF41_hu   ITQAVIFINTRRKVDWLTEKMHARDFTVSAMHGDMDQKERDVIMREFRSGSSRVLITTDLLARGIDVQQV
eIF4A_sc  VTQAVIFCNTRRKVEELTTKLRNDRFTVSAIYSDLPQQERDTIMREFRSGSSRILISTDLLARGIDVQQV
           :*:***  **:***:  :: *:.   .:  ..***::_:: ::.*: :*_ ****:**:*:*:*: ***:** :*

               VI
LeIF      NIVINFDLPTNRENYLHRIGRGGRYGRKGVAINFVTEKDVELLHEIEAHYHTQIDELFVDFAAYIGE
DDX48_hu  SLIINYDLPMNRELYIHRIGRSGRYGRKGVAINFVKNDDIRILRDIEQYYSTQIDEMPMNVADLI--
IF42_hu   SLVINYDLPTNRENYIHRIGRGGRFGRKGVAINFVTEEDKRILRDIETFYNTTVEEMPMNVADLI--
IF41_hu   SLVINYDLPTNRENYIHRIGRGGRFGRKGVAINMMVTEEDKRTLRDIETFYNTSIEEMPLNVADLI--
eIF4A_sc  SLVINYDLPANRENYIHRIGRGGRFGRKGVAINFVTNEDVGAMRELEKFYSTQIEELPSDIATLLN-
           _.:.**:*** *:* *:*****_**:********:*._:.*    :::*  _* * ::*:* :_* :
```

FIGURE 12

Europäisches
Patentamt

European
Patent Office

Office européen
des brevets

## RAPPORT DE RECHERCHE EUROPEENNE

Numéro de la demande

EP 08 29 0522

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | WO 2008/023077 A (TRINITY COLLEGE DUBLIN [IE]; BOWIE ANDREW [IE]; SCHROEDER MARTINA [IE]) 28 février 2008 (2008-02-28) | 1,5,6 | INV. A61K38/43 A61P35/00 |
| Y | * abrégé * | 1-3, 12-15 | A61P33/00 A61P33/02 |
| | * page 1, ligne 5-10 * * page 2, ligne 24 - page 3, ligne 6 * * page 4, ligne 28 - page 15, ligne 19 * * page 7, ligne 1 - page 9, ligne 3 * * page 15, ligne 19 - page 17, ligne 11 * * page 26, ligne 10 - page 27, ligne 18; revendications 17-20 * ----- | | |
| X | WO 99/29341 A (CORIXA CORP [US]) 17 juin 1999 (1999-06-17) * abrégé * * page 1, ligne 6-10 * * page 2, ligne 10 - page 4, ligne 25 * * page 9, ligne 33 - page 10, ligne 20 * | 12-15 | |
| Y | * page 22, ligne 3 - page 24, ligne 33; exemples 5-10,12,15-18 * ----- | 1-3, 12-15 | DOMAINES TECHNIQUES RECHERCHES (IPC) |
| D,X | SKEIKY ET AL: "LeIF: a recombinant Leishmania protein that induces and IL-12-mediated Th cytokine profile" JOURNAL OF IMMUNOLOGY, AMERICAN ASSOCIATION OF IMMUNOLOGISTS, US, 1 janvier 1998 (1998-01-01), pages 6171-6179, XP002103156 ISSN: 0022-1767 | 12 | A61K |
| Y | * abrégé * * page 6176, colonne de gauche, alinéa 2 - colonne de droite, alinéa 2 * * page 6178, colonne de gauche, alinéa 2 - colonne de droite, alinéa 1 * * page 6179, colonne de gauche, alinéa 1-3 * ----- -/-- | 1-3, 12-15 | |

Le présent rapport a été établi pour toutes les revendications

| Lieu de la recherche | Date d'achèvement de la recherche | Examinateur |
|---|---|---|
| Berlin | 12 novembre 2008 | Mateo Rosell, A |

CATEGORIE DES DOCUMENTS CITES

X : particulièrement pertinent à lui seul
Y : particulièrement pertinent en combinaison avec un
   autre document de la même catégorie
A : arrière-plan technologique
O : divulgation non-écrite
P : document intercalaire

T : théorie ou principe à la base de l'invention
E : document de brevet antérieur, mais publié à la
   date de dépôt ou après cette date
D : cité dans la demande
L : cité pour d'autres raisons

& : membre de la même famille, document correspondant

EPO FORM 1503 03.82 (P04C02)

Europäisches
Patentamt

European
Patent Office

Office européen
des brevets

**RAPPORT DE RECHERCHE EUROPEENNE**

Numéro de la demande

EP 08 29 0522

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| D,X | PROBST P ET AL: "A Leishmania protein that modulates interleukin (IL)-12, IL-10 and tumor necrosis factor-[alpha] production and expression of B7-1 in human monocyte-derived antigen-presenting cells" EUROPEAN JOURNAL OF IMMUNOLOGY 199710 DE, vol. 27, no. 10, octobre 1997 (1997-10), pages 2634-2642, XP002503337 ISSN: 0014-2980 | 12 | |
| Y | * abrégé *  *  page 2634, colonne de droite, alinéa 1 - page 2635, colonne de gauche, alinéa 3 *  * page 2639, colonne de droite, dernier alinéa - page 2641, colonne de droite, alinéa 2 *  ----- | 1-3, 12-15 | |
| D,Y | BARHOUMI M ET AL: "Leishmania infantum LeIF protein is an ATP-dependent RNA helicase and an eIF4A-like factor that inhibits translation in yeast" FEBS JOURNAL 200611 GB, vol. 273, no. 22, novembre 2006 (2006-11), pages 5086-5100, XP002503338 ISSN: 1742-464X 1742-4658 * abrégé * * page 5086, colonne de droite, alinéa 2 - page 5087, colonne de gauche, alinéa 1 * * page 5088, colonne de gauche, alinéa 2 - page 5089, colonne de gauche, alinéa 1; figure 1; tableau 1 * * page 5093, colonne de droite, alinéa 3 - page 5094, colonne de gauche, alinéa 1 * * page 5094, colonne de gauche, dernier alinéa - page 5096, colonne de gauche, alinéa 1 *  ----- | 1-3, 12-15 | **DOMAINES TECHNIQUES RECHERCHES (IPC)** |

-/--

Le présent rapport a été établi pour toutes les revendications

| Lieu de la recherche | Date d'achèvement de la recherche | Examinateur |
|---|---|---|
| Berlin | 12 novembre 2008 | Mateo Rosell, A |

CATEGORIE DES DOCUMENTS CITES

X : particulièrement pertinent à lui seul
Y : particulièrement pertinent en combinaison avec un
autre document de la même catégorie
A : arrière-plan technologique
O : divulgation non-écrite
P : document intercalaire

T : théorie ou principe à la base de l'invention
E : document de brevet antérieur, mais publié à la
date de dépôt ou après cette date
D : cité dans la demande
L : cité pour d'autres raisons

    .................................................................................
& : membre de la même famille, document correspondant

EPO FORM 1503 03.82 (P04C02)

Europäisches
Patentamt

European
Patent Office

Office européen
des brevets

**RAPPORT DE RECHERCHE EUROPEENNE**

Numéro de la demande

EP 08 29 0522

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| Y | ROCAK S ET AL: "Characterization of the ATPase and unwinding activities of the yeast DEAD-box protein Has1p and the analysis of the roles of the conserved motifs"<br>NUCLEIC ACIDS RESEARCH 2005 GB,<br>vol. 33, no. 3, 2005, pages 999-1009,<br>XP002503659<br>ISSN: 0305-1048 1362-4962<br>* abrégé; tableau 1 *<br>* page 1006, colonne de gauche, alinéa 1 - colonne de droite, alinéa 3 *<br>* page 1007, colonne de droite, alinéa 2 *<br>----- | 1-3,<br>12-15 | |
| Y | SONG PING ET AL: "RNA helicase-related genes of Plasmodium falciparum and Plasmodium cynomolgi"<br>BIOCHEMICAL AND BIOPHYSICAL RESEARCH COMMUNICATIONS,<br>vol. 255, no. 2,<br>16 février 1999 (1999-02-16), pages 312-316, XP002503339<br>ISSN: 0006-291X<br>* abrégé *<br>* page 312, colonne de gauche, alinéa 1 - colonne de droite, alinéa 2 *<br>* page 313, colonne de droite, dernier alinéa - page 315, colonne de droite, dernier alinéa; figure 1 *<br>-----<br><br>-/-- | 1-3,<br>12-15 | DOMAINES TECHNIQUES RECHERCHES (IPC) |

Le présent rapport a été établi pour toutes les revendications

| Lieu de la recherche | Date d'achèvement de la recherche | Examinateur |
|---|---|---|
| Berlin | 12 novembre 2008 | Mateo Rosell, A |

CATEGORIE DES DOCUMENTS CITES

X : particulièrement pertinent à lui seul
Y : particulièrement pertinent en combinaison avec un
    autre document de la même catégorie
A : arrière-plan technologique
O : divulgation non-écrite
P : document intercalaire

T : théorie ou principe à la base de l'invention
E : document de brevet antérieur, mais publié à la
    date de dépôt ou après cette date
D : cité dans la demande
L : cité pour d'autres raisons
...................................................................................................
& : membre de la même famille, document correspondant

EPO FORM 1503 03.82 (P04C02)

**Europäisches Patentamt**
**European Patent Office**
**Office européen des brevets**

## RAPPORT DE RECHERCHE EUROPEENNE

Numéro de la demande

EP 08 29 0522

### DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| Y | ABDELHALEEM MOHAMED: "Do human RNA helicases have a role in cancer?" BIOCHIMICA ET BIOPHYSICA ACTA 6 JUL 2004, vol. 1704, no. 1, 6 juillet 2004 (2004-07-06), pages 37-46, XP004517447 ISSN: 0006-3002 * abrégé * * page 37, colonne de droite, alinéa 2 - page 42, colonne de droite, alinéa 1; tableau 1 * ----- | 1-3, 12-15 | |
| D,Y | CORDIN OLIVIER ET AL: "The DEAD-box protein family of RNA helicases" GENE, vol. 367, février 2006 (2006-02), pages 17-37, XP005298313 ISSN: 0378-1119 * abrégé * * page 18, colonne de gauche, dernier alinéa - colonne de droite, alinéa 2; figure 1 * * page 21, colonne de gauche, alinéa 1 - page 25, colonne de droite, alinéa 2; figures 4,5; tableau 1 * * page 31, colonne de droite, alinéa 1 - page 34, colonne de gauche, alinéa 2 * ----- | 1-3, 12-15 | DOMAINES TECHNIQUES RECHERCHES (IPC) |

Le présent rapport a été établi pour toutes les revendications

| Lieu de la recherche | Date d'achèvement de la recherche | Examinateur |
|---|---|---|
| Berlin | 12 novembre 2008 | Mateo Rosell, A |

CATEGORIE DES DOCUMENTS CITES

X : particulièrement pertinent à lui seul
Y : particulièrement pertinent en combinaison avec un
     autre document de la même catégorie
A : arrière-plan technologique
O : divulgation non-écrite
P : document intercalaire

T : théorie ou principe à la base de l'invention
E : document de brevet antérieur, mais publié à la
     date de dépôt ou après cette date
D : cité dans la demande
L : cité pour d'autres raisons

 

& : membre de la même famille, document correspondant

EPO FORM 1503 03.82 (P04C02)

**ANNEXE AU RAPPORT DE RECHERCHE EUROPEENNE
RELATIF A LA DEMANDE DE BREVET EUROPEEN NO.**

EP 08 29 0522

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

12-11-2008

| Document brevet cité au rapport de recherche | Date de publication | Membre(s) de la famille de brevet(s) | Date de publication |
|---|---|---|---|
| WO 2008023077 A | 28-02-2008 | AUCUN | |
| WO 9929341 A | 17-06-1999 | AU 1724999 A | 28-06-1999 |

EPO FORM P0460

Pour tout renseignement concernant cette annexe : voir Journal Officiel de l'Office européen des brevets, No.12/82

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **Altmann et al.** *EMBO,* 1995, vol. 14, 3820-3827
- **Badaro et al.** *Braz J Infect Dis,* 2001, vol. 5, 223-232
- **Banroques et al.** *Mol Cell Biol,* 2008, vol. 28, 3359-71
- **Barhoumi et al.** *FEBS J,* 2006, vol. 273, 5086-5100
- **Bates et al.** *EMBO J,* 2005, vol. 24, 543-553
- **Bente et al.** *Proteomics,* 2003, vol. 3 (9), 1811-29
- **Benz et al.** *Structure Fold Des,* 1999, vol. 7, 671-679
- **Bizebard et al.** *Biochemistry,* 2004, vol. 43, 7857-7866
- **Carmel ; Matthews.** *RNA,* 2004, vol. 10, 66-74
- **Caruthers et al.** *Proc Natl Acad Sci USA,* 2000, vol. 97, 13080-13085
- **Caruthers ; McKay.** *Curr Opin Struct Biol,* 2002, vol. 12, 123-133
- **Chan et al.** *RNA,* 2004, vol. 10, 200-209
- **Chang et al.** *Nucleic Acids Res,* 1997, vol. 25, 5033-5040
- **Chuang et al.** *Science,* 1997, vol. 275, 1468-1471
- **Coler et al.** *Infect Immun,* 2002, vol. 70, 4215-4225
- **Coler et al.** *Infect Immun,* 2007, vol. 75, 4648-4654
- **Cordin et al.** *EMBO J,* 2004, vol. 23, 2478-2487
- **Cordin et al.** *Gene,* 2006, vol. 367, 17-37
- **de la Cruz et al.** *Proc Natl Acad Sci USA,* 1997, vol. 94 (10), 5201-6
- **de la Cruz et al.** *Trends Biochem Sci,* 1999, vol. 24, 192-198
- **Emery et al.** *Mol Microbiol,* 2004, vol. 52, 141-158
- **Ferraiuolo et al.** *Proc Natl Acad Sci USA,* 2004, vol. 101, 4118-4123
- **Flores-Rozas ; Hurwitz.** *J Biol Chem,* 1993, vol. 268, 21372-21383
- **Fuller-Pace et al.** *EMBO J,* 1993, vol. 12, 3619-3626
- **Gillian ; Svaren.** *J Biol Chem,* 2004, vol. 279, 9056-9063
- **Gorbalenya et al.** *Nucleic Acids Res.,* 1989, vol. 17 (12), 4713-30
- **Gorbalenya ; Koonin.** *Curr Opin Struct Biol,* 1993, vol. 3, 419-429
- **Hall ; Matson.** *Mol Microbiol,* 1999, vol. 34, 867-877
- **He et al.** *World J Gastroenterol,* 2008, vol. 14 (4), 532-40
- **Hirling et al.** *Nature,* 1989, vol. 339, 562-564
- **Hodge et al.** *EMBO J,* 1999, vol. 18, 5778-5788
- **Huang ; Liu.** *J Biol Chem,* 2002, vol. 277, 12810-12815
- **Ilyina et al.** *J Mol Evol.,* 1992, vol. 34 (4), 351-7
- **Iost et al.** *JBiol Chem,* 1999, vol. 274, 17677-17683
- **Johnson ; McKay.** *RNA,* 1999, vol. 5, 1526-1534
- **Kadare ; Haenni.** *J Virol,* 1997, vol. 71, 2583-2590
- **Kim et al.** *Structure,* 1998, vol. 6, 89-100
- **Korolev et al.** *Cell,* 1997, vol. 90, 635-647
- **Kossen ; Uhlenbeck.** *Nucleic Acids Res,* 1999, vol. 27, 3811-3820
- **Kossen et al.** *J Mol Biol,* 2002, vol. 324, 625-636
- **Kressler et al.** *Mol Cell Biol,* 1997, vol. 17, 7283-7294
- **Kressler et al.** *Mol Cell Biol,* 1999, vol. 19 (12), 7897-912
- **Lamm et al.** *Nucleic Acids Res,* 1996, vol. 24, 3739-3747
- **Lenzi et al.** *J. Transl Med.,* 2007, vol. 5, 66
- **Li et al.** *Mol Cell Biol,* 1999, vol. 19, 7336-7346
- **Linder.** *Biol Cell,* 2003, vol. 95 (3-4), 157-167
- **Linder et al.** *Nature,* 1989, vol. 337, 121-122
- **Linder ; Stutz.** *Curr Biol,* 2002, vol. 11, 961-963
- **Lorsh ; Herschlag.** *Biochemistry,* 1998, vol. 37, 2180-2193
- **Luking et al.** *Crit Rev Biochem Mol Biol,* 1998, vol. 33, 259-296
- **Myler et al.** *Proc Natl Acad Sci USA,* 1999, vol. 96, 2902-2906
- **Noueiry et al.** *Proc Natl Acad Sci USA,* 2000, vol. 97, 12985-12990
- **Nugent et al.** *Mol Biochem Parasitol,* 2004, vol. 136, 51-62
- **Palacios et al.** *Nature,* 2004, vol. 427, 753-757

- **Patel ; Picha.** *Annu Rev Biochemi,* 2000, vol. 69, 651-697
- **Pause ; Sonenberg.** *EMBO J,* 1992, vol. 11, 2643-2653
- **Pause et al.** *Mol Cell Biol,* 1993, vol. 13, 6789-6798
- **Probst et al.** *Eur J Immunol,* 1997, vol. 27, 2634-2642
- **Reed ; Hurt.** *Cell,* 2002, vol. 108, 523-531
- **Rocak ; Linder.** *Nat Rev Mol Cell Biol,* 2004, vol. 5, 232-241
- **Rocak et al.** *Nucleic Acids Res,* 2005, vol. 33, 999-10009
- **Rogers et al.** *J Biol Chem,* 1999, vol. 274, 12236-12244
- **Rogers et al.** *J Biol Chem,* 2001, vol. 276, 12598-12608
- **Rogers et al.** *J Biol Chem,* 2001, vol. 276, 30914-30922
- **Rogers et al.** *Prog Nucleic Acid Res Mol Biol,* 2002, vol. 72, 307-331
- **Rossler et al.** *Nucleic Acids Res,* 2001, vol. 29, 2088-2096
- **Rozen et al.** *Mol Cell Biol,* 1990, vol. 10, 1134-1144
- **Sabel et al.** *Surgery,* 2007, vol. 142, 749-760
- **Salay et al.** *Clin Vaccine Immunol,* 2007, vol. 14, 1173-1181
- **Schmitt et al.** *EMBO J,* 1999, vol. 18, 4332-4347
- **Schmid ; Linder.** *Mol Microbiol,* 1992, vol. 6, 283-291
- **Schwer ; Mezaros.** *EMBO J,* 2000, vol. 19, 6582-6591
- **Shi et al.** *Proc Natl Acad Sci USA,* 2004, vol. 101, 17628-17633
- **Shibuya et al.** *Nat Struct Mol Biol,* 2004, vol. 11, 346-351
- **Shiratori et al.** *Cancer Sci.,* 2007, vol. 98, 1936-1942
- **Shneider et al.** *J Biol Chem,* 2004, vol. 279, 8617-8626
- **Silverman et al.** *Gene,* 2003, vol. 312, 1-16
- **Singleton ; Wigley.** *J Bacteriol,* 2002, vol. 184, 1819-1826
- **Skeiky et al.** *J Exp Med,* 1995, vol. 181, 1527-1537
- **Skeiky et al.** *J Immunol,* 1998, vol. 161, 6171-6179
- **Son ; Kim.** *Arch Pharm Res.,* 2007, vol. 30 (8), 1047-50
- **Sonenberg ; Diver.** *Curr Opin Stuct Biol,* 2003, vol. 13, 56-63
- **Staley ; Gutherie.** *Mol Cell,* 1999, vol. 3, 55-64
- **Story ; Steitz.** *Nature,* 1992, vol. 55, 374-376
- **Story et al.** *Proc Natl Acad Sci USA,* 2001, vol. 98, 1465-1470
- **Strasser ; Hurt.** *Nature,* 2001, vol. 413, 648-652
- **Subramanya et al.** *Nature,* 1996, vol. 28, 379-383
- **Svitkin et al.** *RNA,* 2001, vol. 7, 382-394
- **Tanner.** *Cell Cycle,* 2003, vol. 2, 9-18
- **Tanner et al.** *Mol Cell,* 2003, vol. 11, 127-138
- **Tanner ; Linder.** *Mol Cell,* 2001, vol. 8, 251-262
- **Theis et al.** *EMBO J,* 1999, vol. 18, 6899-6907
- **Tseng et al.** *EMBO J,* 1998, vol. 17, 2651-2662
- **Tsu ; Uhlenbeck.** *Biochemistry,* 1998, vol. 37, 16989-16996
- **Tsu et al.** *RNA,* 2001, vol. 7, 702-709
- **Tuteja ; Tuteja.** *Eur J Biochem,* 2004, vol. 271, 1835-1840
- **Tuteja ; Tuteja.** *Eur J Biochem,* 2004, vol. 271, 1849-1863
- **Valdez et al.** *Eur J Biochem,* 1997, vol. 250, 800-807
- **Valdez.** *Eur J Biochem,* 2000, vol. 267 (21), 6395-402
- **Velankar et al.** *Cell,* 1999, vol. 97, 75-84
- **Walker et al.** *EMBO J,* 1982, vol. 1, 945-951
- **Weiss et al.** *Expert Opin Biol Ther,* 2007, vol. 7, 1705-1721
- **Wilson et al.** *BMC Mol Biol,* 2004, vol. 5, 11
- **Xu et al.** *EMBO J,* 2004, vol. 23, 376-385
- **Yan et al.** *Mol Cell Biol,* 2003, vol. 23, 414-423
- **Yao et al.** *Nat Struct Biol,* 1997, vol. 4, 463-467
- **Ye et al.** *Biochim Biophys Acta,* 2004, vol. 1659, 1-18
- **Zhao et al.** *EMBO J,* 2002, vol. 21, 1177-1187
- **Zhao et al.** *Structure,* 2004, vol. 12, 1373-1381

SEQUENCE LISTING

<110> INSTITUT PASTEUR
INSTITUT PASTEUR DE TUNIS

<120> UTILISATION D'UNE ARN HELICASE A BOITE DEAD POUR INDUIRE LA
PRODUCTION DE CYTOKINES

<130> XRNcp226/162EP

<160> 56

<170> PatentIn version 3.3

<210> 1
<211> 9
<212> PRT
<213> Artificial

<220>
<223> motif Q

<220>
<221> VARIANT
<222> (2)..(2)
<223> / replace = "Trp"

/ replace = "Tyr"

<220>
<221> VARIANT
<222> (3)..(3)
<223> / replace = "Ala", "Arg", "Asn", "Asp", "Cys", "Gln", "Gly",
"His", "Ile", "Leu", "Lys", "Met", "Phe", "Pro", "Ser", "Thr",
"Trp", "Tyr" or "Val"

<220>
<221> VARIANT
<222> (4)..(4)
<223> / replace = "Asp"

/ replace = "His"

/ replace = "Lys"

/ replace = "Arg"

<220>
<221> VARIANT
<222> (6)..(6)
<223> / replace = "Thr"

<220>
<221> VARIANT
<222> (7)..(7)
<223> / replace = "Phe", "Gly", "Ile", "Leu", "Met", "Pro", "Val",
"Trp" or "Tyr"

<220>
<221> VARIANT
<222> (8)..(8)
<223> / replace = "Leu"

/ replace = "Val"

<400> 1

Gly Phe Glu Glu Pro Ser Ala Ile Gln
1               5

<210>   2
<211>   8
<212>   PRT
<213>   Artificial

<220>
<223>   motif I


<220>
<221>   VARIANT
<222>   (2)..(2)
<223>   / replace = "Ala", "Arg", "Asn", "Asp", "Cys", "Glu", "Gln",
        "Gly", "His", "Ile", "Leu", "Lys", "Met", "Phe", "Pro", "Thr",
        "Trp", "Tyr" or "Val"

<220>
<221>   VARIANT
<222>   (3)..(3)
<223>   / replace = "Ala", "Arg", "Asn", "Asp", "Cys", "Glu", "Gln",
        "Gly", "His", "Ile", "Leu", "Lys", "Met", "Phe", "Pro", "Ser",
        "Trp", "Tyr" or "Val"

<220>
<221>   VARIANT
<222>   (5)..(5)
<223>   / replace = "Ser"

<400>   2

Ala Gln Ser Gly Thr Gly Lys Thr
1               5


<210>   3
<211>   6
<212>   PRT
<213>   Artificial

<220>
<223>   motif Ia

<400>   3

Pro Thr Arg Glu Leu Ala
1               5


<210>   4
<211>   5
<212>   PRT
<213>   Artificial

<220>
<223>   motif Ib


<220>
<221>   VARIANT
<222>   (5)..(5)
<223>   / replace = 'Ile"

/ replace = "Leu"

```
<400>  4

Thr Pro Gly Arg Val
1               5



<210>  5
<211>  4
<212>  PRT
<213>  Artificial

<220>
<223>  motif II

<400>  5

Asp Glu Ala Asp
1



<210>  6
<211>  3
<212>  PRT
<213>  Artificial

<220>
<223>  motif III

<400>  6

Ser Ala Thr
1



<210>  7
<211>  9
<212>  PRT
<213>  Artificial

<220>
<223>  motif IV



<220>
<221>  VARIANT
<222>  (1)..(1)
<223>  / replace = "Ile"

/ replace = "Leu"

<220>
<221>  VARIANT
<222>  (2)..(2)
<223>  / replace = "Val"

/ replace = "Leu"

<220>
<221>  VARIANT
<222>  (4)..(4)
<223>  / replace = "Ala", "Phe", "Gly", "Ile", "Leu", "Met", "Pro",
       "Val", "Trp" or "Tyr"

<220>
<221>  VARIANT
<222>  (5)..(5)
<223>  / replace = "Ala", "Arg", "Asn", "Asp", "Cys", "Gln", "Gly",
       "His", "Ile", "Leu", "Lys", "Met", "Phe", "Pro", "Ser", "Thr",
```

"Trp", "Tyr" or "Val"

```
<220>
<221>  VARIANT
<222>  (7)..(7)
<223>  / replace = "Ala", "Asn", "Asp", "Cys", "Glu", "Gln", "Gly",
       "His", "Ile", "Leu", "Lys", "Met", "Phe", "Pro", "Ser", "Thr",
       "Trp", "Tyr" or "Val"

<220>
<221>  VARIANT
<222>  (8)..(8)
<223>  / replace = "His"
```

/ replace = "Lys"

```
<220>
<221>  VARIANT
<222>  (9)..(9)
<223>  / replace = "Asp", "Glu", "His" or "Lys"

<400>  7
```

Val Ile Phe Cys Asn Thr Arg Arg Lys
1               5

```
<210>  8
<211>  9
<212>  PRT
<213>  Artificial

<220>
<223>  motif V


<220>
<221>  VARIANT
<222>  (2)..(2)
<223>  / replace = "Asn"

<220>
<221>  VARIANT
<222>  (3)..(3)
<223>  / replace = "Ala", "Arg", "Asn", "Asp", "Cys", "Glu", "Gln",
       "Gly", "His", "Ile", "Lys", "Met", "Phe", "Pro", "Ser", "Thr",
       "Trp", "Tyr" or "Val"

<220>
<221>  VARIANT
<222>  (4)..(4)
<223>  / replace = "Ala", "Arg", "Asn", "Asp", "Cys", "Glu", "Gln",
       "Gly", "His", "Ile", "Lys", "Met", "Phe", "Pro", "Ser", "Thr",
       "Trp", "Tyr" or "Val"

<220>
<221>  VARIANT
<222>  (8)..(8)
<223>  / replace = "Leu"
```

/ replace = "Val"

```
<400>  8
```

Thr Asp Leu Leu Ala Arg Gly Ile Asp
1               5

<210> 9
<211> 8
<212> PRT
<213> Artificial

<220>
<223> motif VI

<220>
<221> VARIANT
<222> (3)..(3)
<223> / replace "Leu"

/ replace "Val"

<220>
<221> VARIANT
<222> (6)..(6)
<223> / replace "Gly"

/ replace "Ser"

<400> 9

His Arg Ile Gly Arg Thr Gly Arg
1               5


<210> 10
<211> 1212
<212> DNA
<213> Leishmania infantum


<220>
<221> CDS
<222> (1)..(1212)

<400> 10
```
atg gcg cag aat gat aag atc gcc ccc cag gac cag gac tcc ttc ctc      48
Met Ala Gln Asn Asp Lys Ile Ala Pro Gln Asp Gln Asp Ser Phe Leu
1               5                  10                  15

gat gac cag ccc ggc gtt cgc ccg atc ccg tcc ttc gac gac atg ccg      96
Asp Asp Gln Pro Gly Val Arg Pro Ile Pro Ser Phe Asp Asp Met Pro
                20                  25                  30

ctg cac cag aac ctg ctg cgc ggc atc tac tcg tac ggg ttc gag aag     144
Leu His Gln Asn Leu Leu Arg Gly Ile Tyr Ser Tyr Gly Phe Glu Lys
            35                  40                  45

ccg tcc agc atc cag cag cgc gcg ata gcc ccc ttc acg cgc ggc ggc     192
Pro Ser Ser Ile Gln Gln Arg Ala Ile Ala Pro Phe Thr Arg Gly Gly
        50                  55                  60

gac atc atc gcg cag gcc cag tcc ggt acc ggc aag acg ggt gcc ttc     240
Asp Ile Ile Ala Gln Ala Gln Ser Gly Thr Gly Lys Thr Gly Ala Phe
65                  70                  75                  80

tcc atc ggt ctg ctg cag cgc ctg gac ttc cgc cac aac ctg atc cag     288
Ser Ile Gly Leu Leu Gln Arg Leu Asp Phe Arg His Asn Leu Ile Gln
                85                  90                  95

ggc ctc gtg ctc tcc ccc act cgc gag ctg gcc ctg cag acg gcg gag     336
Gly Leu Val Leu Ser Pro Thr Arg Glu Leu Ala Leu Gln Thr Ala Glu
            100                 105                 110
```

```
gtg atc agc cgc atc ggt gag ttc ctg tcg aac agc tcc aag ttc tgc          384
Val Ile Ser Arg Ile Gly Glu Phe Leu Ser Asn Ser Ser Lys Phe Cys
        115                 120                 125

gag acc ttt gtc ggc ggc acg cgc gtg cag gat gac ctg cgc aag ctg          432
Glu Thr Phe Val Gly Gly Thr Arg Val Gln Asp Asp Leu Arg Lys Leu
    130                 135                 140

cag gcc ggc gtc atc gtt gcc gtg ggc acg ccg ggc cgc gtg tcc gac          480
Gln Ala Gly Val Ile Val Ala Val Gly Thr Pro Gly Arg Val Ser Asp
145                 150                 155                 160

gtg atc aag cgc ggc gcg ctg cgc acg gag tcg ctg cgc gtg ctg gtg          528
Val Ile Lys Arg Gly Ala Leu Arg Thr Glu Ser Leu Arg Val Leu Val
                165                 170                 175

ctc gac gag gct gat gag atg ctg tct cag ggc ttc gcg gac cag att          576
Leu Asp Glu Ala Asp Glu Met Leu Ser Gln Gly Phe Ala Asp Gln Ile
                180                 185                 190

tac gag atc ttc cgc ttc ctg ccg aag gac atc cag gtc gcg ctc ttc          624
Tyr Glu Ile Phe Arg Phe Leu Pro Lys Asp Ile Gln Val Ala Leu Phe
            195                 200                 205

tcc gcc acg atg ccg gag gag gtg ctg gag ctg acg aag aag ttc atg          672
Ser Ala Thr Met Pro Glu Glu Val Leu Glu Leu Thr Lys Lys Phe Met
        210                 215                 220

cgc gac ccc gtg cgc att ctc gtg aag cgc gag agc ctg acg ctg gag          720
Arg Asp Pro Val Arg Ile Leu Val Lys Arg Glu Ser Leu Thr Leu Glu
225                 230                 235                 240

ggc atc aag cag ttc ttc atc gcc gtc gaa gag gag cac aag ctg gac          768
Gly Ile Lys Gln Phe Phe Ile Ala Val Glu Glu Glu His Lys Leu Asp
                245                 250                 255

acg ctg atg gac ctg tac gag acc gtg tcc atc gcg cag tcc gtc atc          816
Thr Leu Met Asp Leu Tyr Glu Thr Val Ser Ile Ala Gln Ser Val Ile
                260                 265                 270

ttc gcc aac acg cgc cgc aag gtg gac tgg atc gcc gag aag ctg aac          864
Phe Ala Asn Thr Arg Arg Lys Val Asp Trp Ile Ala Glu Lys Leu Asn
            275                 280                 285

cag agc aac cac acc gtc agc agc atg cac gcc gag atg ccc aag agc          912
Gln Ser Asn His Thr Val Ser Ser Met His Ala Glu Met Pro Lys Ser
        290                 295                 300

gac cgc gag cgc gtc atg aac acc ttc cgc agc ggc agc tcc cgc gtg          960
Asp Arg Glu Arg Val Met Asn Thr Phe Arg Ser Gly Ser Ser Arg Val
305                 310                 315                 320

ctc gtc acg acc gac ctc gtg gcg cgc ggt atc gac gtg cac cac gtg         1008
Leu Val Thr Thr Asp Leu Val Ala Arg Gly Ile Asp Val His His Val
                325                 330                 335

aac atc gtc atc aac ttc gac ctg cca acg aac aag gag aac tac ctg         1056
Asn Ile Val Ile Asn Phe Asp Leu Pro Thr Asn Lys Glu Asn Tyr Leu
                340                 345                 350

cat cgc att ggc cgc ggc ggc cgc tac ggc cgt aag ggt gtt gcc atc         1104
His Arg Ile Gly Arg Gly Gly Arg Tyr Gly Arg Lys Gly Val Ala Ile
            355                 360                 365

aac ttc gtg acg gag aag gac gtg gag ctg ctg cac gag atc gag gcg         1152
Asn Phe Val Thr Glu Lys Asp Val Glu Leu Leu His Glu Ile Glu Ala
        370                 375                 380
```

```
cac tac cac acg cag atc gac gag ctc ccg gtc gac ttc gct gcc tac    1200
His Tyr His Thr Gln Ile Asp Glu Leu Pro Val Asp Phe Ala Ala Tyr
385             390             395             400

ctt ggc gag taa                                                    1212
Leu Gly Glu
```

<210> 11
<211> 403
<212> PRT
<213> Leishmania infantum

<400> 11

```
Met Ala Gln Asn Asp Lys Ile Ala Pro Gln Asp Gln Asp Ser Phe Leu
1               5               10              15

Asp Asp Gln Pro Gly Val Arg Pro Ile Pro Ser Phe Asp Asp Met Pro
            20              25              30

Leu His Gln Asn Leu Leu Arg Gly Ile Tyr Ser Tyr Gly Phe Glu Lys
        35              40              45

Pro Ser Ser Ile Gln Gln Arg Ala Ile Ala Pro Phe Thr Arg Gly Gly
    50              55              60

Asp Ile Ile Ala Gln Ala Gln Ser Gly Thr Gly Lys Thr Gly Ala Phe
65              70              75              80

Ser Ile Gly Leu Leu Gln Arg Leu Asp Phe Arg His Asn Leu Ile Gln
            85              90              95

Gly Leu Val Leu Ser Pro Thr Arg Glu Leu Ala Leu Gln Thr Ala Glu
            100             105             110

Val Ile Ser Arg Ile Gly Glu Phe Leu Ser Asn Ser Ser Lys Phe Cys
        115             120             125

Glu Thr Phe Val Gly Gly Thr Arg Val Gln Asp Asp Leu Arg Lys Leu
    130             135             140

Gln Ala Gly Val Ile Val Ala Val Gly Thr Pro Gly Arg Val Ser Asp
145             150             155             160

Val Ile Lys Arg Gly Ala Leu Arg Thr Glu Ser Leu Arg Val Leu Val
            165             170             175

Leu Asp Glu Ala Asp Glu Met Leu Ser Gln Gly Phe Ala Asp Gln Ile
            180             185             190

Tyr Glu Ile Phe Arg Phe Leu Pro Lys Asp Ile Gln Val Ala Leu Phe
        195             200             205
```

Ser Ala Thr Met Pro Glu Glu Val Leu Glu Leu Thr Lys Lys Phe Met
    210             215             220

Arg Asp Pro Val Arg Ile Leu Val Lys Arg Glu Ser Leu Thr Leu Glu
225             230             235             240

Gly Ile Lys Gln Phe Phe Ile Ala Val Glu Glu Glu His Lys Leu Asp
                245             250             255

Thr Leu Met Asp Leu Tyr Glu Thr Val Ser Ile Ala Gln Ser Val Ile
            260             265             270

Phe Ala Asn Thr Arg Arg Lys Val Asp Trp Ile Ala Glu Lys Leu Asn
            275             280             285

Gln Ser Asn His Thr Val Ser Ser Met His Ala Glu Met Pro Lys Ser
    290             295             300

Asp Arg Glu Arg Val Met Asn Thr Phe Arg Ser Gly Ser Ser Arg Val
305             310             315             320

Leu Val Thr Thr Asp Leu Val Ala Arg Gly Ile Asp Val His His Val
            325             330             335

Asn Ile Val Ile Asn Phe Asp Leu Pro Thr Asn Lys Glu Asn Tyr Leu
            340             345             350

His Arg Ile Gly Arg Gly Gly Arg Tyr Gly Arg Lys Gly Val Ala Ile
        355             360             365

Asn Phe Val Thr Glu Lys Asp Val Glu Leu Leu His Glu Ile Glu Ala
    370             375             380

His Tyr His Thr Gln Ile Asp Glu Leu Pro Val Asp Phe Ala Ala Tyr
385             390             395             400

Leu Gly Glu

<210> 12
<211> 395
<212> PRT
<213> Saccharomyces cerevisiae

<400> 12

Met Ser Glu Gly Ile Thr Asp Ile Glu Glu Ser Gln Ile Gln Thr Asn
1               5               10              15

Tyr Asp Lys Val Val Tyr Lys Phe Asp Asp Met Glu Leu Asp Glu Asn
            20              25              30

Leu Leu Arg Gly Val Phe Gly Tyr Gly Phe Glu Glu Pro Ser Ala Ile

|  | 35 |  |  |  | 40 |  |  |  | 45 |  |
|---|---|---|---|---|---|---|---|---|---|---|

Gln Gln Arg Ala Ile Met Pro Ile Ile Glu Gly His Asp Val Leu Ala
    50                55                60

Gln Ala Gln Ser Gly Thr Gly Lys Thr Gly Thr Phe Ser Ile Ala Ala
65                70                75                80

Leu Gln Arg Ile Asp Thr Ser Val Lys Ala Pro Gln Ala Leu Met Leu
            85                90                95

Ala Pro Thr Arg Glu Leu Ala Leu Gln Ile Gln Lys Val Val Met Ala
            100                105                110

Leu Ala Phe His Met Asp Ile Lys Val His Ala Cys Ile Gly Gly Thr
        115                120                125

Ser Phe Val Glu Asp Ala Glu Gly Leu Arg Asp Ala Gln Ile Val Val
    130                135                140

Gly Thr Pro Gly Arg Val Phe Asp Asn Ile Gln Arg Arg Arg Phe Arg
145                150                155                160

Thr Asp Lys Ile Lys Met Phe Ile Leu Asp Glu Ala Asp Glu Met Leu
            165                170                175

Ser Ser Gly Phe Lys Glu Gln Ile Tyr Gln Ile Phe Thr Leu Leu Pro
            180                185                190

Pro Thr Thr Gln Val Val Leu Leu Ser Ala Thr Met Pro Asn Asp Val
        195                200                205

Leu Glu Val Thr Thr Lys Phe Met Arg Asn Pro Val Arg Ile Leu Val
    210                215                220

Lys Lys Asp Glu Leu Thr Leu Glu Gly Ile Lys Gln Phe Tyr Val Asn
225                230                235                240

Val Glu Glu Glu Glu Tyr Lys Tyr Glu Cys Leu Thr Asp Leu Tyr Asp
            245                250                255

Ser Ile Ser Val Thr Gln Ala Val Ile Phe Cys Asn Thr Arg Arg Lys
            260                265                270

Val Glu Glu Leu Thr Thr Lys Leu Arg Asn Asp Lys Phe Thr Val Ser
        275                280                285

Ala Ile Tyr Ser Asp Leu Pro Gln Gln Glu Arg Asp Thr Ile Met Lys
    290                295                300

Glu Phe Arg Ser Gly Ser Ser Arg Ile Leu Ile Ser Thr Asp Leu Leu

```
                305                      310                      315                      320

Ala Arg Gly Ile Asp Val Gln Gln Val Ser Leu Val Ile Asn Tyr Asp
                325                      330                      335

Leu Pro Ala Asn Lys Glu Asn Tyr Ile His Arg Ile Gly Arg Gly Gly
                340                      345                      350

Arg Phe Gly Arg Lys Gly Val Ala Ile Asn Phe Val Thr Asn Glu Asp
                355                      360                      365

Val Gly Ala Met Arg Glu Leu Glu Lys Phe Tyr Ser Thr Gln Ile Glu
        370                      375                      380

Glu Leu Pro Ser Asp Ile Ala Thr Leu Leu Asn
385                      390                      395


<210>  13
<211>  399
<212>  PRT
<213>  Saccharomyces cerevisiae

<400>  13

Met Ser Phe Asp Arg Glu Glu Asp Gln Lys Leu Lys Phe Lys Thr Ser
1               5                       10                      15

Lys Lys Leu Lys Val Ser Ser Thr Phe Glu Ser Met Asn Leu Lys Asp
            20                      25                      30

Asp Leu Leu Arg Gly Ile Tyr Ser Tyr Gly Phe Glu Ala Pro Ser Ser
            35                      40                      45

Ile Gln Ser Arg Ala Ile Thr Gln Ile Ile Ser Gly Lys Asp Val Ile
        50                      55                      60

Ala Gln Ala Gln Ser Gly Thr Gly Lys Thr Ala Thr Phe Thr Ile Gly
65                      70                      75                      80

Leu Leu Gln Ala Ile Asp Leu Arg Lys Lys Asp Leu Gln Ala Leu Ile
                85                      90                      95

Leu Ser Pro Thr Arg Glu Leu Ala Ser Gln Ile Gly Gln Val Val Lys
                100                     105                     110

Asn Leu Gly Asp Tyr Met Asn Val Asn Ala Phe Ala Ile Thr Gly Gly
            115                     120                     125

Lys Thr Leu Lys Asp Asp Leu Lys Lys Met Gln Lys His Gly Cys Gln
        130                     135                     140

Ala Val Ser Gly Thr Pro Gly Arg Val Leu Asp Met Ile Lys Lys Gln
145                     150                     155                     160
```

```
Met Leu Gln Thr Arg Asn Val Gln Met Leu Val Leu Asp Glu Ala Asp
            165                 170                 175

Glu Leu Leu Ser Glu Thr Leu Gly Phe Lys Gln Gln Ile Tyr Asp Ile
            180                 185                 190

Phe Ala Lys Leu Pro Lys Asn Cys Gln Val Val Val Val Ser Ala Thr
        195             200                 205

Met Asn Lys Asp Ile Leu Glu Val Thr Arg Lys Phe Met Asn Asp Pro
    210             215                 220

Val Lys Ile Leu Val Lys Arg Asp Glu Ile Ser Leu Glu Gly Ile Lys
225                 230             235                     240

Gln Tyr Val Val Asn Val Asp Lys Glu Glu Trp Lys Phe Asp Thr Leu
            245                 250                 255

Cys Asp Ile Tyr Asp Ser Leu Thr Ile Thr Gln Cys Val Ile Phe Cys
            260             265                 270

Asn Thr Lys Lys Lys Val Asp Trp Leu Ser Gln Arg Leu Ile Gln Ser
        275             280                 285

Asn Phe Ala Val Val Ser Met His Gly Asp Met Lys Gln Glu Glu Arg
    290             295                 300

Asp Lys Val Met Asn Asp Phe Arg Thr Gly His Ser Arg Val Leu Ile
305             310                 315                     320

Ser Thr Asp Val Trp Ala Arg Gly Ile Asp Val Gln Gln Val Ser Leu
            325                 330                 335

Val Ile Asn Tyr Asp Leu Pro Glu Ile Ile Glu Asn Tyr Ile His Arg
            340                 345                 350

Ile Gly Arg Ser Gly Arg Phe Gly Arg Lys Gly Val Ala Ile Asn Phe
        355             360                 365

Ile Thr Lys Ala Asp Leu Ala Lys Leu Arg Glu Ile Glu Lys Phe Tyr
    370             375                 380

Ser Ile Lys Ile Asn Pro Met Pro Ala Asn Phe Ala Glu Leu Ser
385                 390                 395
```

```
<210>  14
<211>  604
<212>  PRT
<213>  Saccharomyces cerevisiae

<400>  14
```

```
Met Ala Glu Leu Ser Glu Gln Val Gln Asn Leu Ser Ile Asn Asp Asn
1               5                   10                  15

Asn Glu Asn Gly Tyr Val Pro Pro His Leu Arg Gly Lys Pro Arg Ser
            20                  25                  30

Ala Arg Asn Asn Ser Ser Asn Tyr Asn Asn Asn Asn Gly Gly Tyr Asn
        35                  40                  45

Gly Gly Arg Gly Gly Gly Ser Phe Phe Ser Asn Asn Arg Arg Gly Gly
    50                  55                  60

Tyr Gly Asn Gly Gly Phe Phe Gly Gly Asn Asn Gly Gly Ser Arg Ser
65                  70                  75                  80

Asn Gly Arg Ser Gly Gly Arg Trp Ile Asp Gly Lys His Val Pro Ala
            85                  90                  95

Pro Arg Asn Glu Lys Ala Glu Ile Ala Ile Phe Gly Val Pro Glu Asp
            100                 105                 110

Pro Asn Phe Gln Ser Ser Gly Ile Asn Phe Asp Asn Tyr Asp Asp Ile
        115                 120                 125

Pro Val Asp Ala Ser Gly Lys Asp Val Pro Glu Pro Ile Thr Glu Phe
    130                 135                 140

Thr Ser Pro Pro Leu Asp Gly Leu Leu Leu Glu Asn Ile Lys Leu Ala
145                 150                 155                 160

Arg Phe Thr Lys Pro Thr Pro Val Gln Lys Tyr Ser Val Pro Ile Val
            165                 170                 175

Ala Asn Gly Arg Asp Leu Met Ala Cys Ala Gln Thr Gly Ser Gly Lys
            180                 185                 190

Thr Gly Gly Phe Leu Phe Pro Val Leu Ser Glu Ser Phe Lys Thr Gly
            195                 200                 205

Pro Ser Pro Gln Pro Glu Ser Gln Gly Ser Phe Tyr Gln Arg Lys Ala
    210                 215                 220

Tyr Pro Thr Ala Val Ile Met Ala Pro Thr Arg Glu Leu Ala Thr Gln
225                 230                 235                 240

Ile Phe Asp Glu Ala Lys Lys Phe Thr Tyr Arg Ser Trp Val Lys Ala
            245                 250                 255

Cys Val Val Tyr Gly Gly Ser Pro Ile Gly Asn Gln Leu Arg Glu Ile
            260                 265                 270
```

```
Glu Arg Gly Cys Asp Leu Leu Val Ala Thr Pro Gly Arg Leu Asn Asp
        275                 280                 285

Leu Leu Glu Arg Gly Lys Ile Ser Leu Ala Asn Val Lys Tyr Leu Val
    290                 295                 300

Leu Asp Glu Ala Asp Arg Met Leu Asp Met Gly Phe Glu Pro Gln Ile
305                 310                 315                 320

Arg His Ile Val Glu Asp Cys Asp Met Thr Pro Val Gly Glu Arg Gln
                325                 330                 335

Thr Leu Met Phe Ser Ala Thr Phe Pro Ala Asp Ile Gln His Leu Ala
            340                 345                 350

Arg Asp Phe Leu Ser Asp Tyr Ile Phe Leu Ser Val Gly Arg Val Gly
        355                 360                 365

Ser Thr Ser Glu Asn Ile Thr Gln Lys Val Leu Tyr Val Glu Asn Gln
    370                 375                 380

Asp Lys Lys Ser Ala Leu Leu Asp Leu Leu Ser Ala Ser Thr Asp Gly
385                 390                 395                 400

Leu Thr Leu Ile Phe Val Glu Thr Lys Arg Met Ala Asp Gln Leu Thr
            405                 410                 415

Asp Phe Leu Ile Met Gln Asn Phe Arg Ala Thr Ala Ile His Gly Asp
            420                 425                 430

Arg Thr Gln Ser Glu Arg Glu Arg Ala Leu Ala Ala Phe Arg Ser Gly
        435                 440                 445

Ala Ala Thr Leu Leu Val Ala Thr Ala Val Ala Ala Arg Gly Leu Asp
    450                 455                 460

Ile Pro Asn Val Thr His Val Ile Asn Tyr Asp Leu Pro Ser Asp Val
465                 470                 475                 480

Asp Asp Tyr Val His Arg Ile Gly Arg Thr Gly Arg Ala Gly Asn Thr
                485                 490                 495

Gly Leu Ala Thr Ala Phe Phe Asn Ser Glu Asn Ser Asn Ile Val Lys
            500                 505                 510

Gly Leu His Glu Ile Leu Thr Glu Ala Asn Gln Glu Val Pro Ser Phe
        515                 520                 525

Leu Lys Asp Ala Met Met Ser Ala Pro Gly Ser Arg Ser Asn Ser Arg
        530                 535                 540
```

Arg Gly Gly Phe Gly Arg Asn Asn Asn Arg Asp Tyr Arg Lys Ala Gly
545             550             555             560

Gly Ala Ser Ala Gly Gly Trp Gly Ser Ser Arg Ser Arg Asp Asn Ser
                565             570             575

Phe Arg Gly Gly Ser Gly Trp Gly Ser Asp Ser Lys Ser Ser Gly Trp
            580             585             590

Gly Asn Ser Gly Gly Ser Asn Asn Ser Ser Trp Trp
        595             600

<210> 15
<211> 406
<212> PRT
<213> Homo sapiens

<400> 15

Met Ser Ala Ser Gln Asp Ser Arg Ser Arg Asp Asn Gly Pro Asp Gly
1               5               10              15

Met Glu Pro Glu Gly Val Ile Glu Ser Asn Trp Asn Glu Ile Val Asp
            20              25              30

Ser Phe Asp Asp Met Asn Leu Ser Glu Ser Leu Leu Arg Gly Ile Tyr
        35              40              45

Ala Tyr Gly Phe Glu Lys Pro Ser Ala Ile Gln Gln Arg Ala Ile Leu
    50              55              60

Pro Cys Ile Lys Gly Tyr Asp Val Ile Ala Gln Ala Gln Ser Gly Thr
65              70              75              80

Gly Lys Thr Ala Thr Phe Ala Ile Ser Ile Leu Gln Gln Ile Glu Leu
                85              90              95

Asp Leu Lys Ala Thr Gln Ala Leu Val Leu Ala Pro Thr Arg Glu Leu
            100             105             110

Ala Gln Gln Ile Gln Lys Val Val Met Ala Leu Gly Asp Tyr Met Gly
        115             120             125

Ala Ser Cys His Ala Cys Ile Gly Gly Thr Asn Val Arg Ala Glu Val
    130             135             140

Gln Lys Leu Gln Met Glu Ala Pro His Ile Ile Val Gly Thr Pro Gly
145             150             155             160

Arg Val Phe Asp Met Leu Asn Arg Arg Tyr Leu Ser Pro Lys Tyr Ile
                165             170             175

```
Lys Met Phe Val Leu Asp Glu Ala Asp Glu Met Leu Ser Arg Gly Phe
            180                 185                 190

Lys Asp Gln Ile Tyr Asp Ile Phe Gln Lys Leu Asn Ser Asn Thr Gln
            195                 200                 205

Val Val Leu Leu Ser Ala Thr Met Pro Ser Asp Val Leu Glu Val Thr
    210                 215                 220

Lys Lys Phe Met Arg Asp Pro Ile Arg Ile Leu Val Lys Lys Glu Glu
225                 230                 235                 240

Leu Thr Leu Glu Gly Ile Arg Gln Phe Tyr Ile Asn Val Glu Arg Glu
                245                 250                 255

Glu Trp Lys Leu Asp Thr Leu Cys Asp Leu Tyr Glu Thr Leu Thr Ile
            260                 265                 270

Thr Gln Ala Val Ile Phe Ile Asn Thr Arg Arg Lys Val Asp Trp Leu
            275                 280                 285

Thr Glu Lys Met His Ala Arg Asp Phe Thr Val Ser Ala Met His Gly
    290                 295                 300

Asp Met Asp Gln Lys Glu Arg Asp Val Ile Met Arg Glu Phe Arg Ser
305                 310                 315                 320

Gly Ser Ser Arg Val Leu Ile Thr Thr Asp Leu Leu Ala Arg Gly Ile
                325                 330                 335

Asp Val Gln Gln Val Ser Leu Val Ile Asn Tyr Asp Leu Pro Thr Asn
            340                 345                 350

Arg Glu Asn Tyr Ile His Arg Ile Gly Arg Gly Gly Arg Phe Gly Arg
            355                 360                 365

Lys Gly Val Ala Ile Asn Met Val Thr Glu Glu Asp Lys Arg Thr Leu
    370                 375                 380

Arg Asp Ile Glu Thr Phe Tyr Asn Thr Ser Ile Glu Glu Met Pro Leu
385                 390                 395                 400

Asn Val Ala Asp Leu Ile
                405
```

```
<210>   16
<211>   411
<212>   PRT
<213>   Homo sapiens

<400>   16

Met Ala Thr Thr Ala Thr Met Ala Thr Ser Gly Ser Ala Arg Lys Arg
```

38

|     |     |     | 1   |     |     |     |     | 5   |     |     |     |     | 10  |     |     |     |     | 15  |     |
|-----|-----|-----|-----|-----|-----|-----|-----|-----|-----|-----|-----|-----|-----|-----|-----|-----|-----|-----|-----|

Leu Leu Lys Glu Glu Asp Met Thr Lys Val Glu Phe Glu Thr Ser Glu
        20              25                  30

Glu Val Asp Val Thr Pro Thr Phe Asp Thr Met Gly Leu Arg Glu Asp
        35              40                  45

Leu Leu Arg Gly Ile Tyr Ala Tyr Gly Phe Glu Lys Pro Ser Ala Ile
    50              55                  60

Gln Gln Arg Ala Ile Lys Gln Ile Ile Lys Gly Arg Asp Val Ile Ala
65              70                  75                  80

Gln Ser Gln Ser Gly Thr Gly Lys Thr Ala Thr Phe Ser Ile Ser Val
                85                  90                  95

Leu Gln Cys Leu Asp Ile Gln Val Arg Glu Thr Gln Ala Leu Ile Leu
            100                 105                 110

Ala Pro Thr Arg Glu Leu Ala Val Gln Ile Gln Lys Gly Leu Leu Ala
        115                 120                 125

Leu Gly Asp Tyr Met Asn Val Gln Cys His Ala Cys Ile Gly Gly Thr
    130                 135                 140

Asn Val Gly Glu Asp Ile Arg Lys Leu Asp Tyr Gly Gln His Val Val
145                 150                 155                 160

Ala Gly Thr Pro Gly Arg Val Phe Asp Met Ile Arg Arg Arg Ser Leu
                165                 170                 175

Arg Thr Arg Ala Ile Lys Met Leu Val Leu Asp Glu Ala Asp Glu Met
        180                 185                 190

Leu Asn Lys Gly Phe Lys Glu Gln Ile Tyr Asp Val Tyr Arg Tyr Leu
        195                 200                 205

Pro Pro Ala Thr Gln Val Val Leu Ile Ser Ala Thr Leu Pro His Glu
    210                 215                 220

Ile Leu Glu Met Thr Asn Lys Phe Met Thr Asp Pro Ile Arg Ile Leu
225                 230                 235                 240

Val Lys Arg Asp Glu Leu Thr Leu Glu Gly Ile Lys Gln Phe Phe Val
                245                 250                 255

Ala Val Glu Arg Glu Glu Trp Lys Phe Asp Thr Leu Cys Asp Leu Tyr
            260                 265                 270

Asp Thr Leu Thr Ile Thr Gln Ala Val Ile Phe Cys Asn Thr Lys Arg

275                    280                    285

Lys Val Asp Trp Leu Thr Glu Lys Met Arg Glu Ala Asn Phe Thr Val
    290             295             300

Ser Ser Met His Gly Asp Met Pro Gln Lys Glu Arg Glu Ser Ile Met
305             310             315             320

Lys Glu Phe Arg Ser Gly Ala Ser Arg Val Leu Ile Ser Thr Asp Val
            325             330             335

Trp Ala Arg Gly Leu Asp Val Pro Gln Val Ser Leu Ile Ile Asn Tyr
            340             345             350

Asp Leu Pro Asn Asn Arg Glu Leu Tyr Ile His Arg Ile Gly Arg Ser
        355             360             365

Gly Arg Tyr Gly Arg Lys Gly Val Ala Ile Asn Phe Val Lys Asn Asp
    370             375             380

Asp Ile Arg Ile Leu Arg Asp Ile Glu Gln Tyr Tyr Ser Thr Gln Ile
385             390             395             400

Asp Glu Met Pro Met Asn Val Ala Asp Leu Ile
            405             410

<210>  17
<211>  195
<212>  PRT
<213>  Leishmania infantum

<400>  17

Met Ala Gln Asn Asp Lys Ile Ala Pro Gln Asp Gln Asp Ser Phe Leu
1               5               10              15

Asp Asp Gln Pro Gly Val Arg Pro Ile Pro Ser Phe Asp Asp Met Pro
            20              25              30

Leu His Gln Asn Leu Leu Arg Gly Ile Tyr Ser Tyr Gly Phe Glu Lys
        35              40              45

Pro Ser Ser Ile Gln Gln Arg Ala Ile Ala Pro Phe Thr Arg Gly Gly
    50              55              60

Asp Ile Ile Ala Gln Ala Gln Ser Gly Thr Gly Lys Thr Gly Ala Phe
65              70              75              80

Ser Ile Gly Leu Leu Gln Arg Leu Asp Phe Arg His Asn Leu Ile Gln
            85              90              95

Gly Leu Val Leu Ser Pro Thr Arg Glu Leu Ala Leu Gln Thr Ala Glu
            100             105             110

```
Val Ile Ser Arg Ile Gly Glu Phe Leu Ser Asn Ser Ser Lys Phe Cys
        115                 120                 125

Glu Thr Phe Val Gly Gly Thr Arg Val Gln Asp Asp Leu Arg Lys Leu
        130                 135                 140

Gln Ala Gly Val Ile Val Ala Val Gly Thr Pro Gly Arg Val Ser Asp
145                 150                 155                 160

Val Ile Lys Arg Gly Ala Leu Arg Thr Glu Ser Leu Arg Val Leu Val
                165                 170                 175

Leu Asp Glu Ala Asp Glu Met Leu Ser Gln Gly Phe Ala Asp Gln Ile
                180                 185                 190

Tyr Glu Ile
        195


<210>  18
<211>  126
<212>  PRT
<213>  Leishmania infantum

<400>  18

Met Ala Gln Asn Asp Lys Ile Ala Pro Gln Asp Gln Asp Ser Phe Leu
1                 5                 10                 15

Asp Asp Gln Pro Gly Val Arg Pro Ile Pro Ser Phe Asp Asp Met Pro
                20                 25                 30

Leu His Gln Asn Leu Leu Arg Gly Ile Tyr Ser Tyr Gly Phe Glu Lys
                35                 40                 45

Pro Ser Ser Ile Gln Gln Arg Ala Ile Ala Pro Phe Thr Arg Gly Gly
        50                 55                 60

Asp Ile Ile Ala Gln Ala Gln Ser Gly Thr Gly Lys Thr Gly Ala Phe
65                 70                 75                 80

Ser Ile Gly Leu Leu Gln Arg Leu Asp Phe Arg His Asn Leu Ile Gln
                85                 90                 95

Gly Leu Val Leu Ser Pro Thr Arg Glu Leu Ala Leu Gln Thr Ala Glu
                100                 105                 110

Val Ile Ser Arg Ile Gly Glu Phe Leu Ser Asn Ser Ser Lys
        115                 120                 125


<210>  19
<211>  237
<212>  PRT
```

<213> Leishmania infantum

<400> 19

Met Ala Gln Asn Asp Lys Ile Ala Pro Gln Asp Gln Asp Ser Phe Leu
1               5                   10                  15

Asp Asp Gln Pro Gly Val Arg Pro Ile Pro Ser Phe Asp Asp Met Pro
            20                  25                  30

Leu His Gln Asn Leu Leu Arg Gly Ile Tyr Ser Tyr Gly Phe Glu Lys
        35                  40                  45

Pro Ser Ser Ile Gln Gln Arg Ala Ile Ala Pro Phe Thr Arg Gly Gly
    50                  55                  60

Asp Ile Ile Ala Gln Ala Gln Ser Gly Thr Gly Lys Thr Gly Ala Phe
65                  70                  75                  80

Ser Ile Gly Leu Leu Gln Arg Leu Asp Phe Arg His Asn Leu Ile Gln
                85                  90                  95

Gly Leu Val Leu Ser Pro Thr Arg Glu Leu Ala Leu Gln Thr Ala Glu
            100                 105                 110

Val Ile Ser Arg Ile Gly Glu Phe Leu Ser Asn Ser Ser Lys Phe Cys
        115                 120                 125

Glu Thr Phe Val Gly Gly Thr Arg Val Gln Asp Asp Leu Arg Lys Leu
    130                 135                 140

Gln Ala Gly Val Ile Val Ala Val Gly Thr Pro Gly Arg Val Ser Asp
145                 150                 155                 160

Val Ile Lys Arg Gly Ala Leu Arg Thr Glu Ser Leu Arg Val Leu Val
                165                 170                 175

Leu Asp Glu Ala Asp Glu Met Leu Ser Gln Gly Phe Ala Asp Gln Ile
            180                 185                 190

Tyr Glu Ile Phe Arg Phe Leu Pro Lys Asp Ile Gln Val Ala Leu Phe
        195                 200                 205

Ser Ala Thr Met Pro Glu Glu Val Leu Glu Leu Thr Lys Lys Phe Met
    210                 215                 220

Arg Asp Pro Val Arg Ile Leu Val Lys Arg Glu Ser Leu
225                 230                 235

<210> 20
<211> 213
<212> PRT
<213> Leishmania infantum

<400> 20

```
Ile Pro Ser Phe Asp Asp Met Pro Leu His Gln Asn Leu Leu Arg Gly
1               5                   10                  15

Ile Tyr Ser Tyr Gly Phe Glu Lys Pro Ser Ser Ile Gln Gln Arg Ala
            20                  25                  30

Ile Ala Pro Phe Thr Arg Gly Gly Asp Ile Ile Ala Gln Ala Gln Ser
        35                  40                  45

Gly Thr Gly Lys Thr Gly Ala Phe Ser Ile Gly Leu Leu Gln Arg Leu
    50                  55                  60

Asp Phe Arg His Asn Leu Ile Gln Gly Leu Val Leu Ser Pro Thr Arg
65                  70                  75                  80

Glu Leu Ala Leu Gln Thr Ala Glu Val Ile Ser Arg Ile Gly Glu Phe
            85                  90                  95

Leu Ser Asn Ser Ser Lys Phe Cys Glu Thr Phe Val Gly Gly Thr Arg
            100                 105                 110

Val Gln Asp Asp Leu Arg Lys Leu Gln Ala Gly Val Ile Val Ala Val
        115                 120                 125

Gly Thr Pro Gly Arg Val Ser Asp Val Ile Lys Arg Gly Ala Leu Arg
    130                 135                 140

Thr Glu Ser Leu Arg Val Leu Val Leu Asp Glu Ala Asp Glu Met Leu
145                 150                 155                 160

Ser Gln Gly Phe Ala Asp Gln Ile Tyr Glu Ile Phe Arg Phe Leu Pro
            165                 170                 175

Lys Asp Ile Gln Val Ala Leu Phe Ser Ala Thr Met Pro Glu Glu Val
            180                 185                 190

Leu Glu Leu Thr Lys Lys Phe Met Arg Asp Pro Val Arg Ile Leu Val
        195                 200                 205

Lys Arg Glu Ser Leu
    210
```

<210> 21
<211> 378
<212> PRT
<213> Leishmania infantum

<400> 21

```
Pro Ser Phe Asp Asp Met Pro Leu His Gln Asn Leu Leu Arg Gly Ile
1               5                   10                  15
```

```
Tyr Ser Tyr Gly Phe Glu Lys Pro Ser Ser Ile Gln Gln Arg Ala Ile
            20              25              30

Ala Pro Phe Thr Arg Gly Gly Asp Ile Ile Ala Gln Ala Gln Ser Gly
        35              40              45

Thr Gly Lys Thr Gly Ala Phe Ser Ile Gly Leu Leu Gln Arg Leu Asp
    50              55              60

Phe Arg His Asn Leu Ile Gln Gly Leu Val Leu Ser Pro Thr Arg Glu
65              70              75              80

Leu Ala Leu Gln Thr Ala Glu Val Ile Ser Arg Ile Gly Glu Phe Leu
            85              90              95

Ser Asn Ser Ser Lys Phe Cys Glu Thr Phe Val Gly Gly Thr Arg Val
            100             105             110

Gln Asp Asp Leu Arg Lys Leu Gln Ala Gly Val Ile Val Ala Val Gly
        115             120             125

Thr Pro Gly Arg Val Ser Asp Val Ile Lys Arg Gly Ala Leu Arg Thr
        130             135             140

Glu Ser Leu Arg Val Leu Val Leu Asp Glu Ala Asp Glu Met Leu Ser
145             150             155             160

Gln Gly Phe Ala Asp Gln Ile Tyr Glu Ile Phe Arg Phe Leu Pro Lys
            165             170             175

Asp Ile Gln Val Ala Leu Phe Ser Ala Thr Met Pro Glu Glu Val Leu
        180             185             190

Glu Leu Thr Lys Lys Phe Met Arg Asp Pro Val Arg Ile Leu Val Lys
        195             200             205

Arg Glu Ser Leu Thr Leu Glu Gly Ile Lys Gln Phe Phe Ile Ala Val
    210             215             220

Glu Glu Glu His Lys Leu Asp Thr Leu Met Asp Leu Tyr Glu Thr Val
225             230             235             240

Ser Ile Ala Gln Ser Val Ile Phe Ala Asn Thr Arg Arg Lys Val Asp
            245             250             255

Trp Ile Ala Glu Lys Leu Asn Gln Ser Asn His Thr Val Ser Ser Met
            260             265             270

His Ala Glu Met Pro Lys Ser Asp Arg Glu Arg Val Met Asn Thr Phe
    275             280             285
```

Arg Ser Gly Ser Ser Arg Val Leu Val Thr Thr Asp Leu Val Ala Arg
290 295 300

Gly Ile Asp Val His His Val Asn Ile Val Ile Asn Phe Asp Leu Pro
305 310 315 320

Thr Asn Lys Glu Asn Tyr Leu His Arg Ile Gly Arg Gly Gly Arg Tyr
325 330 335

Gly Arg Lys Gly Val Ala Ile Asn Phe Val Thr Glu Lys Asp Val Glu
340 345 350

Leu Leu His Glu Ile Glu Ala His Tyr His Thr Gln Ile Asp Glu Leu
355 360 365

Pro Val Asp Phe Ala Ala Tyr Leu Gly Glu
370 375

<210> 22
<211> 98
<212> PRT
<213> Leishmania infantum

<400> 22

Glu Thr Phe Val Gly Gly Thr Arg Val Gln Asp Asp Leu Arg Lys Leu
1 5 10 15

Gln Ala Gly Val Ile Val Ala Val Gly Thr Pro Gly Arg Val Ser Asp
20 25 30

Val Ile Lys Arg Gly Ala Leu Arg Thr Glu Ser Leu Arg Val Leu Val
35 40 45

Leu Asp Glu Ala Asp Glu Met Leu Ser Gln Gly Phe Ala Asp Gln Ile
50 55 60

Tyr Glu Ile Phe Arg Phe Leu Pro Lys Asp Ile Gln Val Ala Leu Phe
65 70 75 80

Ser Ala Thr Met Pro Glu Glu Val Leu Glu Leu Thr Lys Lys Phe Met
85 90 95

Arg Asp

<210> 23
<211> 133
<212> PRT
<213> Leishmania infantum

<400> 23

```
Glu Thr Phe Val Gly Gly Thr Arg Val Gln Asp Asp Leu Arg Lys Leu
1               5               10              15

Gln Ala Gly Val Ile Val Ala Val Gly Thr Pro Gly Arg Val Ser Asp
            20              25              30

Val Ile Lys Arg Gly Ala Leu Arg Thr Glu Ser Leu Arg Val Leu Val
        35              40              45

Leu Asp Glu Ala Asp Glu Met Leu Ser Gln Gly Phe Ala Asp Gln Ile
    50              55              60

Tyr Glu Ile Phe Arg Phe Leu Pro Lys Asp Ile Gln Val Ala Leu Phe
65              70              75              80

Ser Ala Thr Met Pro Glu Glu Val Leu Glu Leu Thr Lys Lys Phe Met
            85              90              95

Arg Asp Pro Val Arg Ile Leu Val Lys Arg Glu Ser Leu Thr Leu Glu
            100             105             110

Gly Ile Lys Gln Phe Phe Ile Ala Val Glu Glu Glu His Lys Leu Asp
        115             120             125

Thr Leu Met Asp Leu
    130
```

```
<210>   24
<211>   208
<212>   PRT
<213>   Leishmania infantum

<400>   24
```

```
Phe Arg Phe Leu Pro Lys Asp Ile Gln Val Ala Leu Phe Ser Ala Thr
1               5               10              15

Met Pro Glu Glu Val Leu Glu Leu Thr Lys Lys Phe Met Arg Asp Pro
            20              25              30

Val Arg Ile Leu Val Lys Arg Glu Ser Leu Thr Leu Glu Gly Ile Lys
        35              40              45

Gln Phe Phe Ile Ala Val Glu Glu Glu His Lys Leu Asp Thr Leu Met
    50              55              60

Asp Leu Tyr Glu Thr Val Ser Ile Ala Gln Ser Val Ile Phe Ala Asn
65              70              75              80

Thr Arg Arg Lys Val Asp Trp Ile Ala Glu Lys Leu Asn Gln Ser Asn
            85              90              95

His Thr Val Ser Ser Met His Ala Glu Met Pro Lys Ser Asp Arg Glu
```

                    100                    105                    110

Arg Val Met Asn Thr Phe Arg Ser Gly Ser Ser Arg Val Leu Val Thr
        115                120                125

Thr Asp Leu Val Ala Arg Gly Ile Asp Val His His Val Asn Ile Val
    130                135                140

Ile Asn Phe Asp Leu Pro Thr Asn Lys Glu Asn Tyr Leu His Arg Ile
145                150                155                160

Gly Arg Gly Gly Arg Tyr Gly Arg Lys Gly Val Ala Ile Asn Phe Val
                165                170                175

Thr Glu Lys Asp Val Glu Leu Leu His Glu Ile Glu Ala His Tyr His
            180                185                190

Thr Gln Ile Asp Glu Leu Pro Val Asp Phe Ala Ala Tyr Leu Gly Glu
            195                200                205

<210>   25
<211>   167
<212>   PRT
<213>   Leishmania infantum

<400>   25

Leu Thr Leu Glu Gly Ile Lys Gln Phe Phe Ile Ala Val Glu Glu Glu
1               5                10                15

His Lys Leu Asp Thr Leu Met Asp Leu Tyr Glu Thr Val Ser Ile Ala
        20                25                30

Gln Ser Val Ile Phe Ala Asn Thr Arg Arg Lys Val Asp Trp Ile Ala
        35                40                45

Glu Lys Leu Asn Gln Ser Asn His Thr Val Ser Ser Met His Ala Glu
    50                55                60

Met Pro Lys Ser Asp Arg Glu Arg Val Met Asn Thr Phe Arg Ser Gly
65                70                75                80

Ser Ser Arg Val Leu Val Thr Thr Asp Leu Val Ala Arg Gly Ile Asp
            85                90                95

Val His His Val Asn Ile Val Ile Asn Phe Asp Leu Pro Thr Asn Lys
            100                105                110

Glu Asn Tyr Leu His Arg Ile Gly Arg Gly Gly Arg Tyr Gly Arg Lys
        115                120                125

Gly Val Ala Ile Asn Phe Val Thr Glu Lys Asp Val Glu Leu Leu His
    130                135                140

Glu Ile Glu Ala His Tyr His Thr Gln Ile Asp Glu Leu Pro Val Asp
145                 150                 155                 160

Phe Ala Ala Tyr Leu Gly Glu
                165

<210> 26
<211> 143
<212> PRT
<213> Leishmania infantum

<400> 26

Leu Tyr Glu Thr Val Ser Ile Ala Gln Ser Val Ile Phe Ala Asn Thr
1               5                   10                  15

Arg Arg Lys Val Asp Trp Ile Ala Glu Lys Leu Asn Gln Ser Asn His
            20                  25                  30

Thr Val Ser Ser Met His Ala Glu Met Pro Lys Ser Asp Arg Glu Arg
            35                  40                  45

Val Met Asn Thr Phe Arg Ser Gly Ser Ser Arg Val Leu Val Thr Thr
        50                  55                  60

Asp Leu Val Ala Arg Gly Ile Asp Val His His Val Asn Ile Val Ile
65                  70                  75                  80

Asn Phe Asp Leu Pro Thr Asn Lys Glu Asn Tyr Leu His Arg Ile Gly
                85                  90                  95

Arg Gly Gly Arg Tyr Gly Arg Lys Gly Val Ala Ile Asn Phe Val Thr
                100                 105                 110

Glu Lys Asp Val Glu Leu Leu His Glu Ile Glu Ala His Tyr His Thr
            115                 120                 125

Gln Ile Asp Glu Leu Pro Val Asp Phe Ala Ala Tyr Leu Gly Glu
        130                 135                 140

<210> 27
<211> 403
<212> PRT
<213> Artificial

<220>
<223> Protéine LeIF de Leishmania infantum mutante présentant une
      substitution de la lysine en position 7 du motif I par l'alanine

<400> 27

Met Ala Gln Asn Asp Lys Ile Ala Pro Gln Asp Gln Asp Ser Phe Leu
1               5                   10                  15

```
    Asp Asp Gln Pro Gly Val Arg Pro Ile Pro Ser Phe Asp Asp Met Pro
                 20                  25                 30

    Leu His Gln Asn Leu Leu Arg Gly Ile Tyr Ser Tyr Gly Phe Glu Lys
             35                  40                 45

    Pro Ser Ser Ile Gln Gln Arg Ala Ile Ala Pro Phe Thr Arg Gly Gly
         50                  55                 60

    Asp Ile Ile Ala Gln Ala Gln Ser Gly Thr Gly Ala Thr Gly Ala Phe
    65                  70                  75                     80

    Ser Ile Gly Leu Leu Gln Arg Leu Asp Phe Arg His Asn Leu Ile Gln
                 85                  90                 95

    Gly Leu Val Leu Ser Pro Thr Arg Glu Leu Ala Leu Gln Thr Ala Glu
                 100                 105                110

    Val Ile Ser Arg Ile Gly Glu Phe Leu Ser Asn Ser Ser Lys Phe Cys
             115                 120                125

    Glu Thr Phe Val Gly Gly Thr Arg Val Gln Asp Asp Leu Arg Lys Leu
         130                 135                140

    Gln Ala Gly Val Ile Val Ala Val Gly Thr Pro Gly Arg Val Ser Asp
    145                 150                 155                    160

    Val Ile Lys Arg Gly Ala Leu Arg Thr Glu Ser Leu Arg Val Leu Val
                 165                 170                175

    Leu Asp Glu Ala Asp Glu Met Leu Ser Gln Gly Phe Ala Asp Gln Ile
                 180                 185                190

    Tyr Glu Ile Phe Arg Phe Leu Pro Lys Asp Ile Gln Val Ala Leu Phe
                 195                 200                205

    Ser Ala Thr Met Pro Glu Glu Val Leu Glu Leu Thr Lys Lys Phe Met
         210                 215                220

    Arg Asp Pro Val Arg Ile Leu Val Lys Arg Glu Ser Leu Thr Leu Glu
    225                 230                 235                    240

    Gly Ile Lys Gln Phe Phe Ile Ala Val Glu Glu Glu His Lys Leu Asp
                 245                 250                255

    Thr Leu Met Asp Leu Tyr Glu Thr Val Ser Ile Ala Gln Ser Val Ile
                 260                 265                270

    Phe Ala Asn Thr Arg Arg Lys Val Asp Trp Ile Ala Glu Lys Leu Asn
             275                 280                285
```

Gln Ser Asn His Thr Val Ser Ser Met His Ala Glu Met Pro Lys Ser
    290                 295             300

Asp Arg Glu Arg Val Met Asn Thr Phe Arg Ser Gly Ser Ser Arg Val
305             310             315                 320

Leu Val Thr Thr Asp Leu Val Ala Arg Gly Ile Asp Val His His Val
                325             330             335

Asn Ile Val Ile Asn Phe Asp Leu Pro Thr Asn Lys Glu Asn Tyr Leu
            340             345             350

His Arg Ile Gly Arg Gly Gly Arg Tyr Gly Arg Lys Gly Val Ala Ile
        355             360             365

Asn Phe Val Thr Glu Lys Asp Val Glu Leu Leu His Glu Ile Glu Ala
    370             375             380

His Tyr His Thr Gln Ile Asp Glu Leu Pro Val Asp Phe Ala Ala Tyr
385             390             395             400

Leu Gly Glu


<210>  28
<211>  9
<212>  PRT
<213>  Artificial

<220>
<223>  motif Q d'eIF4A

<400>  28

Gly Phe Glu Glu Pro Ser Ala Ile Gln
1               5


<210>  29
<211>  8
<212>  PRT
<213>  Artificial

<220>
<223>  motif I d'eIF4A

<400>  29

Ala Gln Ser Gly Thr Gly Lys Thr
1               5


<210>  30
<211>  6
<212>  PRT
<213>  Artificial

<220>
<223>  motif Ia d'eIF4A

```
<400>   30

Pro Thr Arg Glu Leu Ala
1               5


<210>   31
<211>   5
<212>   PRT
<213>   Artificial

<220>
<223>   motif Ib d'eIF4A

<400>   31

Thr Pro Gly Arg Val
1               5


<210>   32
<211>   4
<212>   PRT
<213>   Artificial

<220>
<223>   motif II d'eIF4A

<400>   32

Asp Glu Ala Asp
1


<210>   33
<211>   8
<212>   PRT
<213>   Artificial

<220>
<223>   motif IV d'eIF4A

<400>   33

Val Ile Phe Cys Asn Thr Arg Arg
1               5


<210>   34
<211>   9
<212>   PRT
<213>   Artificial

<220>
<223>   motif V d'eIF4A

<400>   34

Thr Asp Leu Leu Ala Arg Gly Ile Asp
1               5


<210>   35
<211>   8
<212>   PRT
<213>   Artificial

<220>
```

<223> motif VI d'eIF4A

<400> 35

His Arg Ile Gly Arg Gly Gly Arg
1               5


<210> 36
<211> 8
<212> PRT
<213> Artificial

<220>
<223> motif I de Prp2

<400> 36

Gly Glu Thr Gly Ser Gly Lys Thr
1               5


<210> 37
<211> 6
<212> PRT
<213> Artificial

<220>
<223> motif Ia de Prp2

<400> 37

Pro Arg Arg Val Ala Ala
1               5


<210> 38
<211> 5
<212> PRT
<213> Artificial

<220>
<223> motif Ib de Prp2

<400> 38

Thr Asp Gly Met Leu
1               5


<210> 39
<211> 4
<212> PRT
<213> Artificial

<220>
<223> motif II de Prp2

<400> 39

Asp Glu Ala His
1


<210> 40
<211> 8
<212> PRT
<213> Artificial

```
<220>
<223>  motif IV de Prp2

<400>  40

Leu Val Phe Leu Thr Gly Gln Glu
1               5


<210>  41
<211>  9
<212>  PRT
<213>  Artificial

<220>
<223>  motif V Prp2

<400>  41

Thr Asn Ile Ala Glu Thr Ser Leu Thr
1               5


<210>  42
<211>  8
<212>  PRT
<213>  Artificial

<220>
<223>  motif VI de Prp2

<400>  42

Gln Arg Ala Gly Arg Ala Gly Arg
1               5


<210>  43
<211>  8
<212>  PRT
<213>  Artificial

<220>
<223>  motif I de NS3

<400>  43

Ala Pro Thr Gly Ser Gly Lys Ser
1               5


<210>  44
<211>  5
<212>  PRT
<213>  Artificial

<220>
<223>  motif Ia de NS3

<400>  44

Pro Ser Val Ala Ala
1               5


<210>  45
<211>  5
```

```
<212>  PRT
<213>  Artificial

<220>
<223>  motif Ib de NS3

<400>  45

Thr Tyr Ser Thr Tyr
1               5


<210>  46
<211>  4
<212>  PRT
<213>  Artificial

<220>
<223>  motif II de NS3

<400>  46

Asp Glu Cys His
1


<210>  47
<211>  8
<212>  PRT
<213>  Artificial

<220>
<223>  motif IV de NS3

<400>  47

Leu Ile Phe Trp His Ser Lys Lys
1               5


<210>  48
<211>  9
<212>  PRT
<213>  Artificial

<220>
<223>  motif V de NS3

<400>  48

Thr Asp Ala Leu Met Thr Gly Tyr Asp
1               5


<210>  49
<211>  8
<212>  PRT
<213>  Artificial

<220>
<223>  motif VI de NS3

<400>  49

Gln Arg Arg Gly Arg Thr Gly Arg
1               5
```

```
<210>   50
<211>   8
<212>   PRT
<213>   Artificial

<220>
<223>   motif I de SKi2

<400>   50

Ala Pro Thr Gly Ala Gly Lys Thr
1                   5


<210>   51
<211>   6
<212>   PRT
<213>   Artificial

<220>
<223>   motif Ia de SKi2

<400>   51

Pro Leu Lys Ala Leu Ser
1                   5


<210>   52
<211>   5
<212>   PRT
<213>   Artificial

<220>
<223>   motif Ib de SKi2

<400>   52

Thr Thr Glu Val Leu
1                   5


<210>   53
<211>   4
<212>   PRT
<213>   Artificial

<220>
<223>   motif II de SKi2

<400>   53

Asp Glu Val His
1


<210>   54
<211>   8
<212>   PRT
<213>   Artificial

<220>
<223>   motif IV de SKi2

<400>   54

Thr Phe Val Phe Ser Arg Phe Gly
1                   5
```

```
<210>  55
<211>  9
<212>  PRT
<213>  Artificial

<220>
<223>  motif V de SKi2

<400>  55

Thr Glu Thr Leu Ala Leu Gly Ile Asn
1               5


<210>  56
<211>  8
<212>  PRT
<213>  Artificial

<220>
<223>  motif VI de SKi2

<400>  56

Gln Leu Thr Gly Arg Ala Gly Arg
1               5
```